(19) Europäisches Patentamt
European Patent Office
Office européen des brevets

(11) Publication number: **0 409 676 B1**

# (12) EUROPEAN PATENT SPECIFICATION

(45) Date of publication of patent specification: **08.03.95**

(51) Int. Cl.[6]: **C07F 7/08**, A61K 31/695

(21) Application number: **90401745.6**

(22) Date of filing: **20.06.90**

## (54) Novel acetylcholinesterase inhibitors.

(30) Priority: **22.06.89 EP 89401775**

(43) Date of publication of application:
**23.01.91 Bulletin 91/04**

(45) Publication of the grant of the patent:
**08.03.95 Bulletin 95/10**

(84) Designated Contracting States:
**AT BE CH DE DK ES FR GB GR IT LI LU NL SE**

(56) References cited:
**US-A- 3 651 115**
**US-A- 4 835 099**

**BIOCHEMISTRY, vol. 24, no. 8, 9th April 1985, pages 1813-1817, Washington, D.C., US; M. GELB et al.: "Fluoro ketone inhibitors of hydrolytic enzymes"**

(73) Proprietor: **MERRELL DOW PHARMACEUTICALS INC.**
**2110 East Galbraith Road**
**Cincinnati**
**Ohio 45215-6300 (US)**

(72) Inventor: **Schirlin, Daniel**
**12, Rue Jean-Jacques Rousseau,**
**Hoenheim**
**F-67800 Bischheim (FR)**
Inventor: **Collard, Jean-Noel**
**11, Rue des Cygnes**
**F-67800 Hoenheim (FR)**
Inventor: **Hornsperger, Jean-Marie, Cité Bellevue**
**Rue des Vergers,**
**Griesheim près Molsheim**
**F-67210 Obernai (FR)**

(74) Representative: **Gillard, Marie-Louise et al**
**Cabinet Beau de Loménie**
**158, rue de l'Université**
**F-75340 Paris Cédex 07 (FR)**

**EP 0 409 676 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to silylated aromatic fluoroketones, to the intermediates and processes for their preparation and to their use in treating diseases associated with deficiencies of cholinergic transmission in the central nervous system.

More specifically, this invention relates to silylated aromatic fluoroketones possessing acetylcholinesterase-inhibiting properties and to their use in the treatment of Alzheimer disease and senile dementia.

Still more specifically, this invention relates to acetylcholinesterase inhibitors of the formula

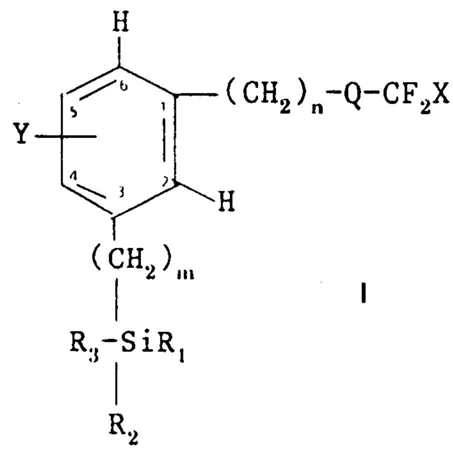

I

and the pharmaceutically acceptable salts thereof, wherein each of m and n is zero or one with the proviso that the sum of m and n is less than two,
Q is

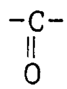, 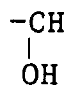 or 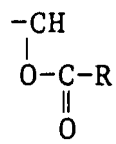

with R being H or $C_{1-10}$ alkyl
X is X′ or X″ with X′ being H, Br, Cl, F or $R_4$ and
X″ being $COR_9$, $CO_2R_5$, $CONHR_5$ or $COR_6$,
$R_1$, $R_2$, $R_3$ and $R_4$ each are $C_{1-10}$ alkyl, or $(CH_2)p$ aryl , with p being zero, one or two,
$R_5$ is H, $C_{1-10}$ alkyl, phenyl, benzyl or phenethyl,
$R_9$ is $C_{1-10}$ alkyl, phenyl, benzyl or phenethyl,
$R_6$ is $(NHCHR_7C(O))_qR_8$ with $R_7$ being the residue of any natural occurring $\alpha$-amino acid, q is one to four and $R_8$ is $OR_5$ or $NHR_5$,
Y is H, OH, $C_{1-6}$ alkyl, $C_{1-6}$ alkoxy, hydroxy $C_{1-6}$ alkyl, amino $C_{1-6}$ alkyl, $NH_2$, azido, CN, $CO_2R_5$, $COR_9$, $-SO_3H$, Br, Cl, F or $-(CH_2)_xSiR_1R_2R_3$ with x being zero, one or two.

As used herein the term "alkyl" includes the straight, branched-chain and cyclized saturated hydrocarbyl moieties having up to 10 (or 6 as otherwise indicated) particularly including such moieties as methyl, ethyl, n-butyl, t-butyl; cyclopropyl, n-propyl, pentyl, hexyl, n-nonyl, decyl, cyclopentyl, cyclohexyl and cyclohexylmethylene. The term "aryl" within the definitions for $R_1$, $R_2$, $R_3$ and $R_4$ includes both carbocyclic and heterocyclic moieties of which phenyl, pyridyl, indolyl, indazolyl, furyl and thienyl are of primary interest; these moieties being inclusive of their position isomers such as, for example, 2-, 3-, or 4-pyridyl, 2- or 3-furyl and thienyl, 1-, 2-, or 3-indolyl or the 1- and 3-indazolyl, as well as the dihydro and tetrahydro analogs of the furyl and thienyl moieties. Also included within the term "aryl" are such fused carbocyclic moieties as pentalenyl, indenyl, naphthalenyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl. Also included within the term "aryl" are such other heterocyciic radicals as 2- or 3-benzo[b]-thienyl, 2-or 3-naphtho[2,3-b]thienyl, 2- or 3-thianthrenyl, 2H-pyran-3-(or 4- or 5-)yl, 1-isobenzofuranyl, 2H-chromenyl-3-yl, 2- or 3-xanthenyl, 2- or 3-phenoxathiinyl, 2- or 3-pyrrolyl, 4- or 3-pyrazolyl, 2-pyrazinyl, 2-pyrimidinyl, 3-pyridazinyl, 2-pyrimidinyl, 3-pyridazinyl, 2-indolizinyl, 1-isoindolyl, 4H-quinolizin-2-yl, 3-isoquinolyl, 2-quinolyl, 1-phthalazinyl, 1,8-naphthyridinyl, 2-quinoxalinyl, 2-quinazolinyl, 3-cinnolinyl, 2-pteridinyl, 4aH-carbazol-2-yl, 2-carbazolyl, β-carbolin-3-yl, 3-phenanthridinyl, 2-acridinyl, 2-perimidinyl, 1-phenazinyl, 3-isothiazolyl, 2-phenothiazinyl, 3-isoxazolyl, 2-phenoxazinyl, 3-isochromanyl, 7-chromanyl, 2-pyrrolidinyl, 2-pyrrolin-3-yl, 2-imidazolidinyl, 2-imidazolin-4-yl, 2-pyrazolidinyl, 3-pyrazolin-3-yl, 2-piperidyl, 2-piperazinyl, 1-indolinyl, 1-isoindolinyl, 3-morpholinyl, benzo[h]isoquinolinyl, and benzo[b]furanyl, including the position isomers thereof except that the heterocyclic moieties cannot be attached directly through their nitrogen atoms.

The term $[NHCHR_7C(O)]_q$ of $R_6$ represents an α-amino acid or a peptide of up to 4 amino acids, with $R_7$ being the residue of any of the naturally occurring α-amino acids (including proline in its natural form) including alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine, nor-valine, n-leucine, 1-naphthylalanine, 2-indolinecarboxylic acid and sarcosin. Of course when q is other than 1 the amino acid moieties may be the same or different. Preferably $R_8$ represents either OH or $NH_2$ but includes the $R_5$ variants thereof, particularly when $R_5$ is methyl or ethyl.

The pharmaceutically acceptable salts of the compounds of formula I include salts formed with nontoxic organic or inorganic acids such as, for example, from the following acids: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic and benzenesulfonic.

The preparation of the compounds of formula I may be effected by a variety of procedures, the choice depending upon the specific combination of the X, Y, m, n, $R_1$, $R_2$ and $R_3$ moieties for any given compound. In any event, the chemical reactions and procedures are analogously known in the art and the selection of any particular route to prepare any particular compound is governed by principles well known and appreciated by those of ordinary skill in the art. Thus, by the application of the below-described procedures and chemical processes generally well-known in the art, the compounds of this invention may be prepared.

To prepare compounds of subgeneric formula

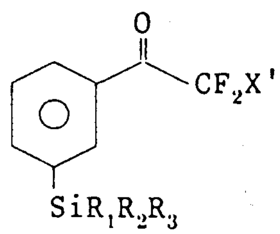

A-10

i.e., those compounds of formula I wherein m and n are zero,
Y is H, and
X′ is H, Cl, Br, F or $R_4$, the compounds may be prepared by the following reaction scheme.

Reaction Scheme A

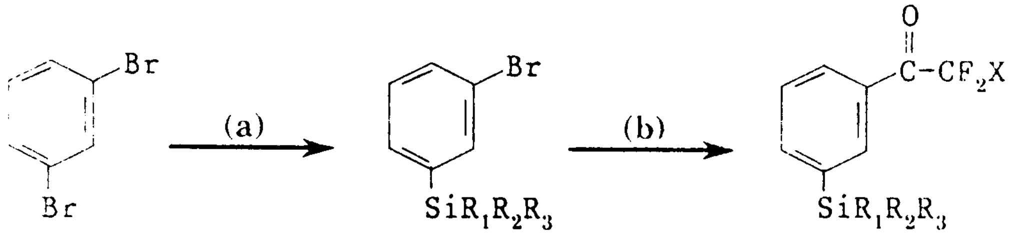


A-11 A-12 A-13

wherein $R_1$, $R_2$, $R_3$ and $R_4$ are as defined in formula I, and X' is H, Cl, Br, F or $R_4$. In the initial step (A-a) the reaction involves the treatment of the dibromo compounds (A-11) with $ClSiR_1R_2R_3$ in the presence of one equivalent of magnesium in a suitable solvent such as diethylether or tetrahydrofuran (THF), said reaction taking place at about the reflux temperature of the mixture to obtain the silylated derivatives (A-12) which, by Step (A-b), are converted to their intermediate lithio derivatives by reaction with an alkyllithium at 0 ° C in diethyl ether or THF and those intermediates are converted to the desired products (A-13) by reaction with three equivalents of the appropriate acid, (preferably as its lithium salt), ester ($X'CF_2CO_2R$), or acid chloride ($X'CF_2COCl$) with R being H or $C_{1-6}$ alkyl, preferably methyl or ethyl). The ketones may be reduced with sodium borohydride or sodium cyano borohydride by reaction in ethanol followed by hydrolysis with saturated ammonium chloride and the resulting alcohols esterified with an acyl halide (ClC-(O)R with R being H or $C_{1-10}$ alkyl), in the presence of TEA (or neat pyridine) in a suitable solvent such as dichloromethane; both these reactions being effected according to well known and understood reaction conditions.

In those instances wherein it is desired to prepare compounds of this invention which are within the sub-genetic scope of the formula

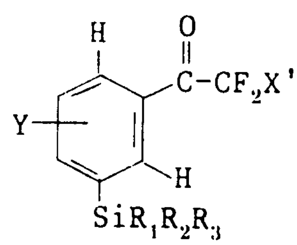

B-10

wherein n = m = o,
Y is other than H, and
X' is H, Br, Cl, F or $R_4$, the chemistry of the following reaction scheme may be utilized.

Reaction Scheme B

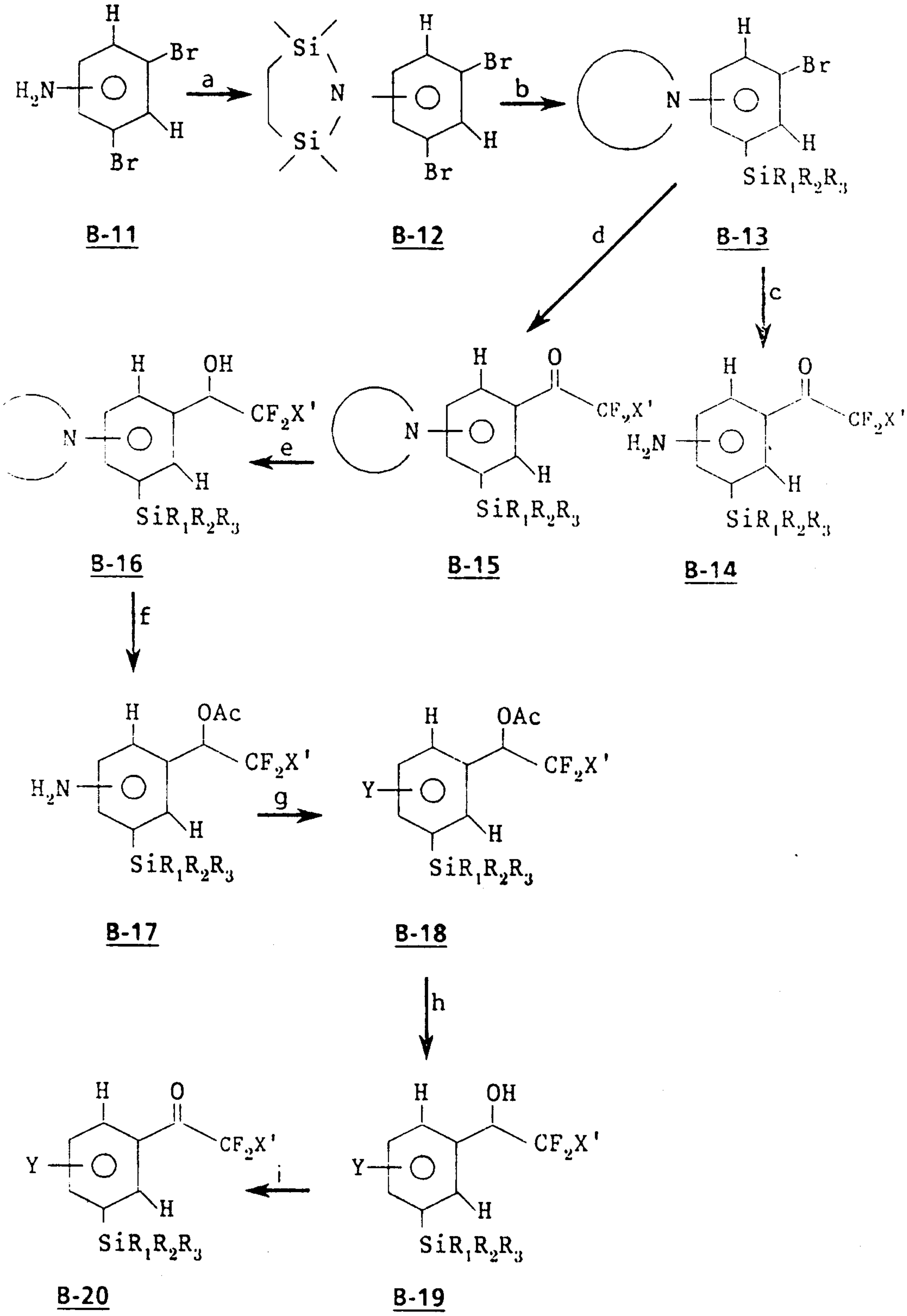


B-11

B-12

B-13

B-14

B-15

B-16

B-17

B-18

B-19

B-20

Again, in effecting the foregoing reactions, standard procedures analogously known in the art are employed. Step (B-a) entails the treatment of the amines (B-11) with one equivalent of butyl lithium in THF at about -30 °C to form an *in situ* intermediate which is reacted with one equivalent of 1,4-dichloro-1,1,4,4-tetramethyldisilylethylene at about -78 °C. The stabase adduct intermediates (B-12) are subjected to Step (B-b) using the silylation process of Step (A-a) and the resulting products (B-13) by Step (B-c) are

sequentially converted to their lithio derivatives by reaction with butyl lithium in diethylether at 0 °C for 15 minutes and these lithio derivatives are reacted with 3 equivalents of an acid, ester ($X'CF_2CO_2R$)-or a lithio salt thereof, or acid chloride ($X'CF_2COCl$) at -78 °C to obtain products (B-14). Alternatively using Step (B-d), which in principle follows Step (B-c), except that the hydrolysis is performed preferably using an aqueous ammonium chloride solution to produce compounds (B-15). Step (B-e) reduces the ketones of (B-15), preferably using sodium cyanoborohydride in ethanol followed by hydrolysis with a saturated aqueous ammonium chloride solution to produce the alcohols of (B-16). Using Step (B-f) the alcohols are protected by reaction with an acyl halide, preferably acetyl chloride, in the presence of triethylamine (TEA) in a solvent such as dichloromethane followed by an acid hydrolysis, preferably using 1N HCl to convert the stabase adduct to an amine (in the form of an ammonium salt) to obtain products (B-17). Step (B-g) involves a multiplicity of reactions which essentially are designed to effect Y-substitution on the phenyl ring of the involved compounds of (B-17). In general, these reactions involve the conversion of the ammonium salt to a diazonium salt (e.g., as with a reaction sodium nitrite or isoamyl nitrite in ice water) followed by treatment of the mixture with a metal salt or other derivative of Y to obtain the variety of Y-substituted derivatives. The Y-substituents involved are those as defined in formula I and include such moieties as OH, alkyl, alkoxy, hydroxy lower alkyl, amino alkyl, azido, cyano, carboxyl, $CO_2R_5$, $COR_5$, $SO_3H$, Br, Cl, F or $(CH_2)_xSiR_1R_2R_3$.

More specifically, Step (B-g) involves substitution of the (B-17) compounds with the foregoing enumerated Y-substituents. Preferably, the amine of B-17 is converted to $N_2^{\oplus}HSO_4^{\ominus}$ or $N_2^{\oplus}Cl^{\ominus}$ diazonium salts and by a variety of reactions the Y-substituted compounds of (B-18) are prepared. To prepare compounds wherein Y is OH, a so-prepared diazonium salt is dissolved in water, heated at about 80 to 120 °C to produce the desired hydroxy function which may be converted to its alkoxy derivative by alkylation in the presence of a base and the appropriate alkyl halide. To produce a fluoro derivative the diazonium salt (formed with $BF_4^{\ominus}N_2^{\oplus}$) is heated at 140 ° to 160 °C to obtain the desired fluoro derivatives.

For the chloro and bromo the diazonium sulfate or chloride salt is treated with cuprous bromide or chloride to obtain the desired bromo or chloro derivatives. To obtain the azide the diazonium salt is treated with sodium azide in water. The cyano derivative is obtained by treatment of the diazonium salt with cuprous cyanide. The carboxy moiety may be obtained by hydrolysis (in a basic aqueous medium) of the cyano moiety and, if desired, the acid may be esterified with the appropriate $R_5$ alcohol, in the presence of DCC and DMAP, to obtain the appropriate $-CO_2R_5$ moiety. The $-SO_3H$ moiety may be obtained by treating the diazonium sulfate salt with cupric chloride in the presence of HCl to obtain the $SO_2Cl$ moiety which, when treated with water, gives the desired $-SO_3H$ moiety. To obtain the desired $-COR_5$ moiety the diazonium salt is treated with an oxime [$R_5CH=NOH$] in the presence of copper sulfate and sodium sulfite, the resulting oxime is hydrolyzed to obtain the desired acyl moiety

$$(Y \text{ is } [O{=}C{-}R_5]),$$

the ketone of which is reduced with sodium borohydride and the resulting alcohol

$$(HO{-}CH{-}R_5)$$

is subjected to a Mitsunobu reaction to form a phthalamide which, upon treatment with hydrazine, is cleared to obtain the

$$H_2N{-}CH{-}R_5$$

amine. In those instances wherein Y is a $-SiR_1R_2R_3$ substituent the starting compound is a tribromobenzene which is treated as in Step (A-a) except that two equivalents of magnesium and $ClSiR_1R_2R_3$ are used to obtain the derivative corresponding to (B-16) which by Step (A-b) is converted to (B-20) wherein Y is a $-SiR_1R_2R_3$ moiety.

Following the formation of the Y-substituted compounds of formula (B-18), step (B-h) is employed to remove the alcohol-protecting group by hydrolysis (e.g., Ac) under basic or acidic conditions (taking in

consideration of the now-present Y-substituents) according to principles and techniques well-known and understood by those of ordinary skill in the art. The resulting alcohols (B-19) are subjected to Step (B-i) wherein the alcohol is oxidized with pyridinium dichromate or with the Dess-Martin periodate oxidant in dichloromethane or with the Swern reaction to produce compounds (B-20). Alternatively, the alcohols may be esterified, as described above, with an acyl halide.

In those instances wherein it is desired to prepare compounds of formula I represented by the sub-generic formula

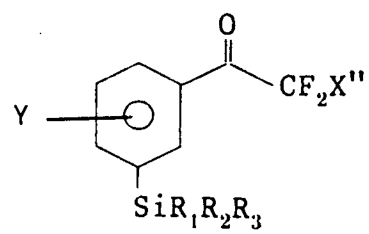

C-10

i.e., those compounds wherein n and m are zero, Y is H, and X″ is $COR_9$, $CO_2R_5$, $CONHR_5$ or $COR_6$, it is preferred to utilize the process of the following reaction scheme.

Reaction Scheme C

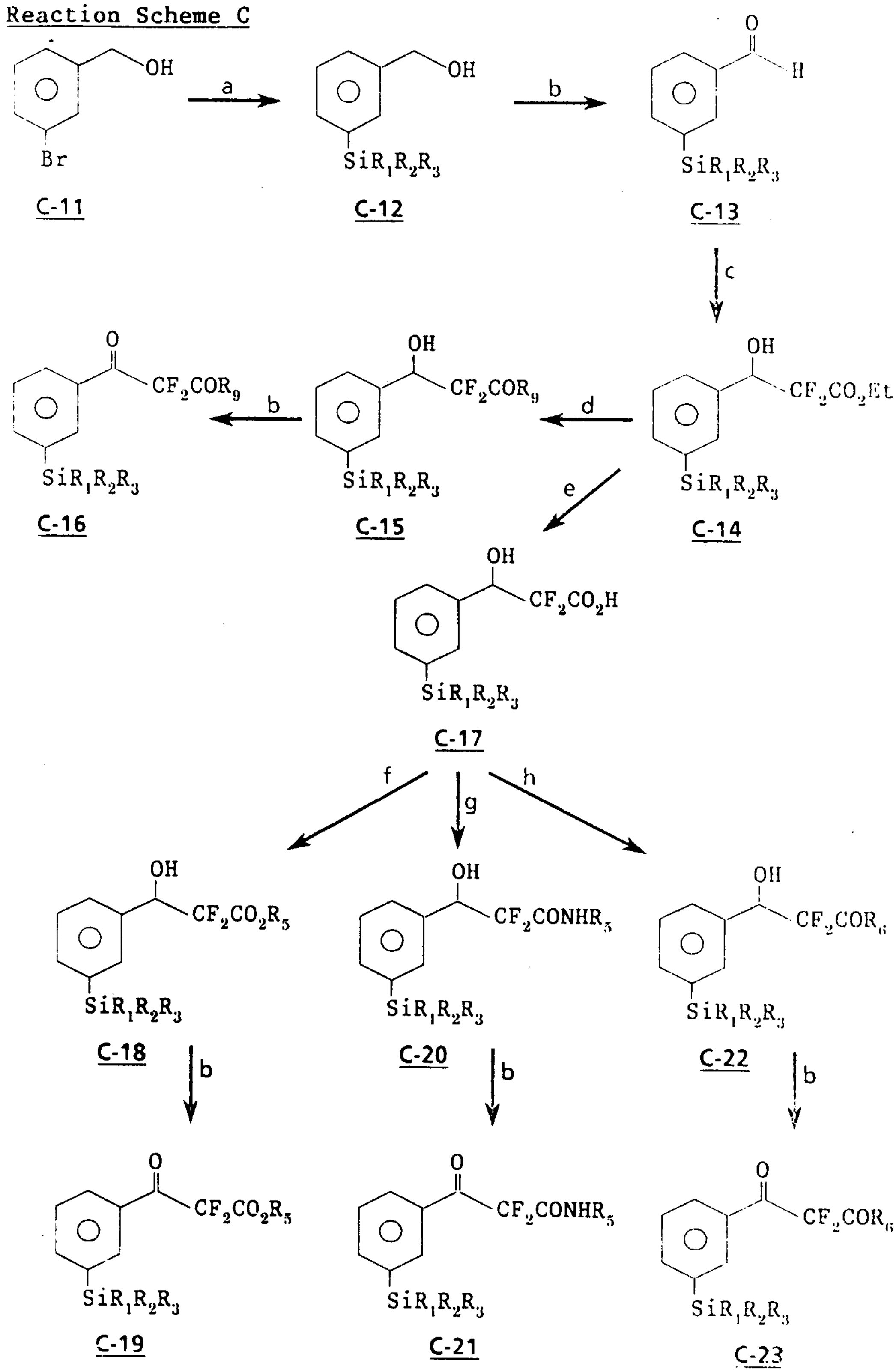


Step (C-a) involves the treatment of 3-bromobenzylalcohol (C-11) with 2 equivalents of butyl lithium in diethyl ether at 0°C, treat the resulting lithio derivatives with 2 equivalents of the desired $ClSiR_1R_2R_3$ and stir the resulting mixture at room temperature until the reaction is complete (generally 15-24 hours). Hydrolize, and after extraction, treat the crude product in 90% aqueous methanol for one hour at reflux temperature to obtain the silylated alcohols (C-12). Step (C-b) oxidizes the alcohols with pyridinium dichromate in dichloromethane to obtain the aldehydes (C-13) which, in Step (C-c), are treated with an alkyl

bromo difluoroacetate in THF in the presence of zinc at reflux temperatures to obtain intermediary hydroxy difluoroesters (C-14). In Step (C-d) these esters (C-14) are treated with two equivalents of a metallo derivative, preferably a lithium or magnesium derivative of the $R_9$ moiety, in diethyl ether or THF to obtain compounds (C-15) which, as in Step (C-b) are oxidized to their corresponding ketones (C-16) using pyridinium dichromate or the Swern or Dess-Martin reactions. Alternatively, using Step (C-e) the hydroxy difluoro esters of formula (C-14) may be hydrolized by treatment with lithium hydroxide in 80% aqueous ethylene glycol dimethylether to obtain the corresponding hydroxy difluoro acids (C-17). These acids, using Step (C-f) may be treated with an alcohol ($R_5OH$) in the presence of dicyclohexyl carbodiimide and 4-dimethylamino pyridine to obtain the esters (C-18). Alternatively, using Step (C-g) the acids (C-17) may be treated with an amine ($R_5NH_2$) in the presence of dicyclohexylcarbodiimide and 1-hydroxybenzotriazole to obtain the amides of formula (C-20). Using Step (C-h) the hydroxy difluoro acids (C-17) may also be reacted with a natural occurring amino acid, or a peptide $R_6H$ in the presence of dicyclohexylcarbodiimide and 1-hydroxy-benzotriazole to obtain the products of formula (C-22). In each instance, using the oxidation step of (C-b), the alcohols of (C-15), (C-18), (C-20) and (C-22) may be oxidized to the corresponding ketones of formulae (C-16), (C-19), (C-21) and (C-23), respectively, or they may be esterified, as described above, with an acyl halide.

In those instances wherein it is desired to prepare compounds of the subgeneric formula

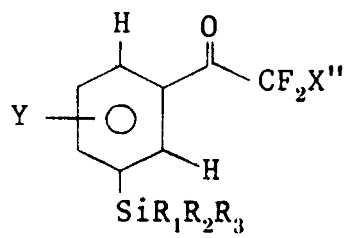

**D-10**

i.e., those compounds wherein n and m are zero,
Y is other than H,
X″ is $COR_9$, $CO_2R_5$, $CONHR_5$ or $COR_6$, the following reaction scheme is utilized.

Reaction Scheme D

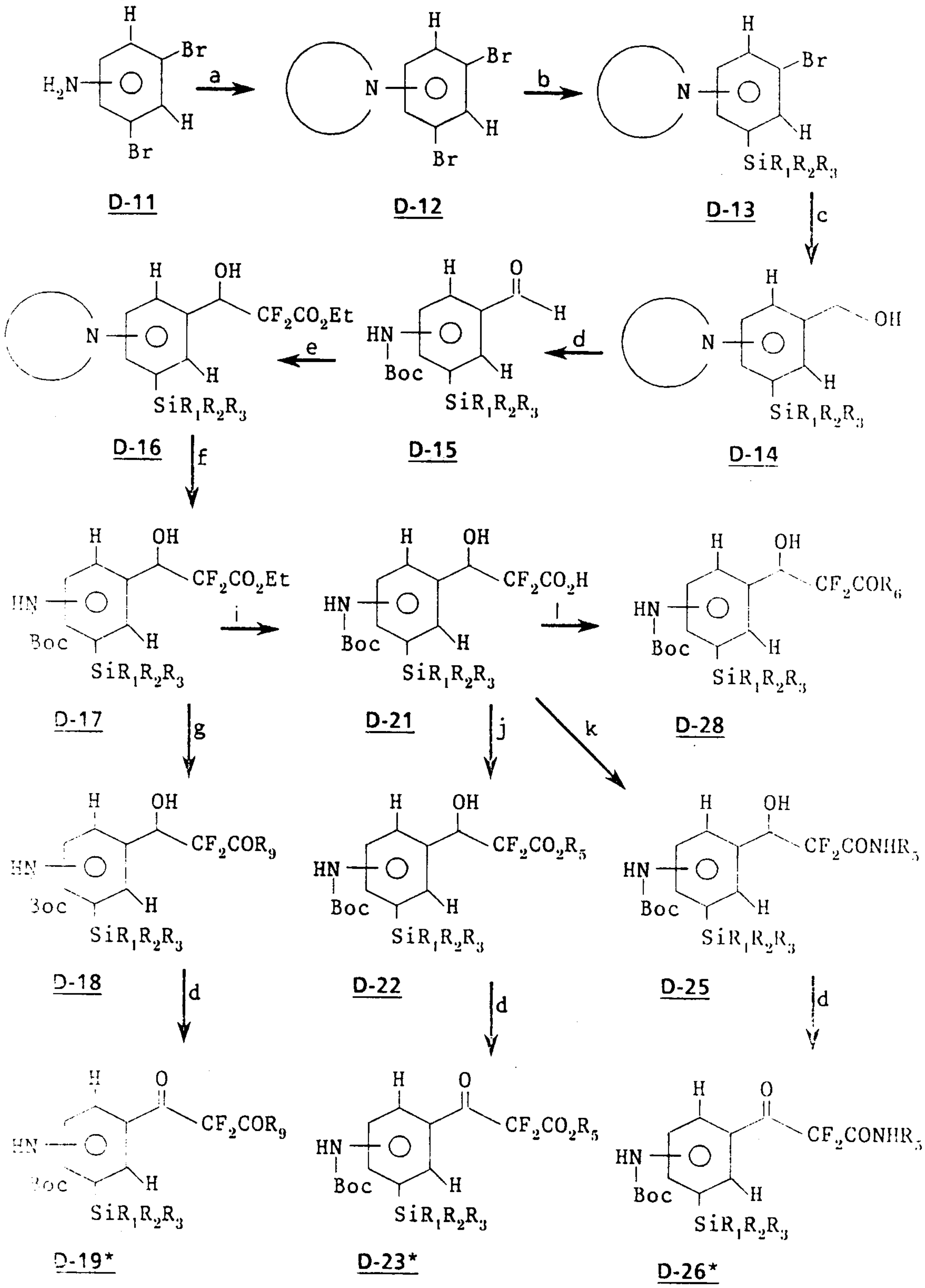


D-11 D-12 D-13 D-14 D-15 D-16 D-17 D-21 D-28 D-18 D-22 D-25 D-19* D-23* D-26*

Reaction.Scheme D (cont'd)

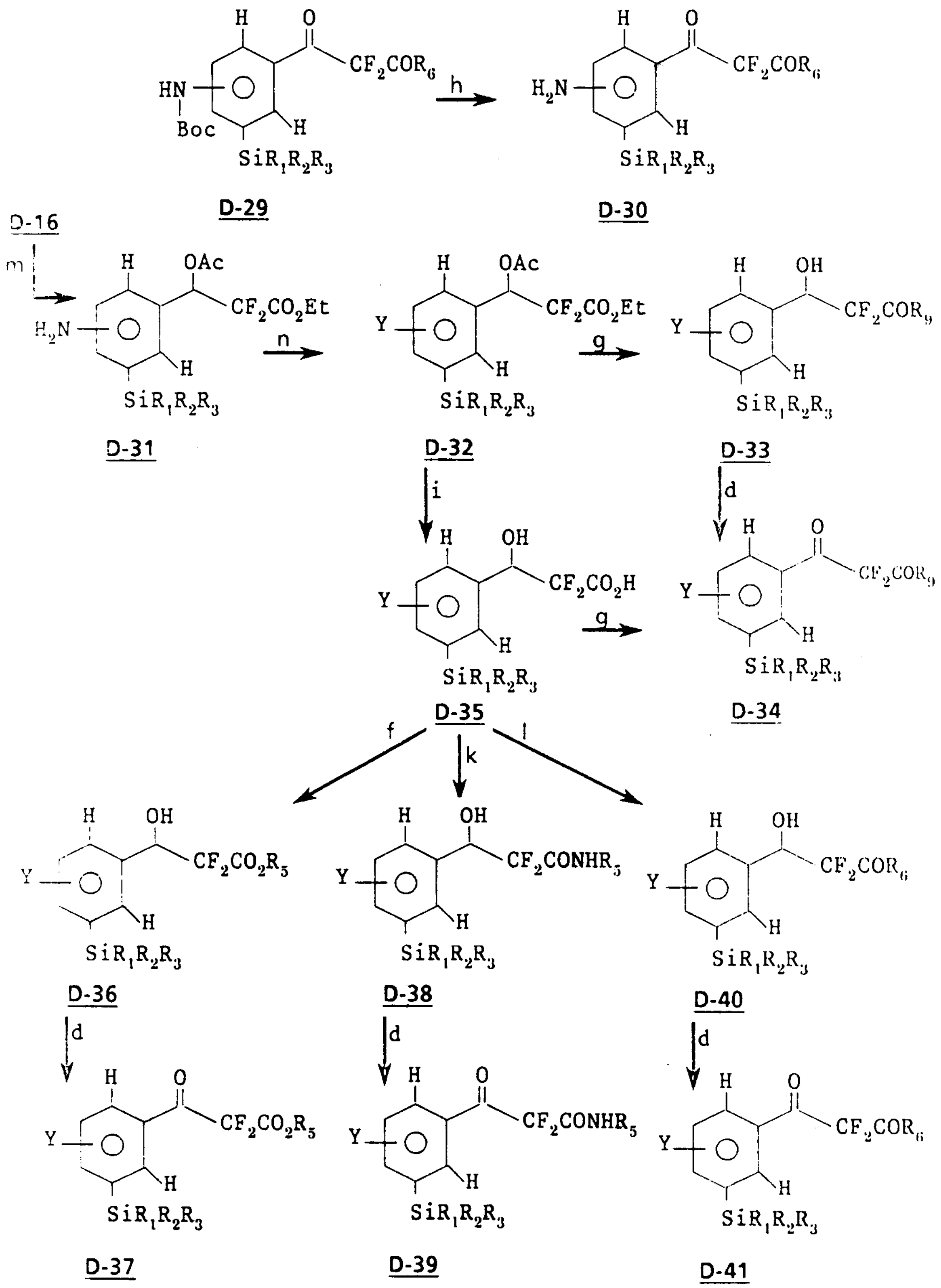


D-29

D-30

D-16

D-31

D-32

D-33

D-34

D-35

D-36

D-38

D-40

D-37

D-39

D-41

[*Compounds (D-19), (D-23), and (D-26) are de-Boced to desired compounds (D-20), (D-24), and (D-27) according to step D-h.]

Compound (D-13) is obtained according to Steps (D-a) and (D-b) using proceses (B-a) and (B-b).

The stabase adducts (D-13), using Step (D-c) are reacted with one equivalent of magnesium in diethylether and the resulting *in situ* intermediate treated with paraformaldehyde (followed by hydrolysis with saturated aqueous ammonium chloride solution) to form the appropriate hydroxymethyl compounds (D-14) which, using Step (D-d), are oxidized according to Step (C-b) and the resulting aldehydes (D-15) using Step (D-e) are converted to their esters (D-16) according to (C-c). Using Step (D-f) these esters are treated with 1N HCl to convert the stabase adduct to its amine and the amine is protected with a Boc protecting group to produce compounds (D-17). These intermediates [using Steps (D-g), (D-d) and (D-h)] are sequentially converted to compounds (D-18), (D-19) and (D-20) using the processes described for Step (C-d) (using 3 equivalents of the metallo derivatives of $R_5$), Step (C-b) and Step (D-h) which is treatment of the Boc derivatives with a solution of HCl in diethyl ether. Compounds (D-17) may also be sequentially converted to (D-21) by Step (D-i) [as described in Step C-e)], then by Step (D-f) to (D-22), then by Step (D-d) [as described in (C-b)] to (D-23), then by Step (D-h) to (D-24).

Compounds (D-21) also may be sequentially treated according to Step (D-k) (see C-g) to obtain (D-25) which are converted to (D-26) according to Step (D-d) (see C-b) and to (D-27) according to Step (D-h). Compounds (D-21) also may be sequentially treated according to Step (D-l) (see C-h) to produce (D-28) to (D-29) according to Step (D-d) (see C-b) and then to compounds (D-30) by Step (D-h).

From compound (D-16), compounds (D-34), (D-37), (D-39) and (D-41) may be obtained using the above described chemistry. The alcohols of (D-33), (D-36), (D-38), and (D-40) may be oxidized to their ketones or they may be converted to their esters as hereinabove described.

To prepare compounds of formula

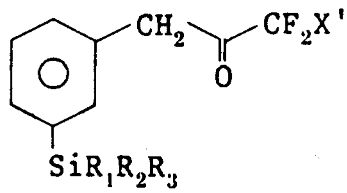

E-10

i.e., compounds wherein
n is one,
m is zero,
Y is H, and
X' is H, Br, Cl, F or $R_4$, the following reaction scheme is utilized

## Reaction Scheme E

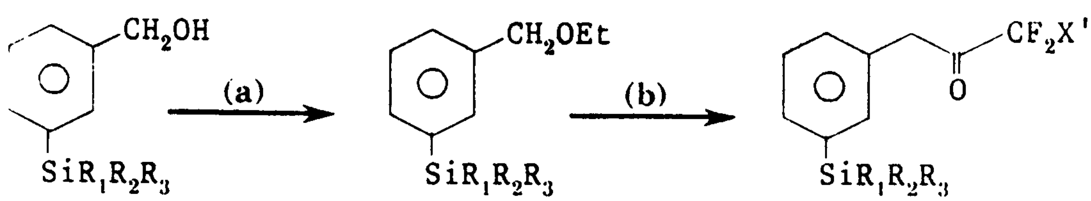


E-11 E-12 E-13

In this sequence, silylated compounds (E-11) are treated with n-butyllithium and then with ethyl iodide to produce compounds (E-12) which, by treatment with six equivalents of lithium in diethyl ether or THF at -10 °C, produce an *in situ* intermediate to which is added an acid, ester ($X'CF_2CO_2R$ wherein R is H or

$C_{1-6}$ alkyl) or an acid chloride ($X'CF_2COCl$), followed by hydrolysis with 1N HCl to obtain compounds (E-13). These ketones may be reduced with sodium borohydride or sodium cyano borohydride by reaction in ethanol followed by hydrolysis with saturated ammonium chloride and the resulting alcohols esterified with an acyl halide (ClC(O)R with R being H or $C_{1-10}$ alkyl), in the presence of TEA (or neat pyridine) in a suitable solvent such as dichloromethane; both these reactions being effected according to well known and understood reaction conditions.

To prepare compounds of formula

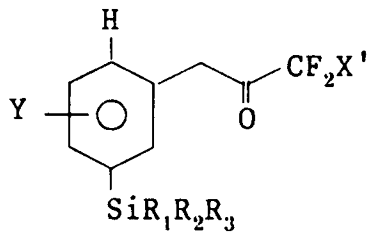

F-10

i.e., compounds wherein
n is one,
m is zero,
Y is other than H, and
X' is H, Br, Cl, F or $R_4$, the following reaction scheme is utilized.

Reaction Scheme F

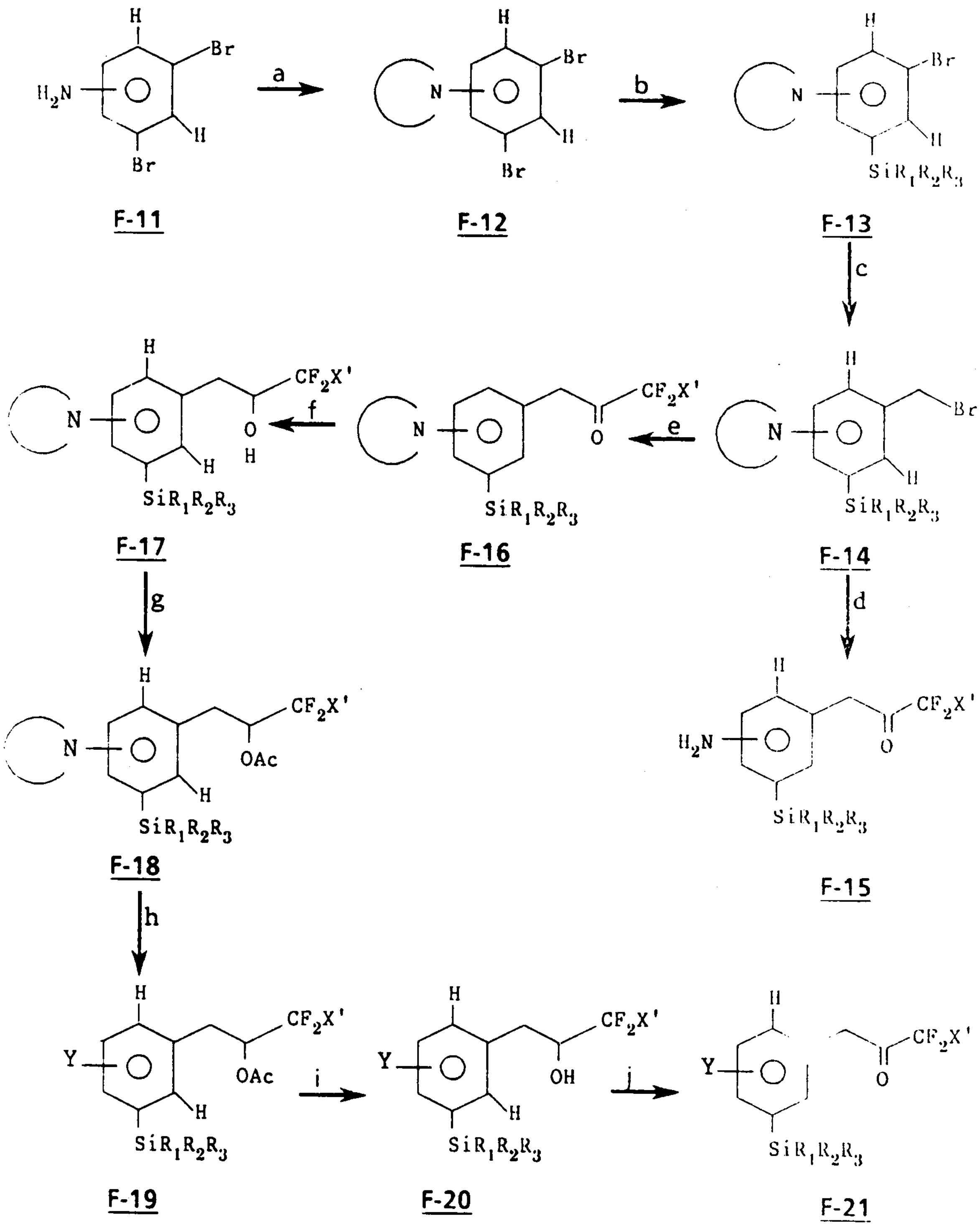


F-11 F-12 F-13 F-14 F-15 F-16 F-17 F-18 F-19 F-20 F-21

Step (F-a) forms the stabase adduct (F-12) with process (B-a).

Step (F-b) silylates (F-12) to (F-13) with process (A-a).

Step (F-c) treats ((F-13) with butyllithium at 0 ° C in ether or THF and then treats with dibromomethane to obtain (F-14).

Step (F-d) treats (F-14) with magnesium in diethyl ether or THF at reflux, then at -78 ° C intermediate is treated with ester ($X'CF_2CO_2R$) or acid chloride ($X'CF_2COCl$), followed by hydrolysis with 1N HCl to obtain (F-15).

Step (F-e) treats (F-14) as in Step (F-d) except the hydrolysis is effected with an ammonium chloride

solution to obtain (F-16).
Step (F-f) treats (F-16) according to process (B-e) to obtain (F-17).
Step (F-g) treats (F-17) according to process (B-f) to obtain (F-18).
Step (F-h) treats (F-18) according to process (B-g) to obtain (F-19).
Step (F-i) treats (F-19) according to process (B-h) to obtain (F-20).
Step (F-j) treats (F-20) according to process (B-i) to obtain (F-21). The alcohols (F-20) may also be esterified with an acyl halide as hereinabove described.

To prepare compounds of formula

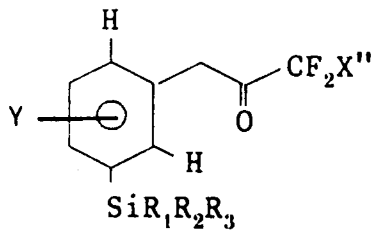

G-10

i.e, compounds wherein
n is one,
m is zero,
Y is H, and
X″ is $COR_9$, $CO_2R_5$, $CONHR_5$ or $COR_6$, the following reaction scheme is utilized.

Reaction Scheme G

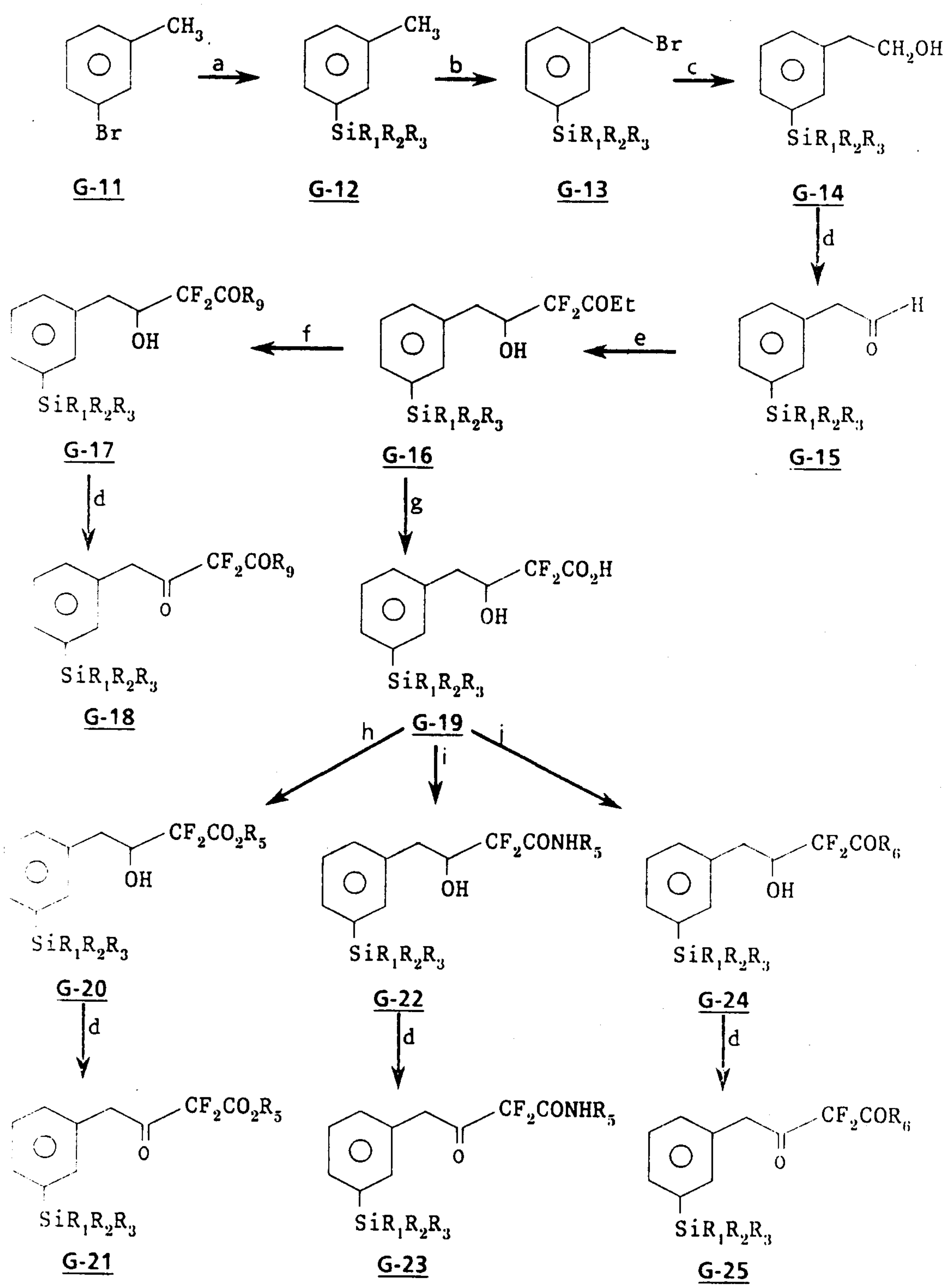


G-11 G-12 G-13 G-14 G-15 G-16 G-17 G-18 G-19 G-20 G-22 G-24 G-21 G-23 G-25

Step (G-a) silylates compounds (G-11) to form compounds (G-12) according to process (A-a).

Step (G-b) treats compounds (G-12) to form compounds (G-13) by treatment with one equivalent of N-bromosuccinamide in $CCl_4$ at reflux temperatures.

Step (G-c) treats compounds (G-13) according to process (D-c) to obtain compounds (G-14).

Step (G-d) treats compounds (G-14), (G-17), (G-20), (G-22) and (G-24) according to process (C-b) to obtain compounds (G-15), (G-18), (G-21), (G-23) and (G-25).

Step (G-e) treats compounds (G-15) according to process (C-c) to obtain compounds (G-16).
Step (G-f) treats compounds (G-16) according to process (C-d) to obtain compounds (G-17).
Step (G-g) treats compounds (G-16) according to process (C-e) to obtain compounds (G-19).
Step (G-h) treats compounds (G-19) according to process (C-f) to obtain compounds (G-20).
Step (G-i) treats compounds (G-19) according to process (C-g) to obtain products (G-22).
Step (G-j) treats compounds (G-19) according to process (C-h) to obtain compounds (G-24). The alcohols of (G-17), (G-21), (G-23), and (G-25) may also be esterified with an acyl halide as hereinabove described.

To prepare compounds of formula

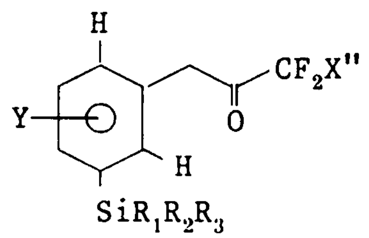

**H-10**

i.e., compounds wherein
n is one,
m is zero,
Y is other than H, and
X", is $COR_9$, $CO_2R_5$, $CONHR_5$ or $COR_6$, the following reaction scheme is utilized.

Reaction Scheme H

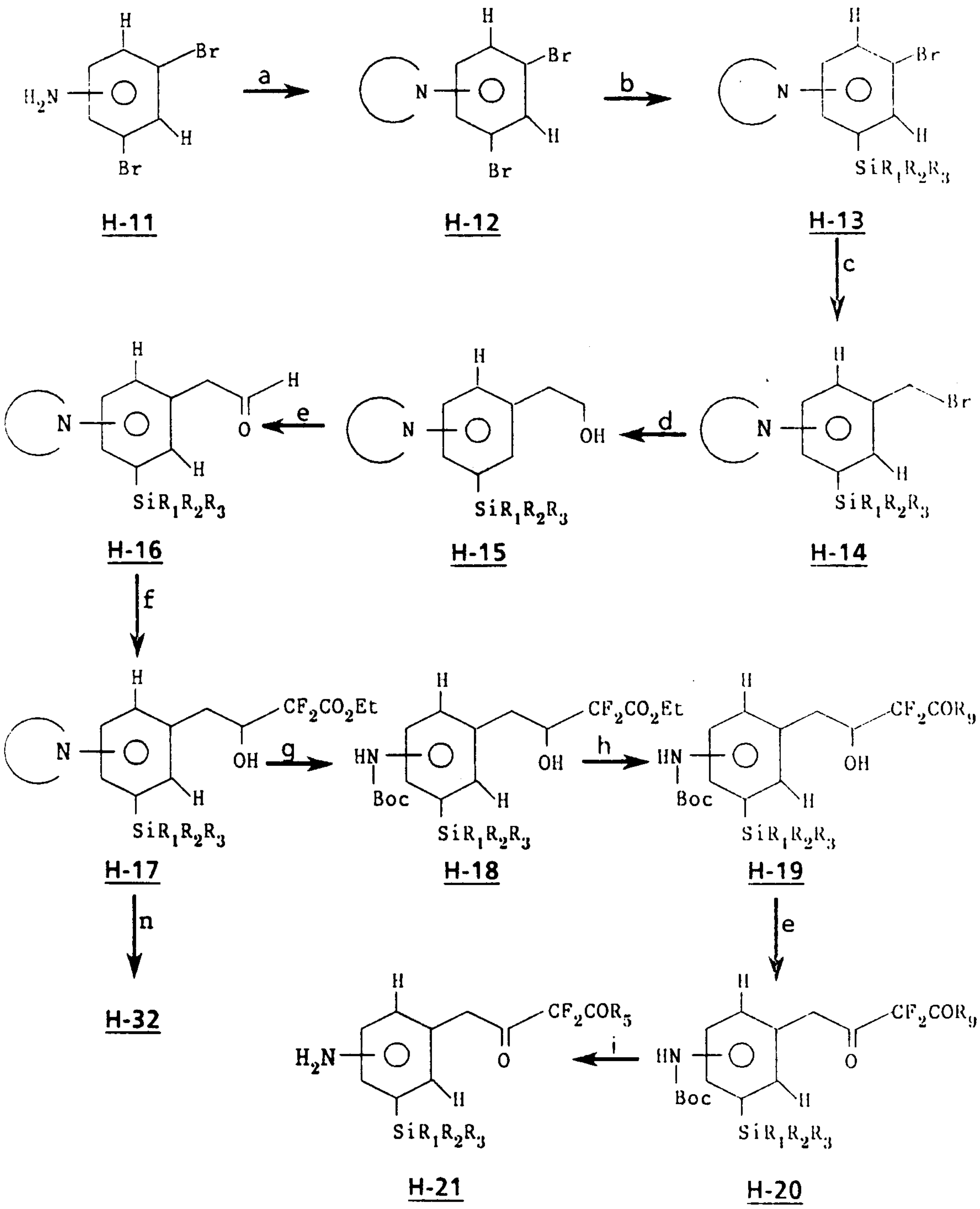


H-11 H-12 H-13

H-16 H-15 H-14

H-17 H-18 H-19

H-32 H-21 H-20

Reaction Scheme H (cont'd)

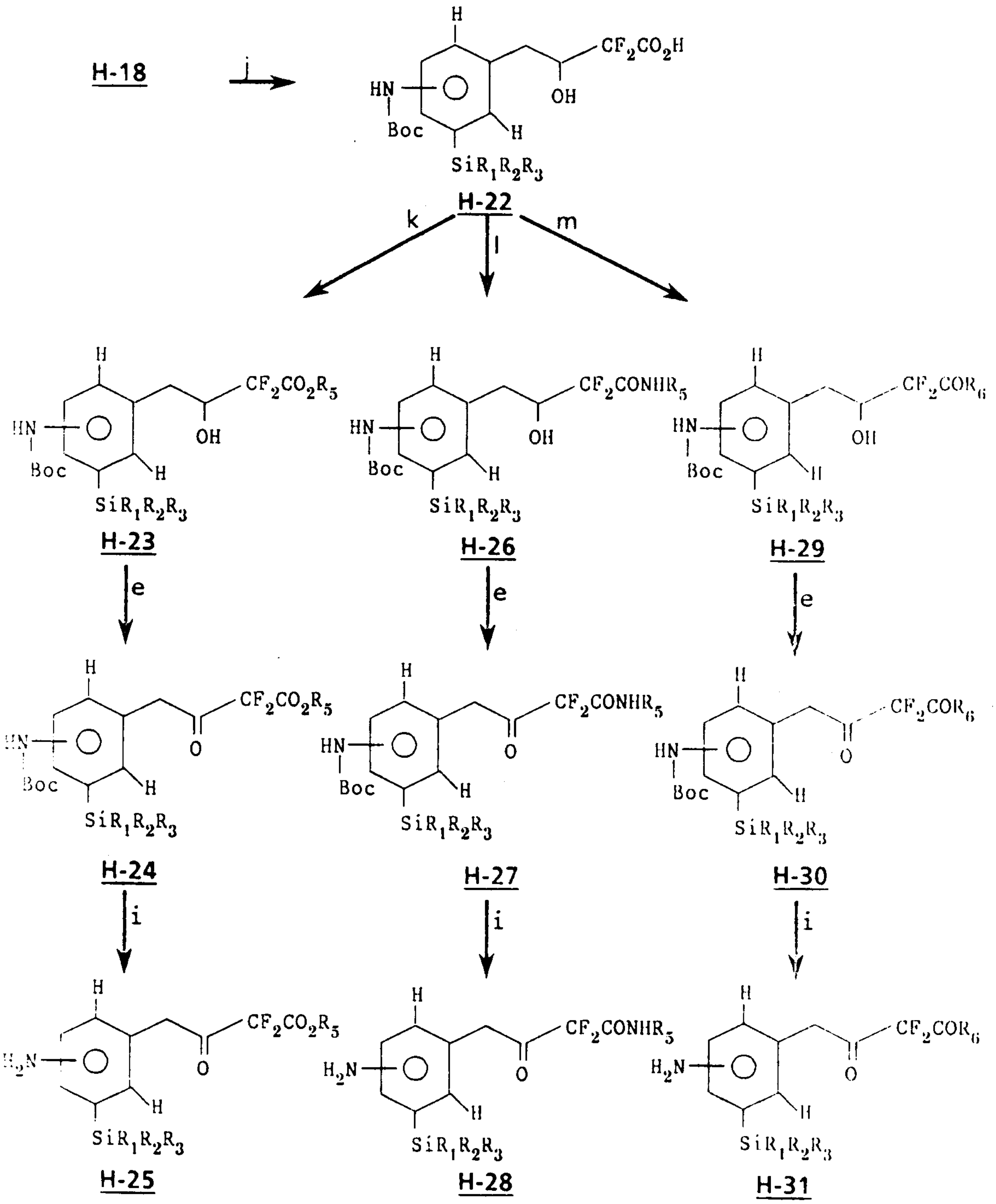


H-18

H-22

H-23 H-26 H-29

H-24 H-27 H-30

H-25 H-28 H-31

Reaction Scheme H (cont'd)

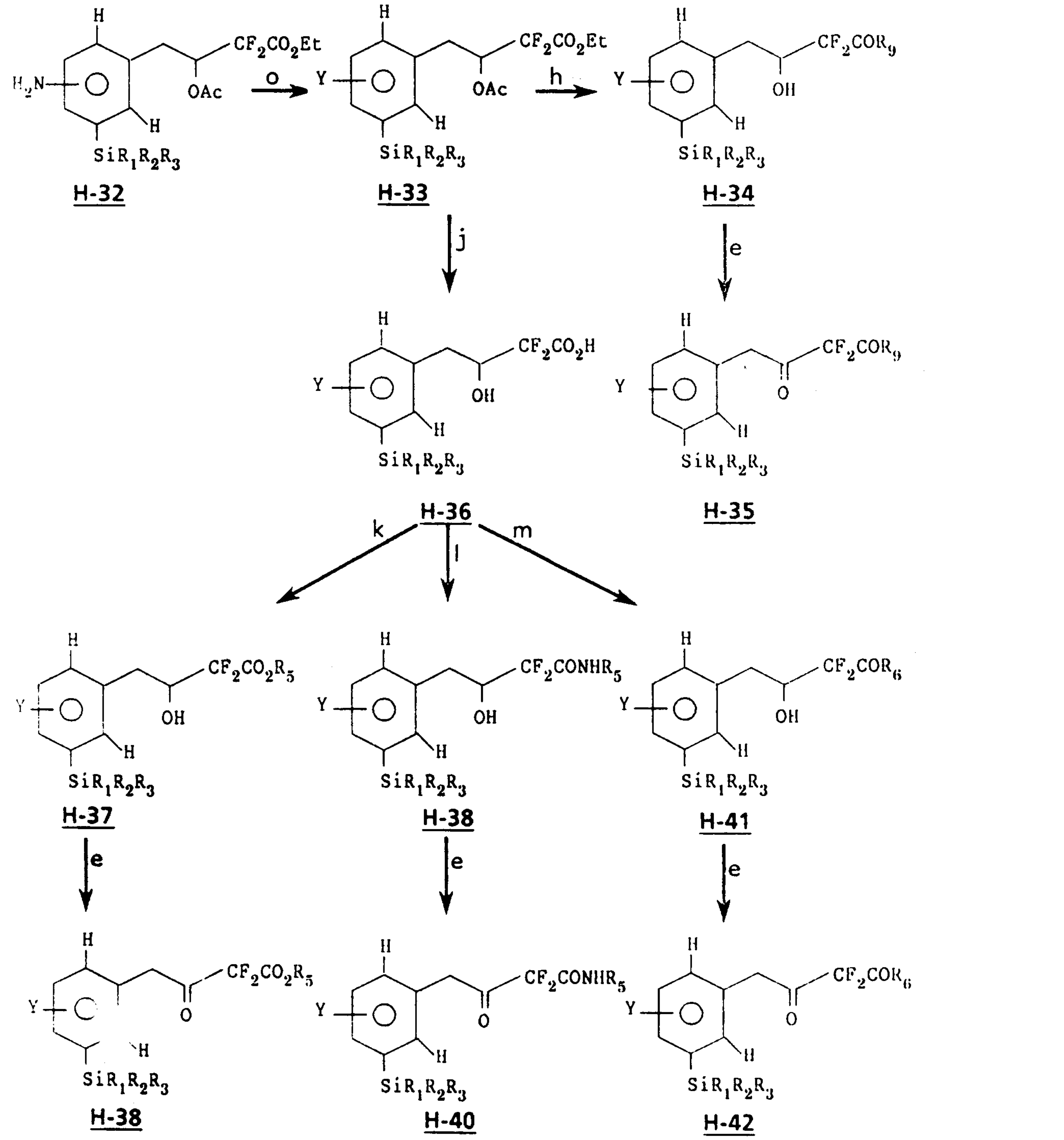


H-32, H-33, H-34, H-36, H-35, H-37, H-38, H-41, H-38, H-40, H-42

Step (H-a) converts compounds (H-11) to their stabase adducts according to the process of (B-a) to obtain compounds (H-12).

Step (H-b) converts compounds (H-12) to compounds (H-13) according to process (B-a).

Step (H-c) converts compounds (H-13) according to process (F-c) to obtain products (H-14).

Step (H-d) converts compounds (H-14) according to process (D-c) to obtain products (H-15).

Step (H-e) converts compounds (H-15), (H-19), (H-23), (H-26) and (H-29) according to process (C-b) to obtain products (H-16), (H-20), (H-24), (H-27) and (H-30), respectively.

Step (H-f) converts compounds (H-16) according to process (C-c) to obtain products (H-17).

Step (H-g) converts compounds (H-17) according to process (D-f) to obtain products (H-18).

Step (H-h) converts compounds (H-18) according to process (C-f) to obtain products (H-19).
Step (H-i) converts compounds (H-20), (H-24), (H-27) and (H-30) according to process (D-h) to obtain products (H-21), (H-25), (H-28) and (H-31), respectively.
Step (H-j) converts compounds (H-18) according to process (C-e) to obtain products (H-22).
Step (H-k) converts compounds (H-22) according to process (C-f) to obtain products (H-23).
Step (H-l) converts compounds (H-22) according to process (C-g) to obtain products (H-26).
Step (H-m) converts compounds (H-22) according to process (C-h) to obtain products (H-29).
Step (H-n) converts compounds (H-17) according to process (B-f) to obtain products (H-32).
Step (H-o) converts compounds (H-32) according to process (B-g) to obtain products (H-33).

Compound (H-33) is converted to compounds (H-35), (H-38), (H-40), (H-42) according to the appropriate above-described processes. The alcohols of (H-34), (H-37), (H-38), and (H-41) may also be esterified with an acyl halide as hereinabove describe.

To prepare compounds of formula

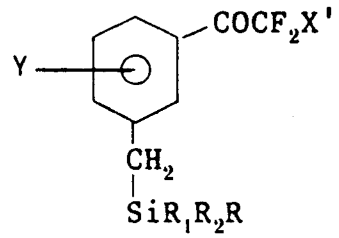

I-10

i.e., compounds wherein
n is zero,
m is one,
Y is H, and
X′ is H, Br, Cl, F or $R_4$, the following reaction scheme is utilized

## Reaction Scheme I

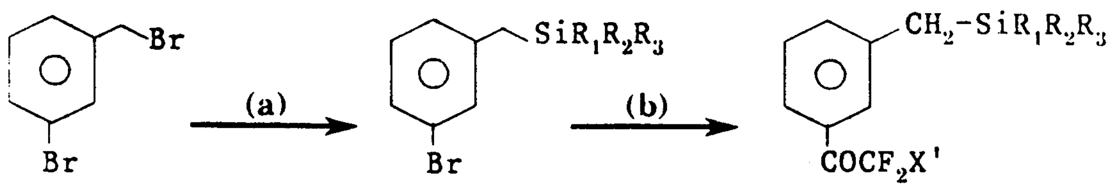


I-11 I-12 I-13

Step (I-a) converts compounds (I-11) according to process (A-a) to obtain products (I-12).
Step (I-b) converts compounds (I-12) according to process (A-b) to obtain products (I-13). The ketones may be reduced with sodium borohydride or sodium cyano borohydride by reaction in ethanol followed by hydrolysis with saturated ammonium chloride and the resulting alcohols esterified with an acyl halide (ClC-(O)R with R being H or $C_{1-10}$ alkyl), in the presence of TEA (or neat pyridine) in a suitable solvent such as dichloromethane; both these reactions being effected according to well known and understood reaction conditions.

To prepare compounds of formula

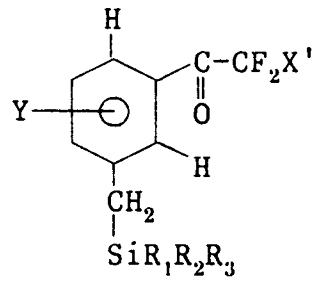

J-10

i.e., compounds wherein n is zero, m is one, Y is other than H, and X′ is H, Br, Cl, F or $R_4$, the following reaction scheme is utilized.

## Reaction Scheme J

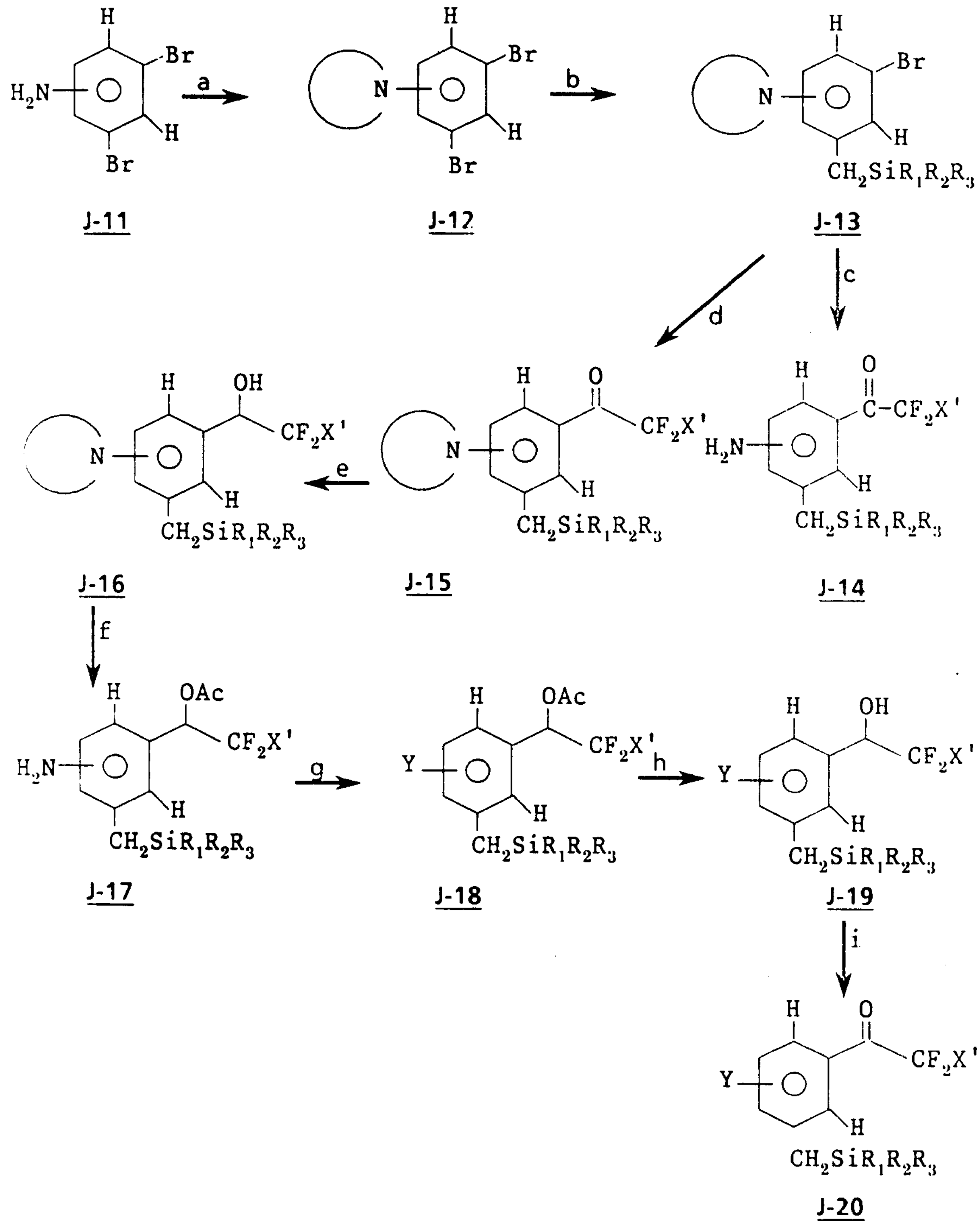


J-11 J-12 J-13 J-14 J-15 J-16 J-17 J-18 J-19 J-20

Step (J-a) converts compounds (J-11) to their stabase adduct as described in (B-a).

Step (J-b) converts compounds (J-12) to their silylated derivatives (J-13) using one equivalent of $ICH_2SiR_1R_2R_3$ after treatment of (J-12) with butyl lithium in ether at 0 °C as in above-described silylating procedures.

Step (J-c) converts compounds (J-13) to (J-14) using procedures analogous to Step (B-c).

Step (J-d) converts compounds (J-13) to (J-15) using procedures analogous to Step (B-d).

Step (J-e) converts compounds (J-15) to (J-16) using procedures analogous to Step (B-e).

Step (J-f) converts compounds (J-16) to (J-17) using procedures analogous to Step (B-f).

Step (J-g) converts compounds (J-17) to (J-18) using procedures analogous to Step (B-g).
Step (J-h) converts compounds (J-18) to (J-19) using procedures analogous to Step (B-h).
Step (J-i) converts compounds (J-19) to (J-20) using procedures analogous to Step (B-i). The alcohols (J-19) may also be esterified, as described above, with an acyl halide.

To prepare compounds of formula

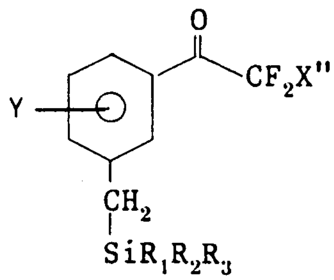

**K-10**

wherein m is one,
n is zero,
Y is H, and
X″ is $COR_9$, $CO_2R_5$, $CONHR_5$ or $COR_6$, the following reaction scheme is utilized.

Reaction Scheme K

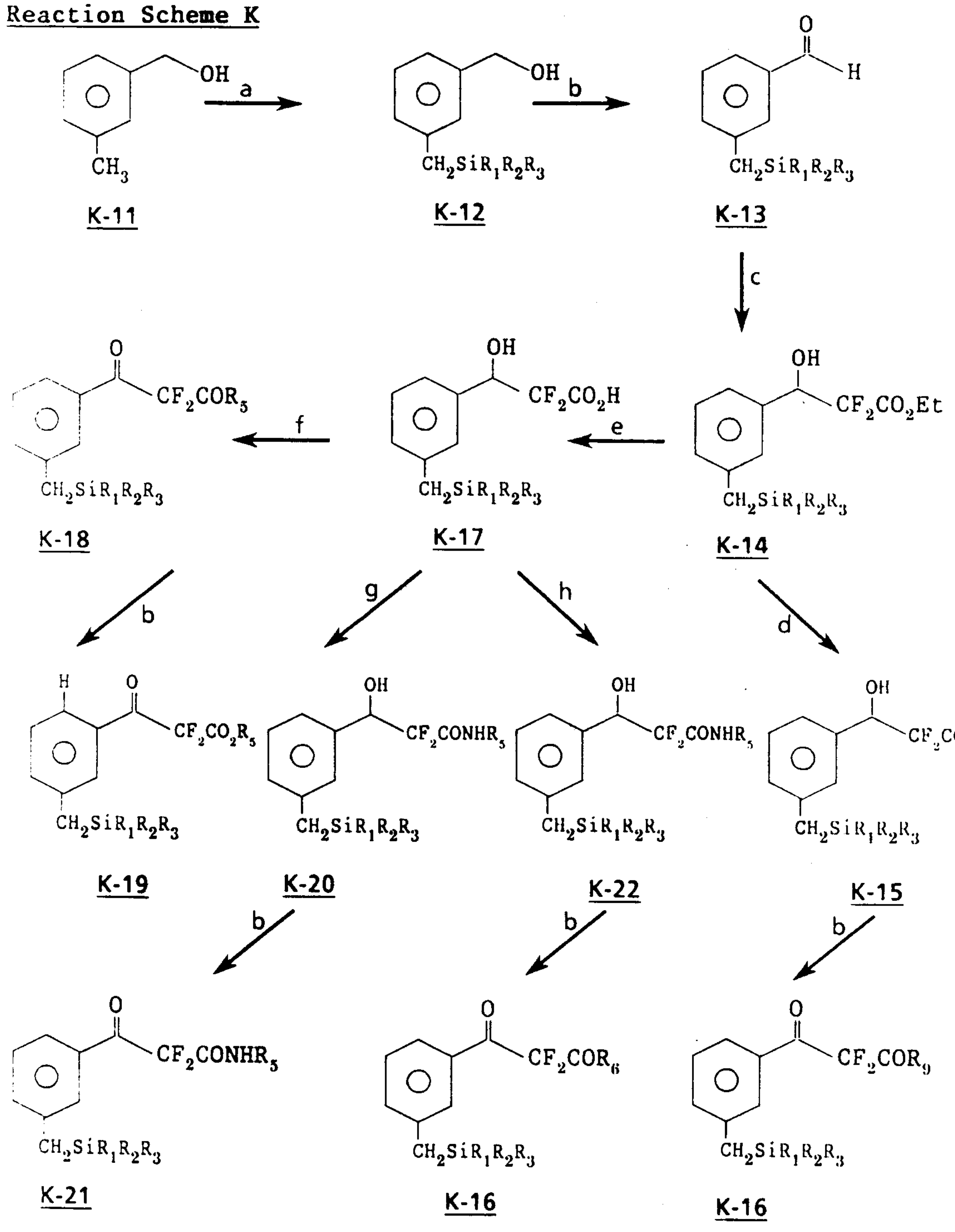


K-11, K-12, K-13, K-14, K-17, K-18, K-19, K-20, K-22, K-15, K-21, K-16, K-16

Step (K-a) converts 3-methylbenzyl alcohol (K-11) to (K-12) by treatment with 3 equivalents of butyllithium at -20 °C in diglyme, followed by treatment with 3 equivalents of $ClSiR_1R_2R_3$ and triethylamine at room temperature, followed by refluxing the extracted crude product in 90% aqueous methanol to obtain the desired silylated product.

Step (K-b) converts (K-12) to compounds (K-13) using procedures analogous to (C-b).

Step (K-c) converts (K-13) to compounds (K-14) using procedures analogous to (C-c).

Step (K-e) converts (K-14) to compounds (K-17) using procedures analogous to (C-e).

Steps (K-f) and (K-b) using procedures analogous to (C-f) and (C-b) produce (K-19).

Steps (K-g) and (K-b) using procedures analogous to (C-g) and (C-b) produce (K-21).

Steps (K-h) and (K-b) using procedures analogous to (C-g) and (C-b) produce (K-23).

Steps (K-d) and (K-b) using procedures analogous to (C-d) and (C-b) produce (K-16). The alcohols of (K-14), (K-15), (K-18), (K-20), and (K-22) may also be esterified, as described above, with an acyl halide.

To prepare compounds of formula I represented by the subgeneric formula

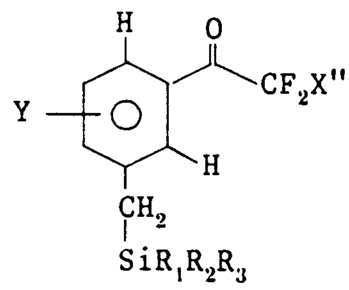

L-10

i.e., compounds wherein
m is one,
n is zero,
Y is other than H, and
X" is $COR_9$, $CO_2R_5$, $CONHR_5$ or $COR_6$, the following reaction scheme is utilized.

Reaction Scheme L

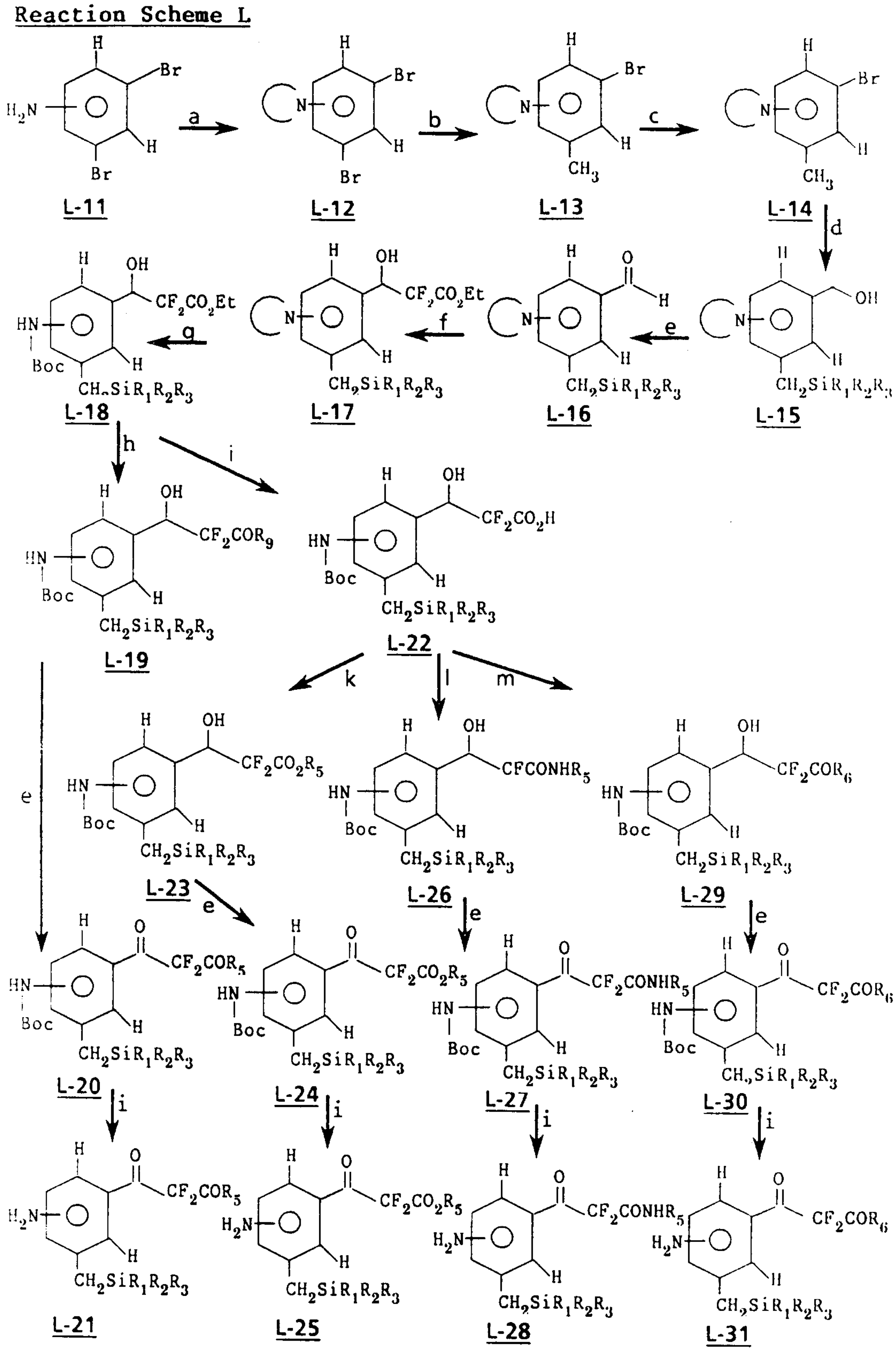


L-11 L-12 L-13 L-14

L-18 L-17 L-16 L-15

L-19 L-22

L-23 L-26 L-29

L-20 L-24 L-27 L-30

L-21 L-25 L-28 L-31

Reaction Scheme L (cont'd)

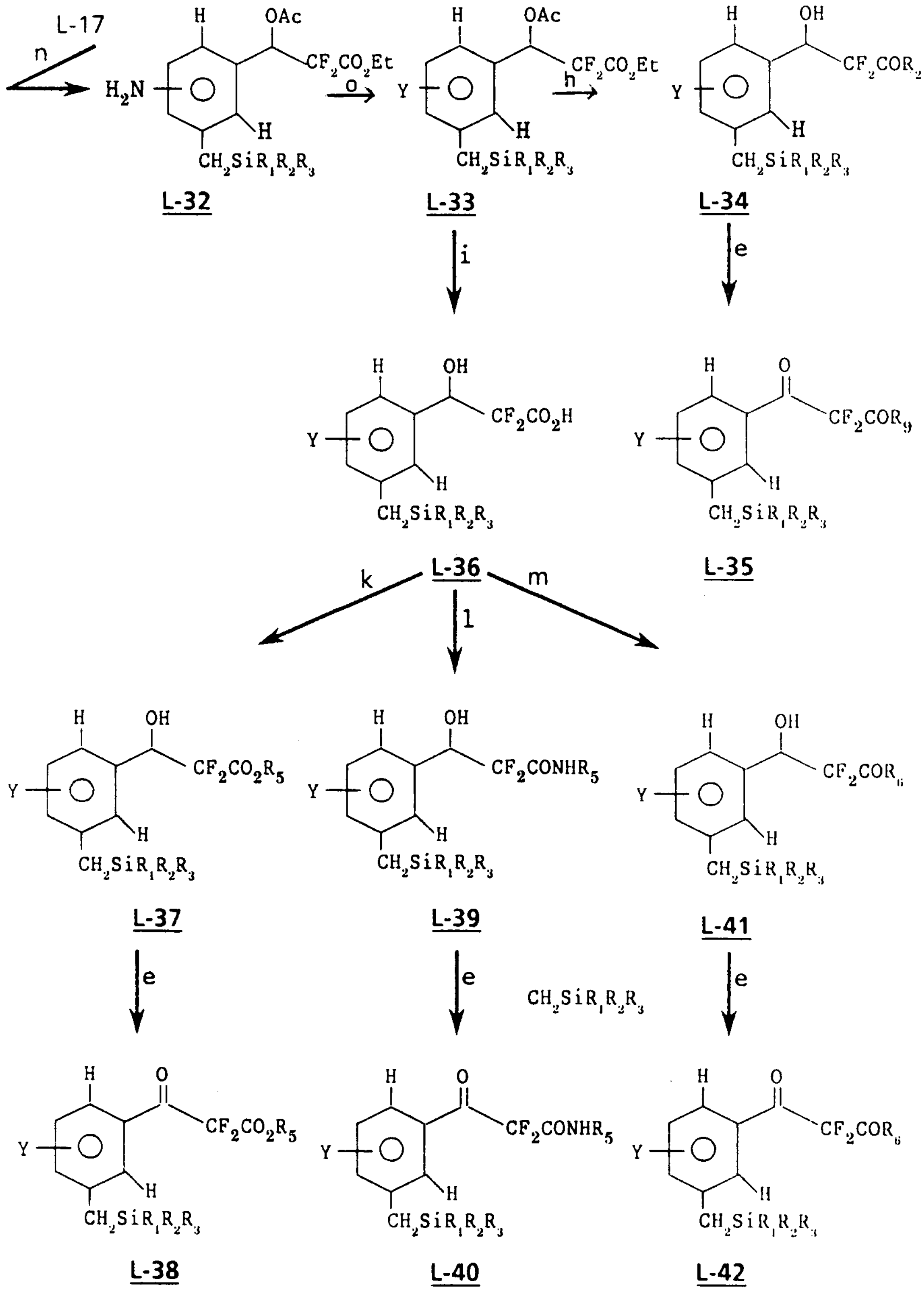


L-32 L-33 L-34 L-35 L-36 L-37 L-39 L-41 L-38 L-40 L-42

Step (L-a) convert (L-11) to (L-12) according to a process analogous to (B-a).

Step (L-b) converts (L-12) to (L-13) by treating the stabase adduct with one equivalent of butyllithium in diethyl ether at 0 ° C followed by reaction with one equivalent of $CH_3I$.

Step (L-c) converts (L-13), to (L-14) using procedures analogous to (D-c).

Step (L-d) converts (L-14) to (L-15) using procedures analogous to (K-a).

From (L-15), using procedures analogous to procedures (D-d), (D-e), (D-f), (D-g) and (D-i), there is produced

(L-19) and (L-22).

From (L-18), using procedures analogous to (K-d) there is produced (L-20).

From (L-19) and (L-22), using the procedures analogous to that for obtaining compounds (D-20), (D-24), (D-27) and (D-30), there are obtained compounds (L-25), (L-28), (L-31) and (L-21). Similarly starting with compound (L-17) and by analogously applying the chemical process reactions performed on (D-6) and its resulting subsequent derivatives to prepare compounds (D-34), (D-37), (D-39) and (D-41) there are also produced compounds (L-35), (L-38), (L-40) and (L-42). The alcohols(L-34), (L-37), (L-39), (L-41) may also be esterified as hereinabove described.

As illustrated in structure (B-12) the stabase moiety is depicted as

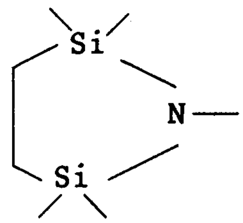

For convenience and following generally accepted practice, that moiety is thereafter abbreviated as

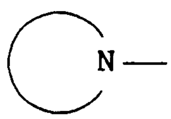

In all instances wherein the abbreviated form is utilized it is fully intended to designate that moiety as depicted in structure (B-12).

Having generically described the methods for the preparation of the compounds of this invention, the following specific examples illustrate the chemistry and techniques by which the synthesis may be effected.

EXAMPLE 1

**2,2,2-Trifluoro-1-(3-trimethylsilylphenyl) ethanone**

Step A:

3-Trimethylsilyl-bromobenzene

A mixture of 1,3-dibromobenzene (25.0 g, 106.4 mmol) and trimethylsilylchloride (11.6 g, 106.4 mmol) in diethyl ether (50 ml) was added dropwise in 1½ hours on magnesium (2.59 g, 106.4 mmol) in diethyl ether (25 ml). Then the mixture was refluxed for 18 hours, cooled to 0 °C, treated with 4N HCl (75 ml). The organic layer was separated, washed with water, brine, dried over $MgSO_4$ and concentrated. 3-Trimethylsilylbromobenzenewas obtained by fractional distillation as a colorless oil (13.4 g, 55% yield, b.p.: 55-62 °C (0.8 mmHg).

Step B:

2,2,2-Trifluoro-1-(3-trimethylsilylphenyl) ethanone

To a solution of 3-trimethylsilyl-bromobenzene (7.62 g, 33.3 mmol) in diethyl ether (35 ml) was added at 0 °C 1.5M n-butyllithium in hexane (22.2 ml, 33.3 mmol) over 10 min. Then the mixture was allowed to react 15 min at room temperature, cooled to -78 °C and ethyltrifluoroacetate (14.2 g, 100 mmol) was added over 5 min. Then the mixture was allowed to react 15 min at -78 °C, the cooling bath was removed and when the temperature rose to 0 °C 3N HCl (35 ml) was added dropwise. The organic layer was separated , washed with water, brine, dried over $MgSO_4$ and concentrated. Chromatography (silica gel, cyclohexane/diethyl

ether: 90/10) followed by distillation under reduced pressure afforded the expected compound as a colorless oil (4.32 g, 53% yield), b.p. 120 °C, Rf: 0.28 (cyclohexane/diethyl ether: 95/5).

EXAMPLE 2

**1-[(4-Methoxy-3-trimethylsilyl)phenyl]-2,2,2-trifluoroethanone**

Step A:

4-Bromo-3-trimethylsilylanisole

To a solution of 2,4-dibromoanisole (13.3 g, 50 mmol) in diethyl ether (50 ml) at 0 °C was added over 20 min 1.5M to butyllithium (33.4 ml) and the reaction was allowed to react 15 min at 0 °C. Then trimethylsilylchloride (5.43 g, 50 mmol) in diethyl ether (20 ml) was added over 10 min and the resulting mixture was stirred 18 hours at room temperature. At 0 °C 3N HCl (50 ml) was added and the organic layer was separated, washed with water, brine, dried over $MgSO_4$ and concentrated. 4-Bromo-3-trimethylsilylanisole was purified by chromatography on silica gel (eluted with cyclohexane) as a colorless oil.

Step B:

1-[(4-Methoxy-3-trimethylsilyl)phenyl]-2,2,2-trifluoroethanone

The title compound was prepared as described in Step B of Example 1. Chromatography on silica gel (cyclohexane/diethyl ether: 95/5) afforded a colorless oil (59% yield).

EXAMPLE 3

**1-[(4-Hydroxy-3-trimethylsilyl)phenyl]-2,2,2-trifluoroethanone**

Step A:

4-Bromo-3-trimethylsilylphenol

To a solution of 2,4-dibromophenol (8.0 g, 31.75 mmol) in diethyl ether (35 ml) at 0 °C was added over 50 min 1.5M butyllithium (42.3 ml) and the mixture was stirred 2½ hours at room temperature. At 0 °C 3N HCl (100 ml) was added, the organic layer was separated, washed with water, brine, dried over $MgSO_4$ and concentrated. The crude product was dissolved in 90% aqueous methanol (50 ml) and refluxed 2 hours, then the solvents were removed and the title compound was purified by chromatography on silica gel (10% ethyl acetate in cyclohexane).

Step B:

1-[(4-Hydroxy-3-trimethylsilyl)phenyl]-2,2,2-trifluoroethanone

To a solution of 4-bromo-3-trimethylsilylphenol (2.54 g, 10 mmol) in diethyl ether (10 ml) at 0 °C was added 1.5M butyllithium (13.4 ml) over 10 min and the mixture was stirred at 0 °C 15 min. At -78 °C was added ethyl trifluoroacetate (5.68 g, 40 mmol) in diethyl ether (10 ml), then the mixture was allowed to warm up to room temperature and was stirred 1 hour. At 0 °C 3N HCl (20 ml) was added, tlie organic layer separated, washed with water and concentrated. The crude product was dissolved in tetrahydrofuran (20 ml) and stirred 1 hour with aqueous sodium hydrogen carbonate (10 ml). The mixture was extracted with diethyl ether and the extract washed with water, brine, dried over $MgSO_4$ and concentrated. The title compound was purified by chromatography on silica gel (20% ethyl acetate in cyclohexane), followed by recrystallization in $CCl_4$. m.p. 178 °C, Rf: 0.50 (cyclohexane/ diethyl ether: 50/50).

EXAMPLE 4

**1-(3,5-Bis-trimethylsilylphenyl)-2,2,2-trifluoroethanone**

Step A:

3,5-Bis-trimethylsilyl-bromobenzene

A mixture of 1,3,5-tribromobenzene (10.0 g, 31.75 mmol) and trimethylsilylchloride (6.90 g, 63.50 mmol) in diethyl ether (35 ml) was added dropwise in 1 hour on magnesium (1.55 g, 63.50 mmol) in diethyl ether (20 ml). Then the mixture was refluxed 18 hours, cooled to 0 ° C, treated with 4N HCl (50 ml). The organic layer was separated, washed with water, brine, dried over $MgSO_4$ and concentrated. 3,5-Bis-trimethylsilyl-bromobenzene was purified by chromatography on silica gel (eluted with heptane) as a colorless oil (5.39 g, 56% yield).

Step B:

1-(3,5-Bis-trimethylsilylphenyl)-2,2,2-trifluoroethanone

The title compound was prepared as described in Step B of Example 1 in 35% yield. b.p. 170 ° C (26 mmHg).

EXAMPLE 5

**Ethyl-2,2-difluoro-3-keto-3-(3-trimethylsilyl)phenylpropionate**

Step A:

3-Trimethylsilylbenzylalcohol

To a solution of 3-bromobenzylalcohol (9.35 g, 50 mmol) in diethyl ether (50 ml) at 0 ° C was added dropwise 1.5M butyllithium (66.7 ml) and the mixture was stirred 30 min. Then trimethylsilylchloride (11.39 g, 105 mmol) in diethyl ether (50 ml) was added dropwise and the mixture was stirred at room temperature 18 hours. The reaction mixture was treated with ice water (100 ml) and the organic layer extracted, washed with water, brine, dried over $MgSO_4$ and concentrated. The crude product was dissolved in 90% aqueous methanol (100 ml) and refluxed 1 hour. The solvents were removed and 3-trimethylsilylbenzylalcohol was purified by distillation.

Step B:

3-Trimethylsilylbenzaldehyde

To a mixture of 3-trimethylsilylbenzylalcohol (3.60 g, 20 mmol) and pyridinium dichromate (11.29 g, 30 mmol) in dichloromethane (60 ml) at 0 ° C was added 3Å molecular sieve powder (16 g) and anhydrous acetic acid (2 ml). Then the reaction was allowed to react 30 min at room temperature, stirred 20 min with celite (10 g), filtered and evaporated under reduced pressure. The residue was treated with diethyl ether (50 ml), filtered through $MgSO_4$ and concentrated. 3-trimethylsilylbenzaldehyde was purified by distillation.

Step C:

Ethyl-2,2-difluoro-3-hydroxy-3-(3-trimethylsilyl)phenylpropionate

A solution of 3-trimethylsilylbenzaldehyde (1.78 g, 10 mmol) and ethyl bromodifluoroacetate (2.23 g, 11 mmol) in tetrahydrofuran (10 ml) was added dropwise on zinc wool (7.85 g, 12 mmol) in refluxing tetrahydrofuran (10 ml). Then the reaction was allowed to react 1 hour, cooled, hydrolysed with a saturated ammonium chloride solution (20 ml) and diluted with diethyl ether (20 ml). The organic layer was washed with brine, dried over $MgSO_4$ and concentrated. The title compound was purified on silica gel (20% ethyl acetate in cyclohexane).

Step D:

Ethyl-2,2-difluoro-3-keto-3-(3-trimethylsilyl)phenylpropionate

The title compound was prepared as described in Step B and purified by distillation.

EXAMPLE 6

**2,2-Difluoro-3-keto-3-(5-nitrile-3-trimethylsilyl)phenyl propanoic acid**

Step A:

3,5-Dibromo-N,N-(1,1,4,4-tetramethyl-1,4-disilylethylene)aniline

To a solution of 3,5-dibromoaniline (25,10 g, 100 mmol) in tetrahydrofuran (100 ml) at -30 °C was added 1.5M butyllithium (66.67 ml). Then at -78 °C was added a solution of 1,1,4,4-tetramethyl-1,4-dichlorosilylethylene (21.50 g, 100 mmol) in tetrahydrofuran (100 ml) and the mixture was allowed to warm at room temperature and stirred 1 hour. The mixture was poured into water (200 ml), diluted with diethyl ether (100 ml) and the ether layer separated. The aqueous layer was washed twice with diethyl ether and the etheral extracts combined, dried over $MgSO_4$ and the solvents removed under reduced pressure. The title compound was recrystallized from hexane.

Step B:

3-Bromo-5-trimethylsilyl-N,N-(1,1,4,4-tetramethyl-1,4-disilylethylene)aniline

The title compound was prepared as described in Step A of Example 1, except for hydrolysis which was made with a saturated ammonium chloride solution. Purification was achieved by recrystallization from hexane.

Step C:

3-Trimethylsilyl-5-[N,N-(1,1,4,4-tetramethyl-1,4-disilylethylene)]aminobenzylalcohol

To a solution of 3-trimethylsilyl-5-[N,N-(1,1,4,4-tetramethyl-1,4-disilylethylene)]aminophenyl magnesium bromide prepared from 3-bromo-5-trimethylsilyl-N,N-(1,1,4,4-tetramethyl-1,4- disilylethylene)aniline (11.26 g, 30 mmol) and magnesium (0.73 g, 30 mmol) in diethyl ether (60ml) was added paraformaldehyde (0.99 g, 33 mmol). Then the mixture was refluxed 18 hours, cooled to 0 °C and treated with a saturated ammonium chloride solution (50 ml). The ether layer was separated, washed with water, brine, dried over $MgSO_4$ and concentrated. The title compound was recrystallized from diethyl ether-hexane.

Step D:

3-Trimethylsilyl-5-[N,N-(1,1,4,4-tetramethyl-1,4-disilylethylene)]aminobenzaldehyde

The title compound was prepared as described in Step B of Example 5 and recrystallized from diethyl ether hexane.

Step E:

Ethyl-2,2-difluoro-3-hydroxy-3-[3-trimethylsilyl-5-[N,N-(1,1,4,4-tetramethyl-1,4-disilylethylene)]amino]phenyl propionate

The title compound was prepared as described in Step C of Example 5 and recrystallized from diethyl ether hexane.

Step F:

Ethyl-2,2-difluoro-3-acetoxy-3-(3-trimethylsilyl-5-amino)phenyl propionate

To a solution of ethyl-2,2-difluoro-3-hydroxy-3-[3-trimethylsilyl-5-[N,N-(1,1,4,4-tetramethyl-1,4-disilylethylene)]amino]phenyl propionate (5.51 g, 12 mmol) and triethylamine (1.31 g, 13 mmol) in tetrahydrofuran (15 ml) at 0 °C was added acetyl chloride (0.94 g, 12 mmol). Then the mixture was stirred 1 hour at room temperature, cooled to 0 °C, treated slowly with 10% HCl in ethanol (20 ml) followed by stirring for 3 hours at room temperature. The solvents were removed under reduced pressure and the residue partitioned between diethyl ether and water. The acidic aqueous layer was separated and made basic, then extracted twice with diethyl ether. The organic extract was dried over $MgSO_4$ and concentrated and the product was purified by conversion to its hydrochloride salt.

Step G:

Ethyl-2,2-difluoro-3-acetoxy-3-(3-trimethylsilyl-5-nitrile)-phenylpropionate

To a solution of ethyl-2,2-difluoro-3-acetoxy-3-(3-trimethylsilyl-5-amino)phenyl propionate hydrochloride (3.96 g, 10 mmol) in 0.5N HCl (20 ml) at 0 °C was added a solution of sodium nitrite (0.69 g, 10 mmol) in water (5 ml), the temperature being kept at 0-5 °C by the addition of cracked ice. Then the mixture was cautiously neutralized by adding dry sodium carbonate. The resulting mixture was added dropwise to a solution of copper cyanide (0.90 g, 10 mmol) in ice water (10 ml) and toluene (20 ml) while vigorous stirring was maintained and the temperature being kept at 0-5 °C. Then the temperature was held at 0-5 °C for 30 min, allowed to rise to room temperature and stirring was continued for 3 hours. Then the toluene layer was separated, washed twice with water, brine, dried over $MgSO_4$ and concentrated under reduced pressure. The title compound was purified on silica gel (30% ethyl acetate in cyclohexane).

Step H:

2,2-Difluoro-3-hydroxy-3-(3-trimethylsilyl-5-nitrile)phenylpropionic acid

To a solution of ethyl-2,2-difluoro-3-acetoxy-3-(3-trimethylsilyl-5-nitrile)phenyl propionate (1.85 g, 5 mmol) in 1,2-dimethoxyethane (8 ml) and water (2 ml) was added lithium hydroxide (0.36 g, 15 mmol) and the mixture was stirred 2 hours at room temperature. Then the solvent was removed under reduced pressure. The crude product was dissolved in water (20 ml) and extracted twice with diethyl ether. Then the aqueous layer was made acidic with 1N HCl and extracted with dichloromethane. The organic layer was dried over $MgSO_4$ and concentrated under reduced pressure. The title compound was recrystallized from diethyl ether.

Step I:

2,2-Difluoro-3-keto-3-(3-trimethylsilyl-5-nitrile)phenylpropionic acid

The title compound was prepared as described in Step B of Example 5 and recrystallized from diethyl ether.

EXAMPLE 7

**1,1,1-Trifluoro-3-(3-trimethylsilyl)phenylpropanone**

Step A:

3-Trimethylsilylbenzylethyl ether

To a solution of 3-trimethylsilylbenzylalcohol (3.60 g, 20 mmol) in tetrahydrofuran (40 ml) at 0 °C was added dropwise 1.5 M butyllithium (13.3 ml) and the mixture was stirred for 10 minutes. Then iodoethane (3.12 g, 20 mmol) in tetrahydrofuran (20 ml) was added dropwise and the mixture was stirred at room temperature for 3 hours. The reaction mixture was treated with water (100 ml) and the crude product was

extracted twice with diethyl ether (100 ml). The organic layer was washed with brine, dried over $MgSO_4$, concentrated and 3-trimethylsilylbenzylethyl ether was purified by distillation.

Step B:

1,1,1-Trifluoro-3-(3-trimethylsilyl)phenylpropanone

To a suspension of lithium (0.42 g, 60 mmol) in tetrahydrofuran (10 ml) at -10 °C was added dropwise 3-trimethylsilylbenzylethyl ether (2.08 g, 10 mmol) in diethyl ether (10 ml). Then the mixture was allowed to react 1 hour at -10 °C and added dropwise to a solution of ethyl trifluoroacetate (2.84 g, 20 mmol) in diethyl ether (20 ml) at -78 °C. Then the cooling bath was removed and the mixture was allowed to warm to room temperature, and treated with 3 N HCl (20 ml). The organic layer was separated, washed with water, brine, dried over $MgSO_4$ and concentrated under reduced pressure. Chromatography on silica gel (10% diethyl ether in cyclohexane), followed by distillation under reduced pressure, afforded the expected product.

EXAMPLE 8

**1,1,1-Trifluoro-3-(3-trimethylsilyl-5-amino)phenylpropanone hydrochloride**

Step A:

3-Trimethylsilyl-5-[N,N-(1,1,4,4-tetramethyl-1,4-disilethylene)]aminobenzylbromide

To a solution of 3-bromo-5-trimethylsilyl-[N,N-(1,1,4,4-tetramethyl-1,4-disilethylene)aniline (3.86 g, 10 mmol) in diethyl ether (10 ml) at 0 °C was added 1.5M butyllithium (6.67 ml). Then the mixture was stirred 15 min, cooled to -78 °C and treated with dibromomethane (1.74 g, 10 mmol) in diethyl ether (5 ml). Stirring was continued 30 min at -78 °C, then the temperature was allowed to rise to room temperature and stirring was continued for 1 hour. To the mixture was added a saturated ammonium chloride solution (20 ml) and the organic layer separated, washed with water, brine, dried over $MgSO_4$ and concentrated under reduced pressure. The title compound was purified by recrystallization from hexane.

Step B:

1,1,1-Trifluoro-3-(3-trimethylsilyl-5-amino)phenylpropanone hydrochloride

To a solution of 3-trimethylsilyl-5-[N,N-(1,1,4,4-tetramethyl-1,4-disilethylene)]aminobenzyl magnesium bromide prepared from 3-trimethylsilyl-5-[N,N-(1,1,4,4-tetramethyl-1,4-disilethylene)]aminobenzylbromide (2.00 g, 5 mmol) and magnesium (0.12 g, 5 mmol) in diethyl ether (10 ml) at -78 °C was added ethyltrifluoroacetate (1.42 g, 10 mmol) over 5 min. Then the cooling bath was removed and the mixture was allowed to warm to 0 °C, treated slowly with 10% HCl in ethanol (10 ml), followed by stirring for 3 hours at room temperature. The solvents were removed under reduced pressure and the residue partitioned between diethyl ether and water. The acidic aqueous layer was separated and made basic, then extracted twice with diethyl ether. The ether extract was dried over $MgSO_4$ and concentrated and the title compound was purified by conversion to its HCl salt.

EXAMPLE 9

**Ethyl-2,2-difluoro-3-keto-4-(3-trimethylsilyl)phenyl butanoate**

Step A:

3-Trimethylsilylbenzylbromide

A mixture of 3-trimethylsilyltoluene (3.28 g, 10 mmol), N-bromosuccinimide (1.78 g, 10 mmol) and benzoylperoxide (10 mg) in $CCl_4$ (20 ml) was stirred under reflux for 3 hours, then cooled and filtered. The solvent was removed under reduced pressure and 3-trimethylsilylbenzylbromide was purified by distillation.

Step B:

2-(3-Trimethylsilyl)phenylethanol

The title compound was prepared as described in Step C of Example 6 and recrystallized from diethyl ether - hexane.

Step C:

2-(3-Trimethylsilyl)phenylethanal

The title compound was prepared as described in Step B of Example 5 and purified by distillation.

Step D:

Ethyl-2,2-difluoro-3-hydroxy-4-(3-trimethylsilyl)phenyl butanoate

The title compound was prepared as described in Step C of Example 5.

Step E:

Ethyl-2,2-difluoro-3-keto-4-(3-trimethylsilyl)phenyl butanoate

The title compound was prepared as described in Step B of Example 5 and purified by distillation.

## EXAMPLE 10

**Ethyl-2,2-difluoro-3-keto-4-(3-trimethylsilyl-5-amino)phenyl butanoate hydrochloride**

Step A:

2-[3-Trimethylsilyl-5-[N,N-(1,1,4,4-tetramethyl-1,4-disilylethylene)amino]]phenyl ethanol

The title compound was prepared as described in Step C of Example 6.

Step B:

2-[3-Trimethylsilyl-5-[N,N-(1,1,4,4-tetramethyl-1,4-disilylethylene)amino]]phenyl ethanal

The title compound was prepared as described in Step B of Example 5.

Step C:

Ethyl-2,2-difluoro-3-hydroxy-4-[3-trimethylsilyl-5-[N,N(1,1,4,4-tetramethyl-1,4-disilethylene)amino]]phenyl butanoate

The title compound was prepared as described in Step C of Example 5.

Step D:

Ethyl-2,2-difluoro-3-keto-4-(3-trimethylsilyl-5-amino)phenyl butanoate hydrochloride

The title compound was prepared by following the procedure described in Step B of Example 5 and purified to its hydrochloride salt by removing the protecting amino group with 10% HCl in ethanol.

EXAMPLE 11

**1,1,1-Trifluoro-2-(3-trimethylsilylmethyl)phenyl ethanone**

Step A:

3-Trimethylsilylmethylphenylbromide

The title compound was prepared as described in Step A of Example 1.

Step B:

1,1,1,-Trifluoro-2-(3-trimethylsilylmethyl)phenyl ethanone

The title compound was prepared as described in Step B of Example 1.

EXAMPLE 12

**1,1,1-Trifluoro-2-(3-trimethylsilylmethyl-5-amino)phenyl ethanone hydrochloride**

Step A:

3-Trimethylsilylmethyl-5-[N,N-(1,1,4,4-tetramethyl-1,4-disilylethylene ]amino phenylbromide

To a solution of 3,5-dibromo-N,N-(1,1,4,4-tetramethyl-1,4-disilylethylene)aniline (7.86 g, 20 mmol) in diethyl ether (20 ml) at 0 °C was added 1.5M butyllithium in hexane (13.3 ml) over 10 min. Then the mixture was stirred 15 min at 0 °C and iodomethyltrimethylsilane (4.28 g, 20 mmol) in diethyl ether (10 ml) was added dropwise. The mixture was stirred 3 hours at room temperature and treated with a saturated ammonium chloride solution (20 ml). The ether layer was separated, washed with water, brine, dried over $MgSO_4$ and concentrated under reduced pressure. The title compound was recrystallized from hexane.

Step B:

1,1,1-Trifluoro-2-(3-trimethylsilylmethyl-5-amino)phenyl ethanone hydrochloride

The title compound was prepared by following the procedure described in Step B of Example 1. After hydrolysis and removing of the organic layer, the aqueous layer was made basic and extracted twice with dichloromethane. The dichloromethane extract was dried over $MgSO_4$, concentrated under reduced pressure and the title compound was purified by conversion to its hydrochloride salt.

EXAMPLE 13

**Ethyl-2,2-difluoro-3-keto-3-(3-trimethylsilylmethyl)phenyl propionate**

Step A:

3-Trimethylsilylmethylbenzylalcohol

To a solution of 3-methylbenzylalcohol (6.10 g, 50 mmol) in anhydrous diglyme (200 ml) at -78 °C was added 1.5M butyllithium (100 ml) over 20 min. The resulting mixture was allowed to warm to -20 °C and stirred for 30 min. Then a mixture of trimethylchlorosilane (19.0 g, 175.0 mmol) and triethylamine (5.05 g, 50 mmol) was added and the final solution was stirred at room temperature for 3 hours. The reaction mixture was treated with ice water (200 ml), extracted three times with diethyl ether (100 ml) and the extracts washed twice with water, brine, dried over $MgSO_4$ and concentrated. The crude product was dissolved in 90% aqueous methanol (200 ml) and refluxed for 1 hour. Then the solvents were removed and 3-trimethylsilylmethylbenzylalcohol was purified by fractional distillation.

Step B:

3-Trimethylsilylmethylbenzaldehyde

The title compound was prepared as described in Step B of Example 5.

Step C:

Ethyl-2,2-difluoro-3-hydroxy-3-(3-trimethylsilylmethyl)phenyl propionate

The title compound was prepared as described in Step C of Example 5.

Step D:

Ethyl-2,2-difluoro-3-keto-3-(3-trimethylsilylmethyl)phenyl propionate

The title compound was prepared as described in Step B of Example 5.

EXAMPLE 14

**Ethyl-2,2-difluoro-3-keto-3-(3-trimethylsilylmethyl-5-amino) phenyl propionate hydrochloride**

Step A:

3-Bromo-5-[N,N(1,1,4,4-tetramethyl-1,4-disilylethylene)amino]-toluene

To a solution of 3,5-dibromo-N,N-(1,1,4,4-tetramethyl-1,4-disilylethylene)aniline (19.65 g, 50 mmol) in diethyl ether (50 ml) at 0°C was added 1.5M butyllithium (33.3 ml) over 15 min. Then the mixture was stirred for 15 min and iodomethane (7.10 g, 50 mmol) in diethyl ether (15 ml) was added over 15 min at 0°C. The mixture was stirred 1 hour at 0°C and was allowed to warm to room temperature and stirred for 3 hours. Then the reaction mixture was poured into ice water (100 ml). The organic layer was separated, washed with brine, dried over $MgSO_4$ and concentrated. The title compound was recrystallized from hexane.

Step B:

3-Methyl-5-[N,N(1,1,4,4-tetramethyl-1,4-disilylethylene)]-aminobenzyl alcohol

The title compound was prepared as described in Step C of Example 6.

Step C:

3-Trimethylsilylmethyl-5-[N,N(1,1,4,4-tetramethyl-1,4-disilylethylene)]aminobenzyl alcohol

The title compound was prepared as described in Step A of Example 13 and recrystallized from hexane.

Step D:

3-Trimethylsilylmethyl-5-[N,N(1,1,4,4-tetramethyl-1,4-disilylethylene ) ]aminobenzaldehyde

The title compound was prepared as described in Step D of Example 6.

Step E:

Ethyl-2,2-difluoro-3-hydroxy-3-[3-trimethylsilylmethyl-5-[N,N-(1,1,4,4-tetramethyl-1,4-disilylethylene)]amino]-phenylpropionate

The title compound was prepared as described in Step E of Example 6.

Step F:

Ethyl-2,2-difluoro-3-keto-3-(3-trimethylsilylmethyl-5-amino)-phenyl propionate hydrochloride

The title compound was prepared as described in Step D of Example 10.

It is now established that Alzheimer's disease and other senile degenerative diseases are characterized by a selective loss in the cerebral cortex of choline acetyltransferase, the enzyme responsible for the biosynthesis of acetylcholine. There also exists a good correlation between memory impairment or dementia and the decrement in cholinergic transmission. Thus, impaired cholinergic transmission in the central nervous system may be, at least in part, responsible for the symptomatology of Alzheimer's disease and senile dementia. In support to these conclusions such compounds as physostigmine and 1,2,3,4-tetrahydro-9-aminoacridine (THA), compounds which prevent the catabolism of acetylcholine have found a place in the treatment of Alzheimer's and other senile degenerative diseases. Indeed, it has been recognized that the extent of improvement of cognitive functions has been closely related to the degree of inhibition of acetylcholinesterase.

The compounds of Formula I are pharmacologically active agents capable of inhibiting acetylcholinesterase as demonstrable in standard biological *in vitro* and *in vivo* test procedures. Indeed, based upon standard laboratory procedures, it is to be shown that the compounds of Formula I are potent and selective, quasi irreversible inhibitors of acetylcholinesterase capable of demonstrating advantages over the prior art, particularly physostigmine, in their use in the treatment of Alzheimer's disease and senile dementia. The compounds, in general, will exert their acetylcholinesterase inhibitory properties within the dose range of about 0.01 mg to 5 mg per kilogram of body weight for the preferred compounds.

For pharmacological end-use applications, the compounds of Formula I are preferentially administered in the form of their pharmaceutically acceptable acid addition salts. Of course, the effective dosage of the compounds will vary according to the individual potency of each compound employed, the severity and nature of the disease being treated and the particular subject being treated. In general, effective results can be achieved by administering a compound at a dosage of about 0.01 mg to about 20 mg per kilogram of body weight per day, administered systemically. Therapy should be initiated at lower dosages. The dosage thereafter may be administered orally in solid dosage forms, e.g., capsules, tablets, or powders, or in liquid forms, e.g., solutions or suspensions. The compounds may also be injected parenterally in the form of sterile solutions or suspensions. Solid oral forms may contain conventional excipients, for instance: lactose, sucrose, magnesium stearate, resins, and like materials. Liquid oral forms may contain various flavoring, coloring, preserving, stabilizing, buffering, solubilizing, or suspending agents. If desired, additives, such as saline or glucose may be added to make the solutions isotonic.

As is true for most classes of compounds suitable for use as therapeutic agents, certain subgeneric groups and certain specific compounds are preferred. In this instance those compounds wherein $R_1$, $R_2$ and $R_3$ are all methyl or ethyl or mixtures thereof are preferred, and wherein one of $R_1$, $R_2$ and $R_3$ is a long-chain (8 to 10 carbon atoms) alkyl. Preferred compounds are those wherein X is F, or hydrogen, or alkyl, or $COR_9$ wherein $R_9$ is alkyl, or $CO_2R_5$ wherein $R_5$ is H or alkyl, or $CONHR_5$ wherein $R_5$ is H or alkyl. Preferred groups of compounds are also those wherein m is one and n is zero; n is one and m is zero or when both are zero. It is preferred that Y be hydrogen, alkoxy or $SiR_1R_2R_3$ with the preferred $SiR_1R_2R_3$ moieties being as described for the $SiR_1R_2R_3$ at the 3-position of depicted Formula I, although when any specific compound is a bis-$SiR_1R_2R_3$ both moieties need not be identified.

Specifically preferred compounds are those charted below as follows:

| | 3-position $SiR_1R_2R_3$ | m | n | X | (Y) | Q |
|---|---|---|---|---|---|---|
| 1. | $CH_3$, $CH_3$, $CH_3$ | 0 | 0 | F | H | $>C=O$ |
| 2. | $CH_3$, Et, Et | 0 | 0 | F | H | $>C=O$ |
| 3. | Et, Et, Et | 0 | 0 | F | H | $>C=O$ |
| 4. | Et, Et, $CH_3$ | 0 | 0 | F | H | $>C=O$ |
| 5. | $CH_3$, $CH_3$, Et | 0 | 0 | F | H | $>C=O$ |
| 6. | $CH_3$, $CH_3$, $CH_3$ | 0 | 0 | F | 5- $SiCH_3CH_3CH_3$ | $>C=O$ |
| 7. | octyl, $CH_3$, $CH_3$ | 0 | 0 | F | H | $>C=O$ |
| 8. | $CH_3$, $CH_3$, $CH_3$ | 1 | 0 | F | H | $>C=O$ |
| 9. | $CH_3$, $CH_3$, $CH_3$ | 0 | 1 | F | H | $>C=O$ |
| 10. | $CH_3$, $CH_3$, $CH_3$ | 0 | 0 | H | H | $>C=O$ |
| 11. | $CH_3$, $CH_3$, $CH_3$ | 0 | 0 | $CH_3$ | H | $>C=O$ |
| 12. | $CH_3$, $CH_3$, $CH_3$ | 0 | 0 | $-COCH_3$ | H | $>C=O$ |
| 13. | $CH_3$, $CH_3$, $CH_3$ | 0 | 0 | $-COOCH_3$ | H | $>C=O$ |
| 14. | $CH_3$, $CH_3$, $CH_3$ | 0 | 0 | $-CONHCH_3$ | H | $>C=O$ |

## Claims

**Claims for the following Contracting States : AT, BE, CH, DE, DK, FR, GB, IT, LI, LU, NL, SE**

**1.** A compound of the formula

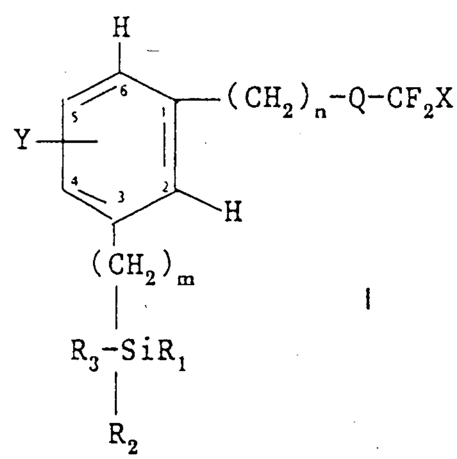

I

and the pharmaceutically acceptable salts thereof, wherein each of m and n is zero or one with the proviso that the sum of m and n is less than two,
Q is

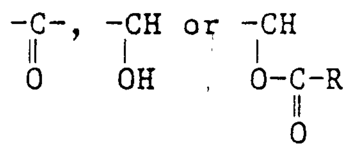

with R being H or $C_{1-10}$ alkyl
X is X' or X" with
X' being H, Br, Cl, F or $R_4$ and
X" being $COR_9$, $CO_2R_5$, $CONHR_5$ or $COR_6$,
$R_1$, $R_2$, $R_3$ and $R_4$ each being $C_{1-10}$ alkyl, or $(CH_2)p$ aryl, with p being zero, one or two,
$R_5$ is H, $C_{1-10}$ alkyl, phenyl, benzyl or phenethyl,
$R_9$ is $C_{1-10}$ alkyl, phenyl, benzyl or phenethyl,
$R_6$ is $(NHCHR_7C(O))_qR_8$ with $R_7$ being the residue of any natural occurring $\alpha$-amino acid, q is one to four and $R_8$ is $OR_5$ or $NHR_5$,
Y is H, OH, $C_{1-6}$ alkyl, $C_{1-6}$ alkoxy, hydroxy $C_{1-6}$ alkyl, amino $C_{1-6}$ alkyl, $NH_2$, azido, CN, $CO_2R_5$, $COR_9$, $-SO_3H$, Br, Cl, F or $-(CH_2)_xSiR_1R_2R_3$ with x being zero, one or two,

2. A compound of Claim 1 wherein Q is

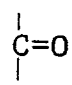

3. A compound of Claim 1 wherein Q is

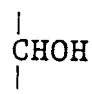

4. A compound of Claim 1 wherein Q is

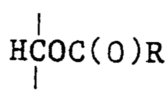

5. A compound of Claim 2 wherein X is X'

6. A compound of Claim 2 wherein X is X".

7. A compound of Claim 2 wherein each of m and n is zero.

8. A compound of Claim 2 wherein m is one.

9. A compound of Claim 2 wherein n is one.

10. A compound of Claim 5 wherein X' is F.

11. A compound of Claim 1 wherein at least one of $R_1$ and $R_2$ are methyl or ethyl and $R_3$ is methyl, ethyl or octyl.

**12.** The specific compounds of Claim 2 as defined in the following chart:

| 3-position $SiR_1R_2R_3$ | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, Et | 0 | 0 | F | H |
| $CH_3$, $CH_3$, $CH_3$ | 0 | 0 | F | 5- $SiCH_3CH_3CH_3$ |
| octyl, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |

wherein Et is ethyl and Q is $\rangle C = O$.

**13.** A process for preparing a compound of the formula

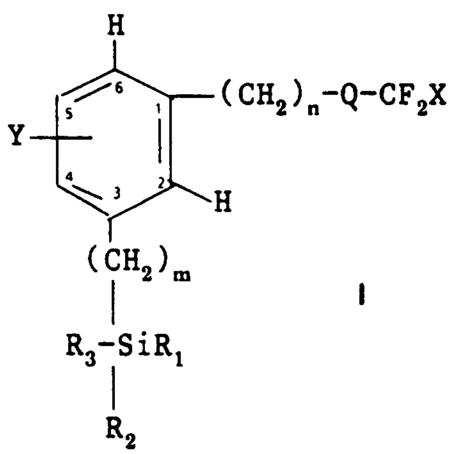

I

and the pharmaceutically acceptable salts thereof, wherein each of m and n is zero or one with the proviso that the sum of m and n is less than two,
Q is

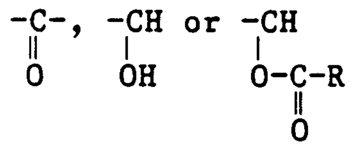

with R being H or $C_{1-10}$ alkyl
X is X′ or X″ with
X′ being H, Br, Cl, F or $R_4$ and
X″ being $COR_9$, $CO_2R_5$, $CONHR_5$ or $COR_6$,
$R_1$, $R_2$, $R_3$ and $R_4$ each being $C_{1-10}$ alkyl, or $(CH_2)p$ aryl, with p being zero, one or two,
$R_5$ is H, $C_{1-10}$ alkyl, phenyl, benzyl or phenethyl,
$R_9$ is $C_{1-10}$ alkyl, phenyl, benzyl or phenethyl,
$R_6$ is $(NHCHR_7C(O))_qR_8$ with $R_7$ being the residue of any natural occurring α-amino acid, q is one to four and $R_8$ is $OR_5$ or $NHR_5$,
Y is H, OH, $C_{1-6}$ alkyl, $C_{1-6}$ alkoxy, hydroxy $C_{1-6}$ alkyl, amino $C_{1-6}$ alkyl, $NH_2$, azido, CN, $CO_2R_5$, $COR_9$, $-SO_3H$, Br, Cl, F or $-(CH_2)_xSiR_1R_2R_3$ with x being zero, one or two,
which comprises
(a) in the instance of preparing compound of the sub-generic formula

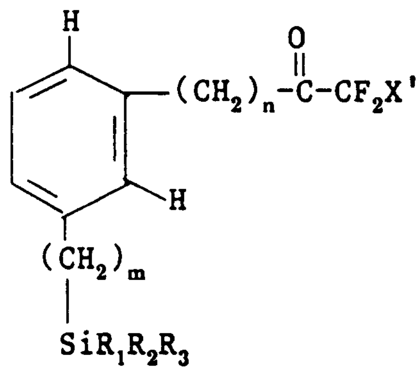

(2)

which comprises forming a lithio derivative of a compound of the formulae

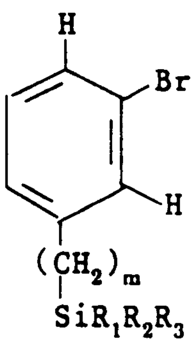

(3)

and

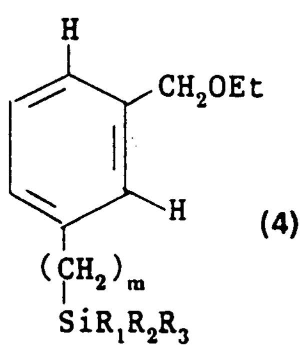

(4)

and reacting said lithio derivative with an acid (as its lithio salt), ester or acid chloride of the formulae $X'CF_2CO_2R$ or $X_4CF_2COCl$ wherein R is H or $C_{1-6}$ alkyl;

b) in the instance of preparing a compound of the sub-generic formula

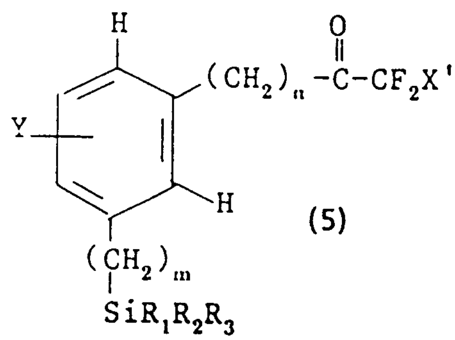

(5)

wherein Y is other than H
which comprises oxidizing a compound of the formula

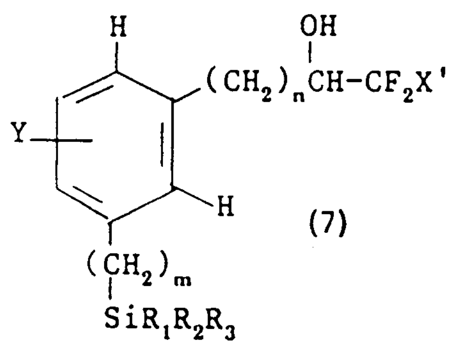

(7)

wherein Y is other than H.
c) in the instance of preparing a compound of the sub-generic formula

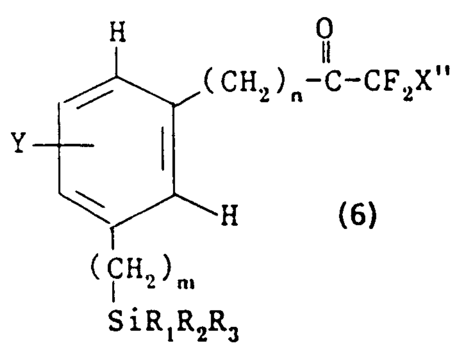

(6)

which comprises oxidizing a compound of the formula

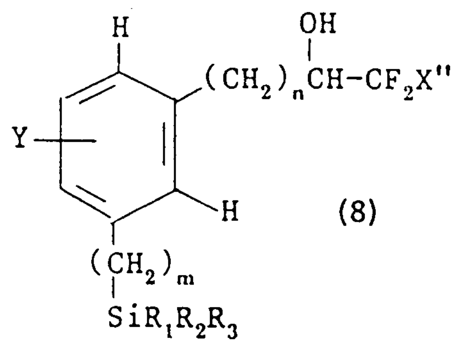

(8)

(d) in the instance of preparing a compound of the subgeneric formula

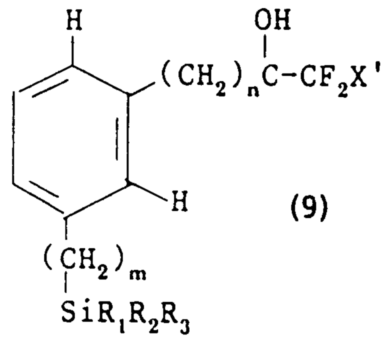

(9)

which comprises chemically reducing a compound of Formula (2) above,
e) in the instance of preparing a compound of the sub-generic formula

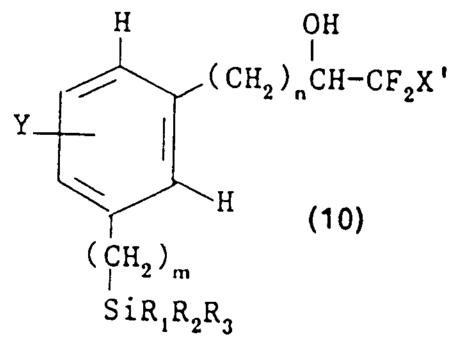

(10)

wherein Y is other than H
which comprises hydrolyzing a compound of the formula

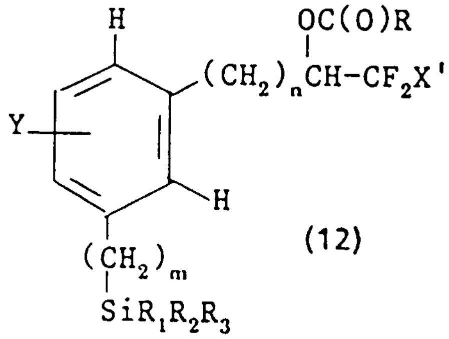

(12)

wherein Y is other than H.

f) in the instance of preparing a compound of the sub-generic formula

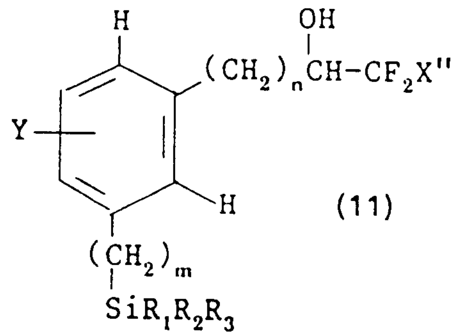

(11)

which comprises hydrolyzing a compound of the formula

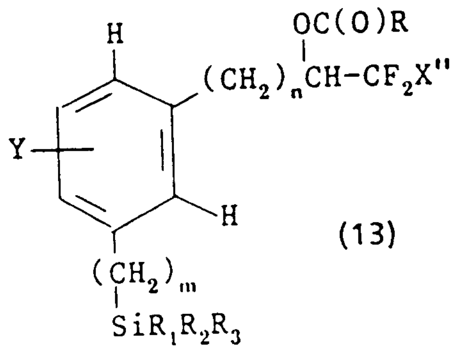

(13)

(g) in the instance of preparing a compound of the sub-generic formula

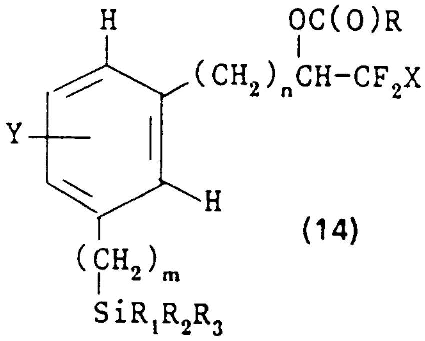

(14)

which comprises esterifying a compound of the formula

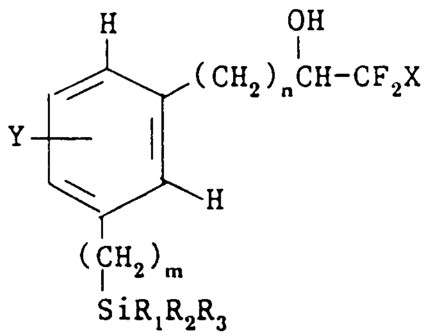

(15)

with an acyl halide,
(h) in the instance of preparing a compound of the sub-generic formula

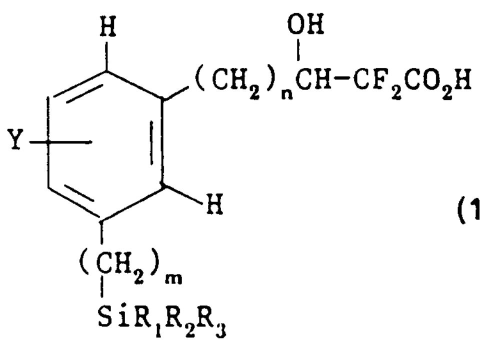

(16)

the process which comprises the hydrolysis of a compound of the formula

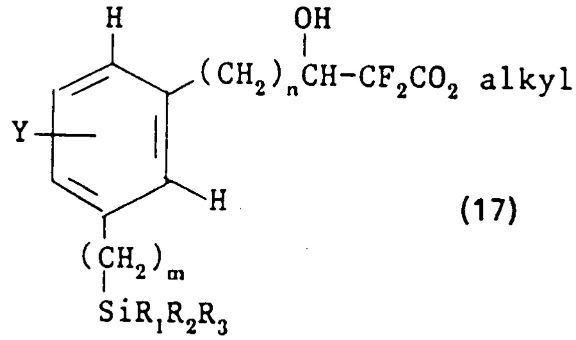

(17)

by reaction with lithium hydroxide in 80% ethylene glycol dimethyl ether.
(i) in the instance of preparing a compound of the sub-generic formula

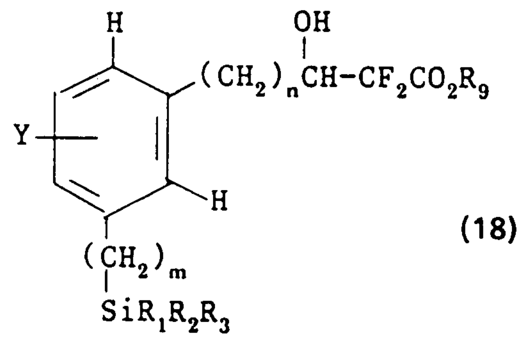

(18)

which comprises reacting a compound of the formula

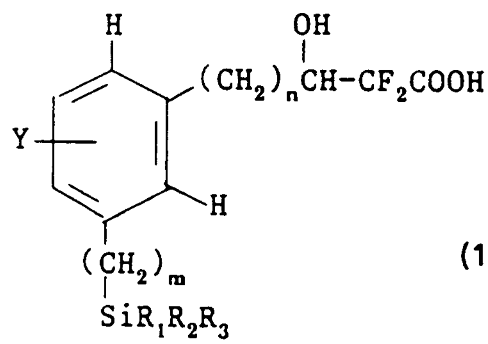

(19)

with a compound of the formula $R_9OH$ in the presence of dicyclohexyl carbodiimide and dimethylamino pyridine,

(j) in the instance of preparing a compound of the sub-generic formula

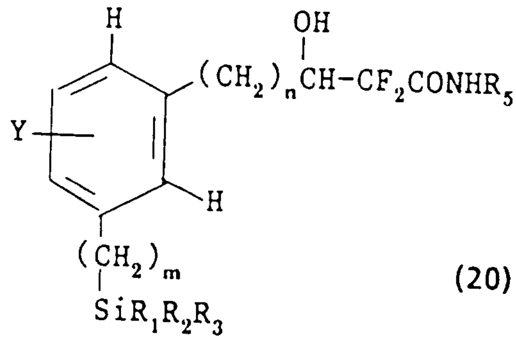

(20)

which comprises reacting a compound of the formula

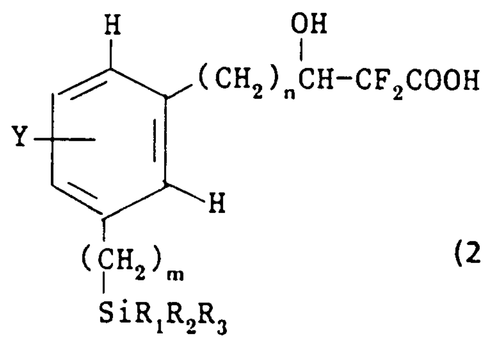

(21)

with an amine of the formula $R_5NH_2$ in the presence of dicyclohexylcarbodiimide and 1-hydroxybenzotriazole,

(k) in the instance of preparing a compound of the sub-generic formula

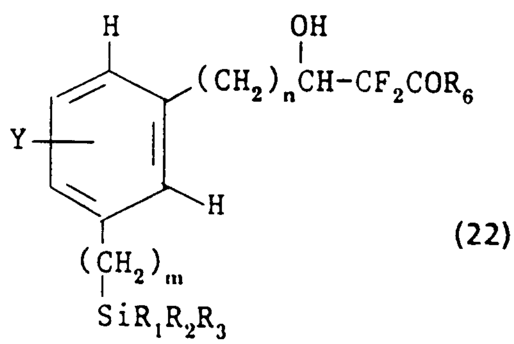

(22)

which comprises reacting a compound of the formula

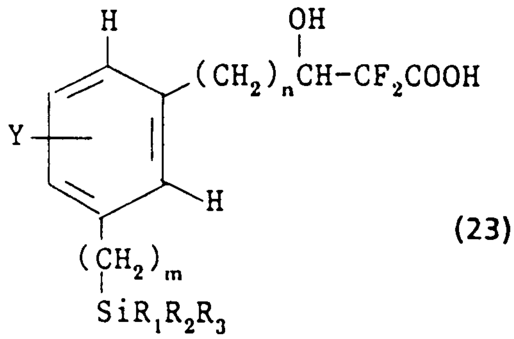

(23)

with a compound of the formula

$$(NHCHR_7C(O))_q-R_8$$

with $R_7$ being the residue of an $\alpha$-amino acid, q is an integer 1 to 4 and $R_8$ is $OR_5$ or $NHR_5$, followed by optionally converting the products of steps (a) thru (i) to their pharmaceutically acceptable salts.

**14.** The compound according to anyone of claims 1 to 12, for use as pharmaceutically active compound.

**15.** Use of the compounds according to anyone of claims 1 to 12, for the preparation of drugs useful for inhibiting acetylcholinesterase.

**16.** Use of the compounds according to anyone of claims 1 to 12, for the preparation of drugs useful for the treatment of Alzheimer's disease and senile dementia.

**Claims for the following Contracting State : ES**

**1.** A process for preparing a compound of the formula

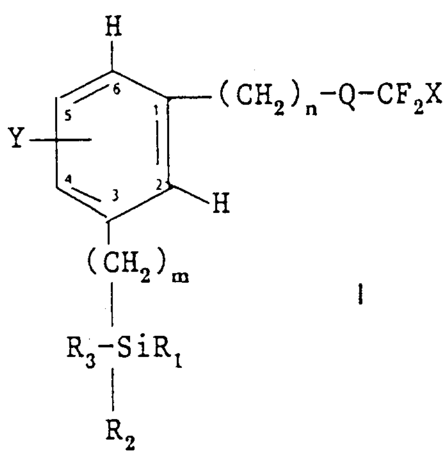

I

and the pharmaceutically acceptable salts thereof, wherein each of m and n is zero or one with the proviso that the sum of m and n is less than two,
Q is

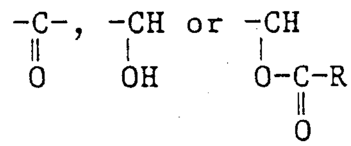

with R being H or $C_{1-10}$ alkyl
X is X' or X" with
X' being H, Br, Cl, F or $R_4$ and
X" being $COR_9$, $CO_2R_5$, $CONHR_5$ or $COR_6$,
$R_1$, $R_2$, $R_3$ and $R_4$ each being $C_{1-10}$ alkyl, or $(CH_2)p$ aryl, with p being zero, one or two,
$R_5$ is H, $C_{1-10}$ alkyl, phenyl, benzyl or phenethyl,
$R_9$ is $C_{1-10}$ alkyl, phenyl, benzyl or phenethyl,
$R_6$ is $(NHCHR_7C(O))_qR_8$ with $R_7$ being the residue of any natural occurring $\alpha$-amino acid, q is one to four and $R_8$ is $OR_5$ or $NHR_5$,
Y is H, OH, $C_{1-6}$ alkyl, $C_{1-6}$ alkoxy, hydroxy $C_{1-6}$ alkyl, amino $C_{1-6}$ alkyl, $NH_2$, azido, CN, $CO_2R_5$, $COR_9$, $-SO_3H$, Br, Cl, F or $-(CH_2)_xSiR_1R_2R_3$ with x being zero, one or two,
which comprises
(a) in the instance of preparing compound of the sub-generic formula

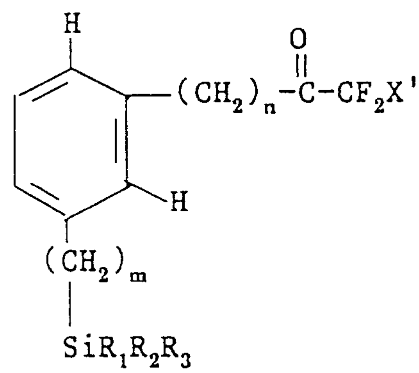

(2)

which comprises forming a lithio derivative of a compound of the formulae

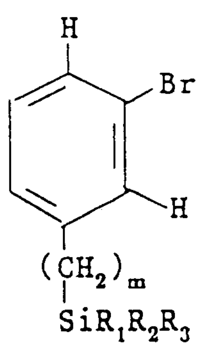

(3)

and

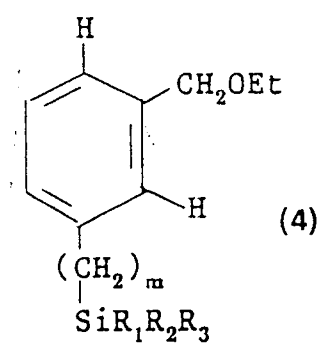

(4)

and reacting said lithio derivative with an acid (as its lithio salt), ester or acid chloride of the formulae $X'CF_2CO_2R$ or $X_4CF_2COCl$ wherein R is H or $C_{1-6}$ alkyl;

b) in the instance of preparing a compound of the sub-generic formula

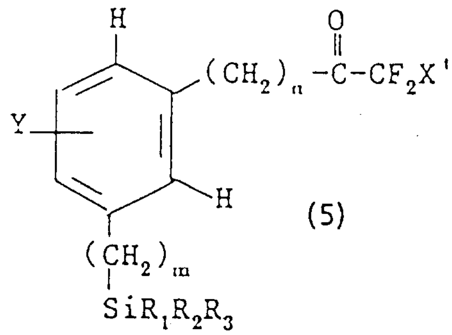

(5)

wherein Y is other than H
which comprises oxidizing a compound of the formula

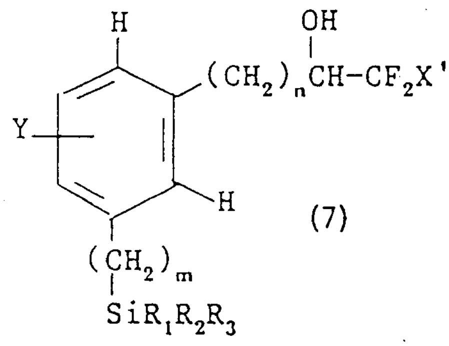

(7)

wherein Y is other than H.
c) in the instance of preparing a compound of the sub-generic formula

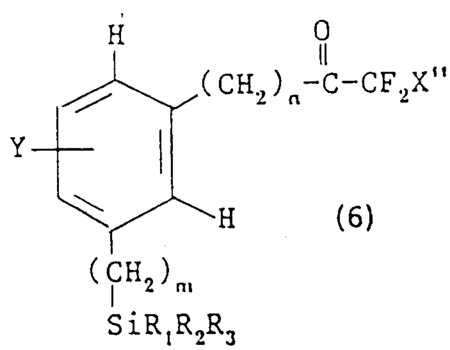

(6)

which comprises oxidizing a compound of the formula

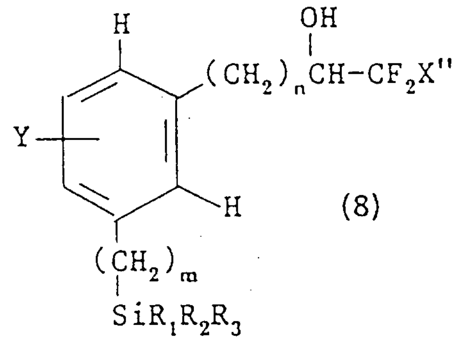

(8)

(d) in the instance of preparing a compound of the subgeneric formula

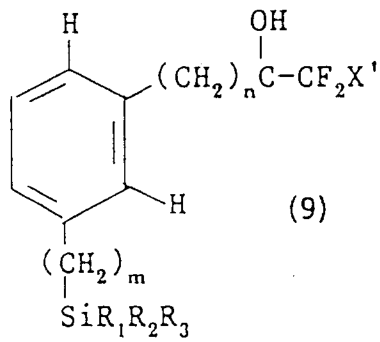

(9)

which comprises chemically reducing a compound of Formula (2) above,
e) in the instance of preparing a compound of the sub-generic formula

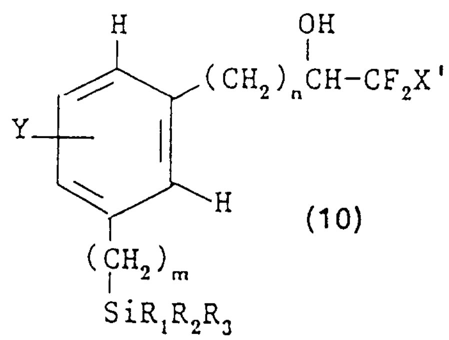

(10)

wherein Y is other than H
which comprises hydrolyzing a compound of the formula

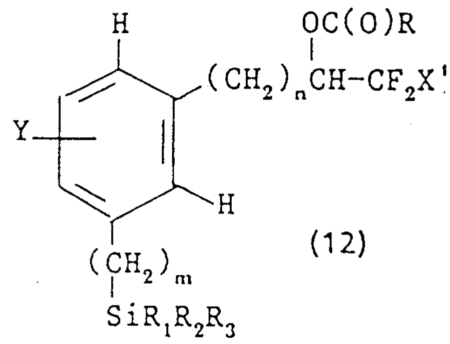

(12)

wherein Y is other than H.

f) in the instance of preparing a compound of the sub-generic formula

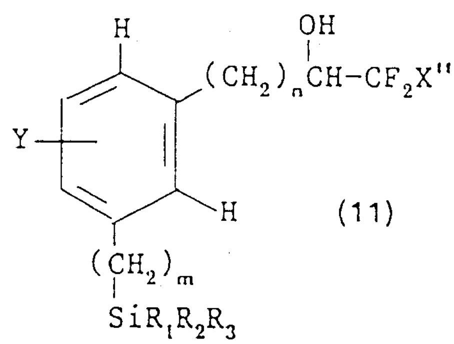

(11)

which comprises hydrolyzing a compound of the formula

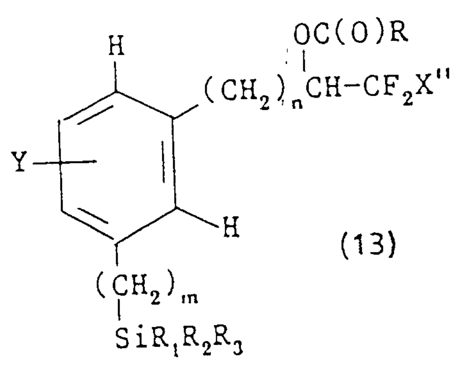

(13)

(g) in the instance of preparing a compound of the sub-generic formula

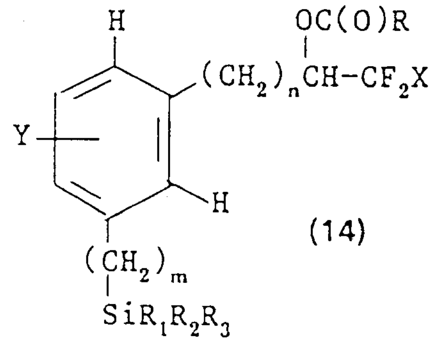

(14)

which comprises esterifying a compound of the formula

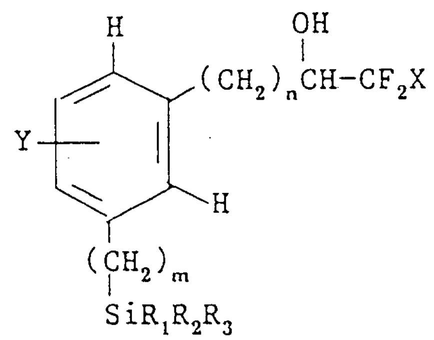

(15)

with an acyl halide,
(h) in the instance of preparing a compound of the sub-generic formula

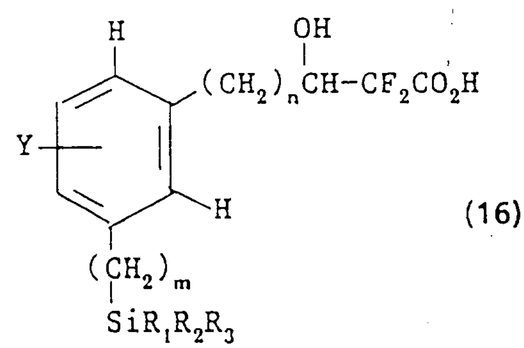

(16)

the process which comprises the hydrolysis of a compound of the formula

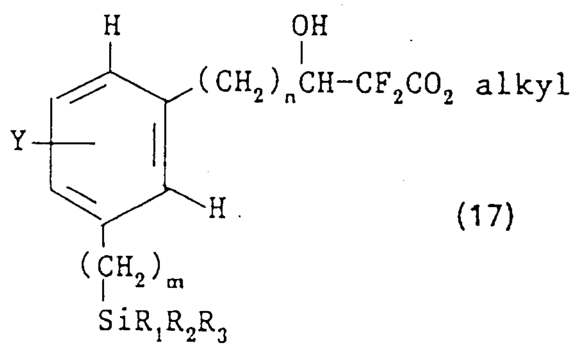

(17)

by reaction with lithium hydroxide in 80% ethylene glycol dimethyl ether.

(i) in the instance of preparing a compound of the sub-generic formula

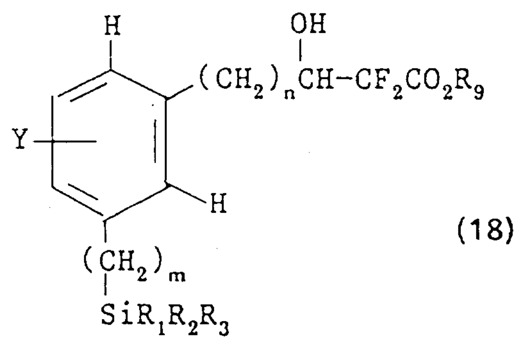

(18)

which comprises reacting a compound of the formula

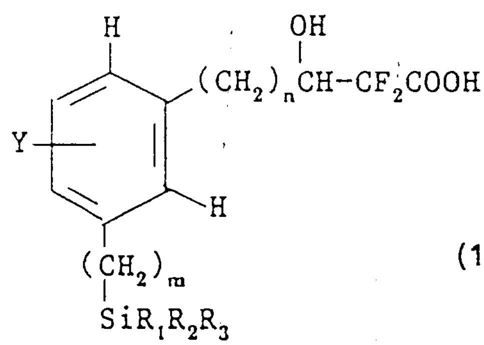

(19)

with a compound of the formula $R_9OH$ in the presence of dicyclohexyl carbodiimide and dimethylamino pyridine,

(j) in the instance of preparing a compound of the sub-generic formula

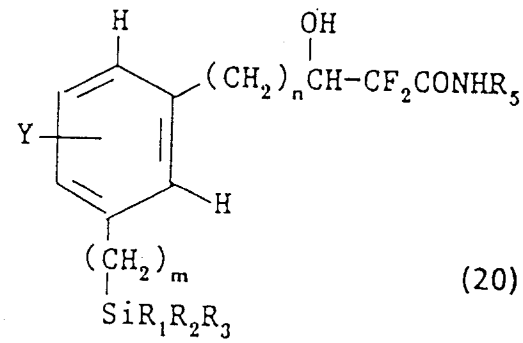

(20)

which comprises reacting a compound of the formula

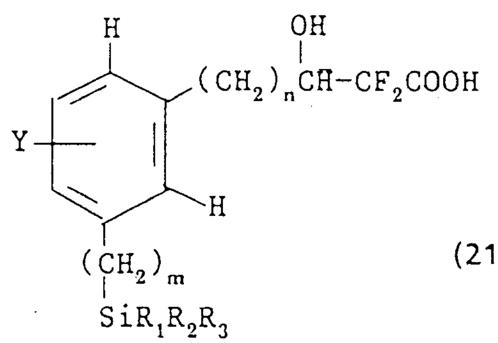

(21)

with an amine of the formula $R_5NH_2$ in the presence of dicyclohexylcarbodiimide and 1-hydroxybenzotriazole,

(k) in the instance of preparing a compound of the sub-generic formula

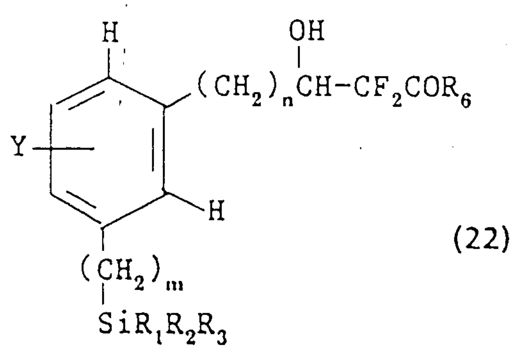

(22)

which comprises reacting a compound of the formula

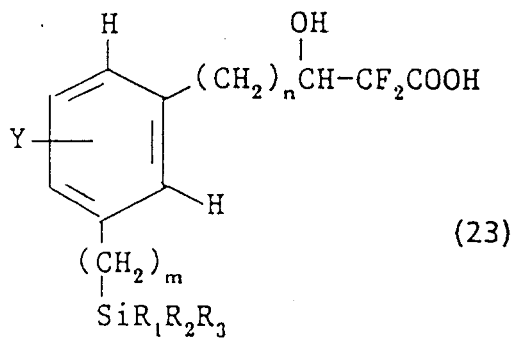

(23)

with a compound of the formula

$$(NHCHR_7C(O))_q-R_8$$

with $R_7$ being the residue of an $\alpha$-amino acid, q is an integer 1 to 4 and $R_8$ is $OR_5$ or $NHR_5$, followed by optionally converting the products of steps (a) thru (i) to their pharmaceutically acceptable salts.

**2.** A process according to claim 1 for preparing a compound of the formula I wherein at least one of $R_1$ and $R_2$ are methyl or ethyl and $R_3$ is methyl, ethyl or octyl, characterized in that in items (a), (b), (c), (d), (e), (f), (g), (h), (i), (j) or (k), compounds of formulae (3) and (4), (7), (8), (2), (12), (13), (15), (17), (19), (21) or (23) as defined in claim 1, wherein $R_1$, $R_2$ and $R_3$ are as defined above, are used.

**3.** A process according to claim 1 for preparing compounds of the formula I wherein Q is $>C=0$ and $R_1$, $R_2$, $R_3$, m, n, X and Y are as defined below :

| 3-position $SiR_1R_2R_3$ | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, Et | 0 | 0 | F | H |
| $CH_3$, $CH_3$, $CH_3$ | 0 | 0 | F | 5- $SiCH_3CH_3CH_3$ |
| octyl, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| wherein Et is ethyl and Q is $>C=0$. | | | | |

and pharmaceutically acceptable salts thereof,
characterized in that :

- in item (a), compounds of formulae (3) and (4) wherein m, $R_1$, $R_2$ and $R_3$ are as defined above, and compounds of formulae $X'CF_2CO_2R$ or $X_4CF_2COCl$ wherein X' is H or F, X is as defined above and R is as defined in claim 1, are used;
- in item (b), a compound of formula (7) wherein Y is $5-SiCH_3CH_3CH_3$, n, m, $R_1$, $R_2$, $R_3$ are as defined above, and X' = H or F is used ;

- in item (c), a compound of formula (8) wherein Y is 5-$SiCH_3CH_3CH_3$ , n, m, $R_1$, $R_2$, $R_3$ are as defined above, and X" is $CH_3$, -$COCH_3$, -$COOCH_3$ or -$CONHCH_3$ is used.

4. A process for the preparation of drugs useful for inhibiting acetylcholinesterase, characterized by mixing a compound prepared by the process according to any one of claims 1 to 3 or pharmaceutically acceptable salts thereof, as active ingredient, with a pharmaceutically acceptable vehicle.

5. A process for the preparation of drugs useful for the treatment of Alzheimer's disease and senile dementia, characterized by mixing a compound prepared by the process according to any one of claims 1 to 3 or pharmaceutically acceptable salts thereof, as active ingredient, with a pharmaceutically acceptable vehicle.

**Claims for the following Contracting State : GR**

1. A compound of the formula

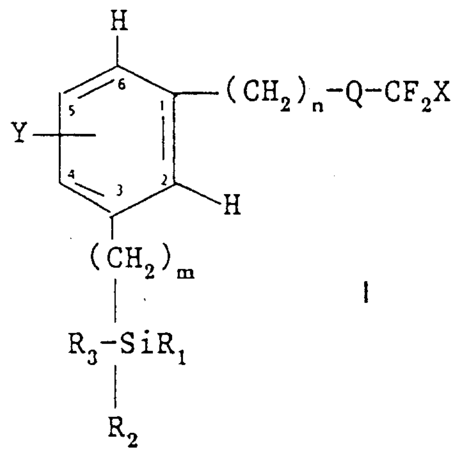

I

and the pharmaceutically acceptable salts thereof, wherein each of m and n is zero or one with the proviso that the sum of m and n is less than two,
Q is

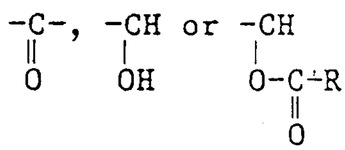

or

with R being H or $C_{1-10}$ alkyl
X is X' or X" with
X' being H, Br, Cl, F or $R_4$ and
X" being $COR_9$, $CO_2R_5$, $CONHR_5$ or $COR_6$,
$R_1$, $R_2$, $R_3$ and $R_4$ each being $C_{1-10}$ alkyl, or $(CH_2)$p aryl, with p being zero, one or two,
$R_5$ is H, $C_{1-10}$ alkyl, phenyl, benzyl or phenethyl,
$R_9$ is $C_{1-10}$ alkyl, phenyl, benzyl or phenethyl,
$R_6$ is $(NHCHR_7C(O))_qR_8$ with $R_7$ being the residue of any natural occurring α-amino acid, q is one to four and $R_8$ is $OR_5$ or $NHR_5$,
Y is H, OH, $C_{1-6}$ alkyl, $C_{1-6}$ alkoxy, hydroxy $C_{1-6}$ alkyl, amino $C_{1-6}$ alkyl, $NH_2$, azido, CN, $CO_2R_5$, $COR_9$, -$SO_3H$, Br, Cl, F or -$(CH_2)_xSiR_1R_2R_3$ with x being zero, one or two,

**2.** A compound of Claim 1 wherein Q is

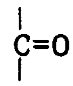

**3.** A compound of Claim 1 wherein Q is

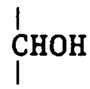

**4.** A compound of Claim 1 wherein Q is

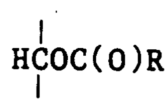

**5.** A compound of Claim 2 wherein X is X'

**6.** A compound of Claim 2 wherein X is X''.

**7.** A compound of Claim 2 wherein each of m and n is zero.

**8.** A compound of Claim 2 wherein m is one.

**9.** A compound of Claim 2 wherein n is one.

**10.** A compound of Claim 5 wherein X' is F.

**11.** A compound of Claim 1 wherein at least each of $R_1$ and $R_2$ are methyl or ethyl and $R_3$ is methyl, ethyl or octyl.

**12.** The specific compounds of Claim 2 as defined in the following chart:

| 3-position $SiR_1R_2R_3$ | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, Et | 0 | 0 | F | H |
| $CH_3$, $CH_3$, $CH_3$ | 0 | 0 | F | 5- $SiCH_3CH_3CH_3$ |
| octyl, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| wherein Et is ethyl and Q is $>C = 0$. | | | | |

**13.** A process for preparing a compound of the formula

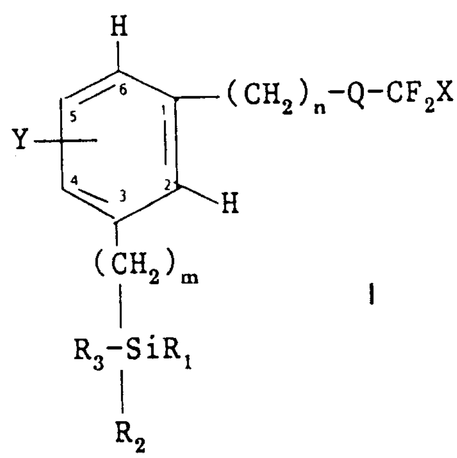

I

and the pharmaceutically acceptable salts thereof, wherein each of m and n is zero or one with the proviso that the sum of m and n is less than two,

Q is

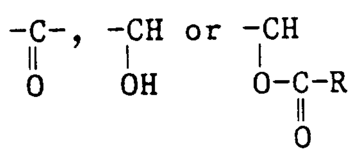

with R being H or $C_{1-10}$ alkyl

X is X' or X'' with

X' being H, Br, Cl, F or $R_4$ and

X'' being $COR_9$, $CO_2R_5$, $CONHR_5$ or $COR_6$,

$R_1$, $R_2$, $R_3$ and $R_4$ each being $C_{1-10}$ alkyl, or $(CH_2)p$ aryl, with p being zero, one or two,

$R_5$ is H, $C_{1-10}$ alkyl, phenyl, benzyl or phenethyl,

$R_9$ is $C_{1-10}$ alkyl, phenyl, benzyl or phenethyl,

$R_6$ is $(NHCHR_7C(O))_qR_8$ with $R_7$ being the residue of any natural occurring $\alpha$-amino acid, q is one to four and $R_8$ is $OR_5$ or $NHR_5$,

Y is H, OH, $C_{1-6}$ alkyl, $C_{1-6}$ alkoxy, hydroxy $C_{1-6}$ alkyl, amino $C_{1-6}$ alkyl, $NH_2$, azido, CN, $CO_2R_5$,

$COR_9$, $-SO_3H$, Br, Cl, F or $-(CH_2)_xSiR_1R_2R_3$ with x being zero, one or two, which comprises

(a) in the instance of preparing compound of the sub-generic formula

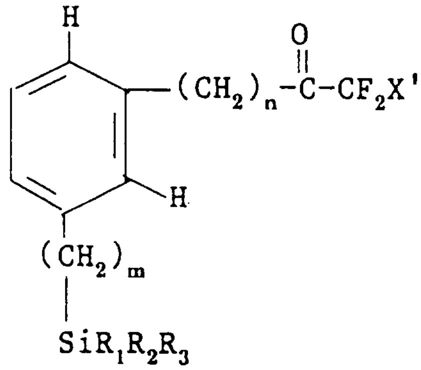

(2)

which comprises forming a lithio derivative of a compound of the formulae

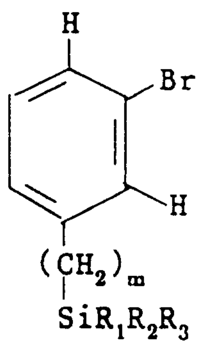

(3)

and

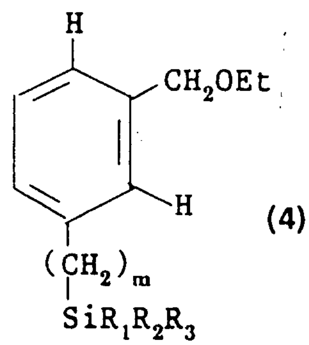

(4)

and reacting said lithio derivative with an acid (as its lithio salt), ester or acid chloride of the formulae $X'CF_2CO_2R$ or $X_4CF_2COCl$ wherein R is H or $C_{1-6}$ alkyl;

b) in the instance of preparing a compound of the sub-generic formula

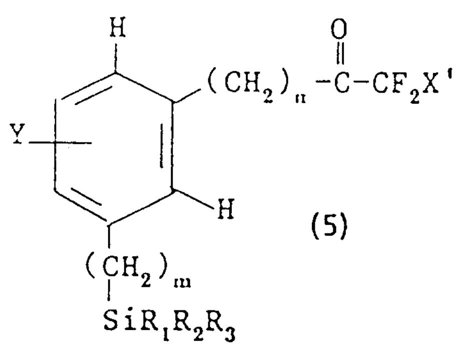

(5)

wherein Y is other than H

which comprises oxidizing a compound of the formula

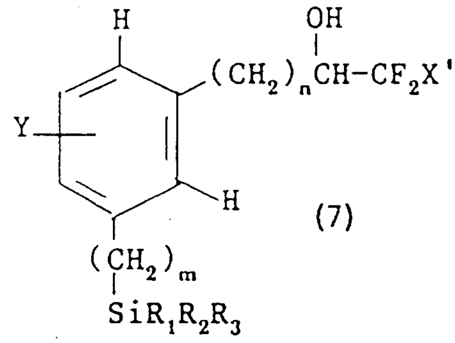

(7)

wherein Y is other than H.

c) in the instance of preparing a compound of the sub-generic formula

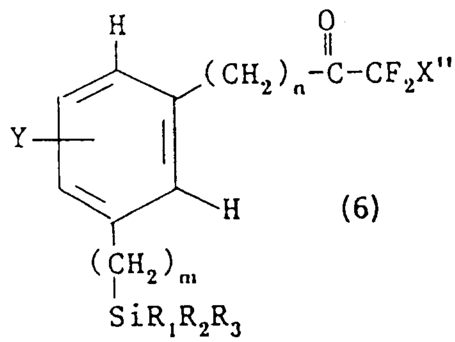

(6)

which comprises oxidizing a compound of the formula

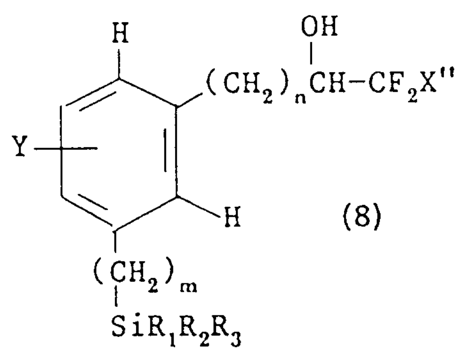

(8)

(d) in the instance of preparing a compound of the subgeneric formula

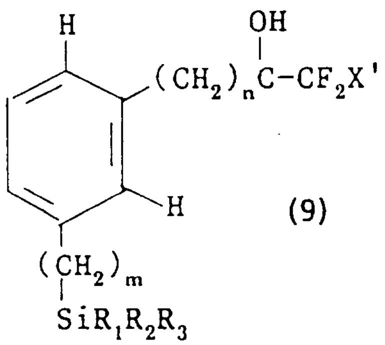

(9)

which comprises chemically reducing a compound of Formula (2) above,
e) in the instance of preparing a compound of the sub-generic formula

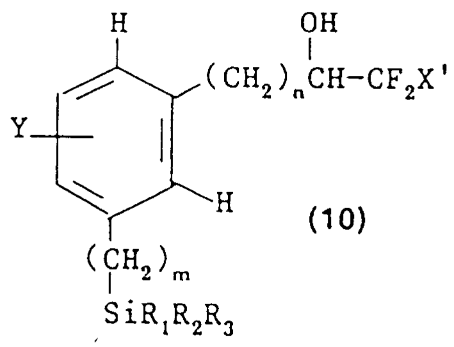

(10)

wherein Y is other than H
which comprises hydrolyzing a compound of the formula

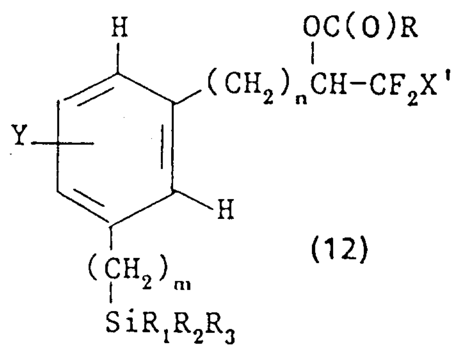

(12)

wherein Y is other than H.

f) in the instance of preparing a compound of the sub-generic formula

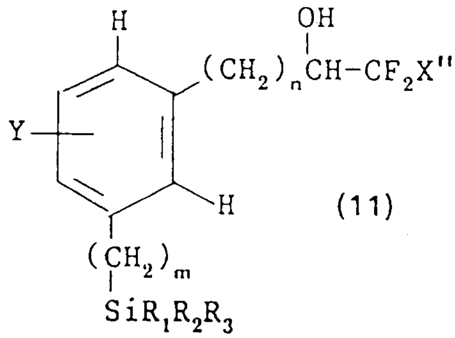

(11)

which comprises hydrolyzing a compound of the formula

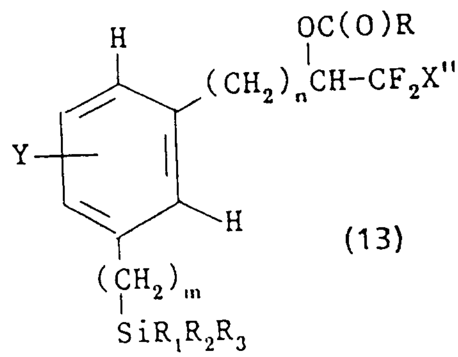

(13)

(g) in the instance of preparing a compound of the sub-generic formula

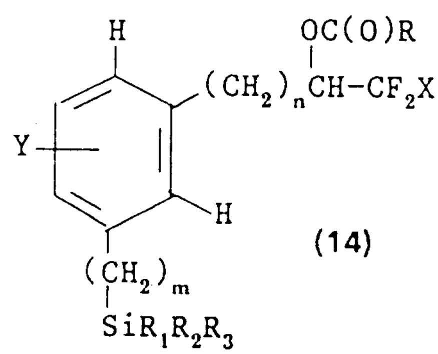

(14)

which comprises esterifying a compound of the formula

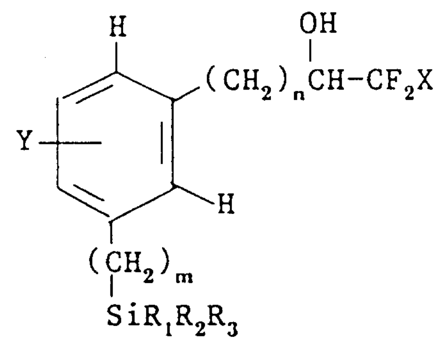

(15)

with an acyl halide,

(h) in the instance of preparing a compound of the sub-generic formula

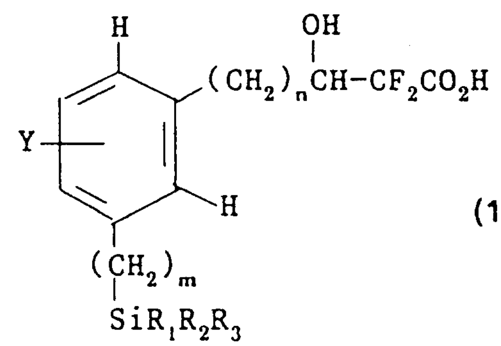

(16)

the process which comprises the hydrolysis of a compound of the formula

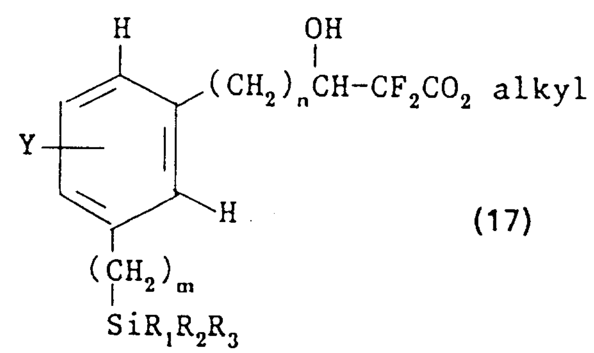

(17)

by reaction with lithium hydroxide in 80% ethylene glycol dimethyl ether.

(i) in the instance of preparing a compound of the sub-generic formula

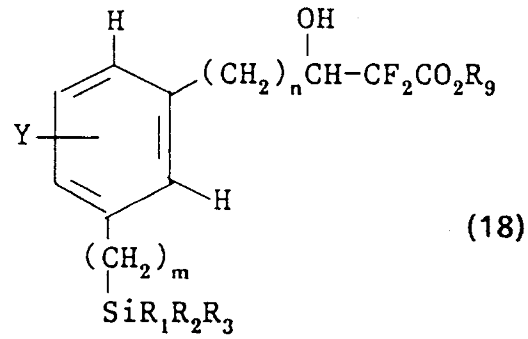

(18)

which comprises reacting a compound of the formula

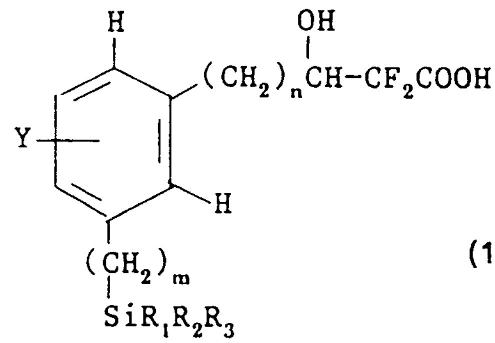

(19)

with a compound of the formula $R_9OH$ in the presence of dicyclohexyl carbodiimide and dimethylamino pyridine,

(j) in the instance of preparing a compound of the sub-generic formula

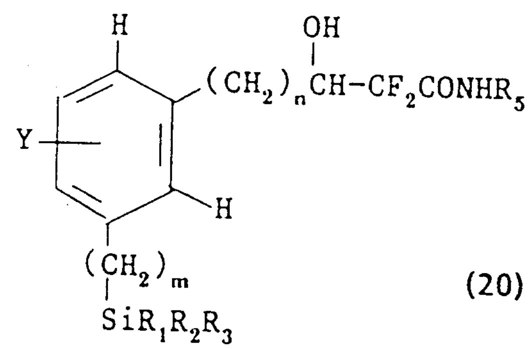

(20)

which comprises reacting a compound of the formula

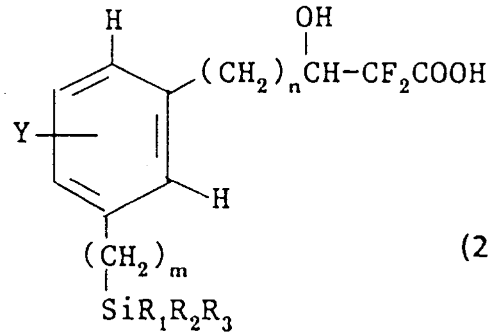

(21)

with an amine of the formula $R_5NH_2$ in the presence of dicyclohexylcarbodiimide and 1-hydroxybenzotriazole,

(k) in the instance of preparing a compound of the sub-generic formula

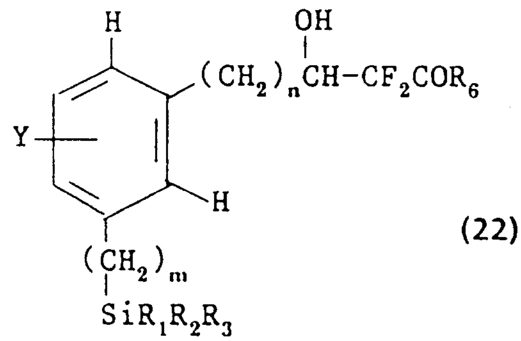

(22)

which comprises reacting a compound of the formula

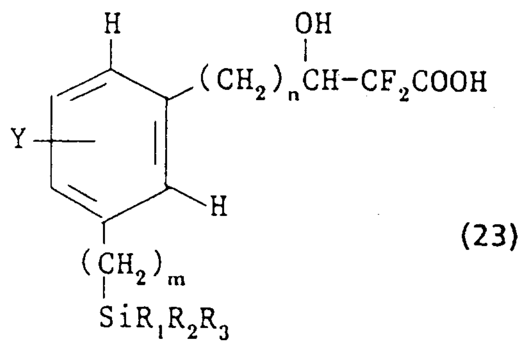

(23)

with a compound of the formula

$$(NHCHR_7C(O))_q-R_8$$

with $R_7$ being the residue of an α-amino acid, q is an integer 1 to 4 and $R_8$ is $OR_5$ or $NHR_5$, followed by optionally converting the products of steps (a) thru (i) to their pharmaceutically acceptable salts.

**14.** A process for the preparation of drugs useful for inhibiting acetylchlolinesterase, characterized by mixing a compound according to any one of claims 1 to 12 or pharmaceutically acceptable salts thereof, as active ingredient, with a pharmaceutically acceptable vehicle.

**15.** A process for the preparation of drugs useful for the treatment of Alzheimer's disease and senile dementia, characterized by mixing a compound according to any one of claims 1 to 12 or pharmaceutically acceptable salts thereof, as active ingredient, with a pharmaceutically acceptable vehicle.

## Patentansprüche

**Patentansprüche für folgende Vertragsstaaten : AT, BE, CH, DE, DK, FR, GB, IT, LI, LU, NL, SE**

**1.** Verbindung der Formel

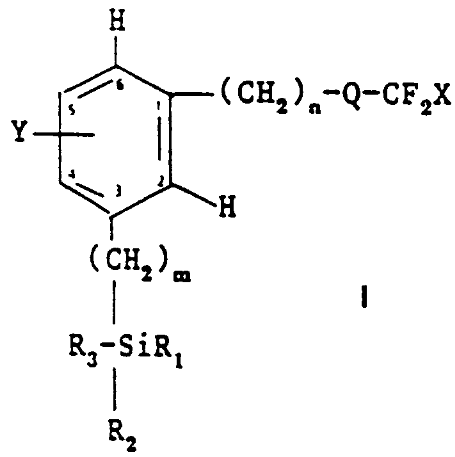

I

und die pharmazeutisch verträglichen Salze davon, wobei m und n jeweils 0 oder 1 bedeuten, mit der Maßgabe, daß die Summe von m und n weniger als zwei ist,
Q

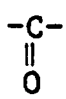
,
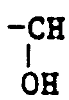
oder
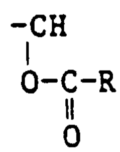

bedeutet, wobei R ein Wasserstoffatom oder einen $C_{1-10}$-Alkylrest darstellt,
X X' oder X'' bedeutet, wobei
X' ein Wasserstoff-, Brom-, Chlor-, Fluoratom oder $R_4$ bedeutet, und
X'' $COR_9$, $CO_2R_5$, $CONHR_5$ oder $COR_6$ darstellt,
$R_1$, $R_2$, $R_3$ und $R_4$ jeweils einen $C_{1-10}$-Alkylrest, oder $(CH_2)_p$-Arylrest bedeuten, wobei p 0, 1 oder 2 bedeutet,
$R_5$ ein Wasserstoffatom, eine $C_{1-10}$-Alkyl-, Phenyl-, Benzyl- oder Phenethylgruppe darstellt,
$R_9$ eine $C_{1-10}$-Alkyl-, Phenyl-, Benzyl- oder Phenethylgruppe darstellt,
$R_6$ einen Rest $(NHCHR_7C(O))_qR_8$ bedeutet, wobei $R_7$ einen Rest einer natürlich vorkommenden $\alpha$-Aminosäure bedeutet, q 1 bis 4 darstellt und $R_8$ $OR_5$ oder $NHR_5$ bedeutet,
Y ein Wasserstoffatom, eine Hydroxy-, $C_{1-6}$-Alkyl-, $C_{1-6}$-Alkoxy-, Hydroxy-$C_{1-6}$-alkyl-, Amino-$C_{1-6}$-alkyl-, Amino-, Azido-, Cyano-, $CO_2R_5$, $COR_9$, $-SO_3H$-Gruppe, ein Brom-, Chlor-, Fluoratom oder den Rest $-(CH_2)_xSiR_1R_2R_3$ bedeutet, wobei X 0, 1 oder 2 darstellt.

**2.** Verbindung nach Anspruch 1, wobei Q

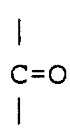

bedeutet.

**3.** Verbindung nach Anspruch 1, wobei Q

|
CHOH
|

bedeutet

**4.** Verbindung nach Anspruch 1, wobei Q

|
HCOC(O)R
|

bedeutet.

**5.** Verbindung nach Anspruch 2, wobei X X' bedeutet.

**6.** Verbindung nach Anspruch 2, wobei X X'' bedeutet.

**7.** Verbindung nach Anspruch 2, wobei m und n jeweils 0 bedeuten.

**8.** Verbindung nach Anspruch 2, wobei m 1 bedeutet.

**9.** Verbindung nach Anspruch 2, wobei n 1 bedeutet.

**10.** Verbindung nach Anspruch 5, wobei X' ein Fluoratom bedeutet.

**11.** Verbindung nach Anspruch 1, wobei mindestens ein Rest von $R_1$ und $R_2$ eine Methyl- oder Ethylgruppe und $R_3$ eine Methyl-, Ethyl- oder Octylgruppe bedeutet

**12.** Verbindungen nach Anspruch 2, die in der nachstehenden Tabelle gekennzeichnet werden:

| $SiR_1R_2R_3$ in 3-Stellung | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, Et | 0 | 0 | F | H |
| $CH_3$, $CH_3$, $CH_3$ | 0 | 0 | F | 5- $SiCH_3CH_3CH_3$ |
| octyl, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$, | 0 | 0 | $-CONHCH_3$ | H |
| wobei Et die Ethylgruppe bedeutet und Q $>C = 0$ | | | | |

darstellt.

**13.** Verfahren zur Herstellung einer Verbindung der Formel

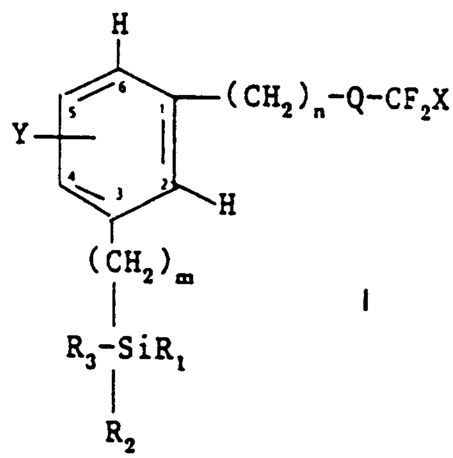

I

und des pharmazeutisch verträglichen Salze davon, wobei m und n jeweils 0 oder 1 bedeuten, mit der Maßgabe, daß die Summe von m und n weniger als zwei ist,
Q

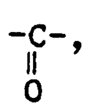 , 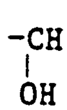 oder 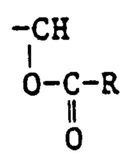

bedeutet, wobei R ein Wasserstoffatom oder einen $c_{1-10}$-Alkylrest darstellt,
X X' oder X'' bedeutet, wobei
X' ein Wasserstoff-, Brom-, Chlor-, Fluoratom oder $R_4$ bedeutet, und
X'' $COR_9$, $CO_2R_5$, $CONHR_5$ oder $COR_6$ darstellt,
$R_1$, $R_2$, $R_3$ und $R_4$ jeweils einen $C_{1-10}$-Alkylrest, oder $(CH_2)_p$-Arylrest bedeuten, wobei p 0, 1 oder 2 bedeutet,
$R_5$ ein Wasserstoffatom, eine $C_{1-10}$-Alkyl-, Phenyl-, Benzyl- oder Phenethylgruppe bedeutet,
$R_9$ eine $C_{1-10}$-Alkyl-, Phenyl-, Benzyl- oder Phenethylgruppe darstellt,

$R_6$ den $(NHCHR_7C(O))_qR_8$-Rest bedeutet, wobei $R_7$ einen Rest einer natürlich vorkommenden $\alpha$-Aminosäure bedeutet, q 1 bis 4 darstellt und $R_8$ $OR_5$ oder $NHR_5$ bedeutet,
Y ein Wasserstoffatom, eine Hydxoxy-, $C_{1-6}$-Alkyl-, $C_{1-6}$-Alkoxy-, Hydxoxy-$C_{1-6}$-alkyl-, Amino-$C_{1-6}$-alkyl-, Amino-, Azido-, Cyano-, $CO_2R_5$, $COR_9$, $-SO_3H$-Gruppe, ein Brom-, Chlor-, Fluoratom oder den Rest $-(CH_2)_xSiR_1R_2R_3$ bedeutet, wobei X 0, 1 oder 2 darstellt, umfassend

(a) zur Herstellung einer Verbindung der Untergruppen-Formel

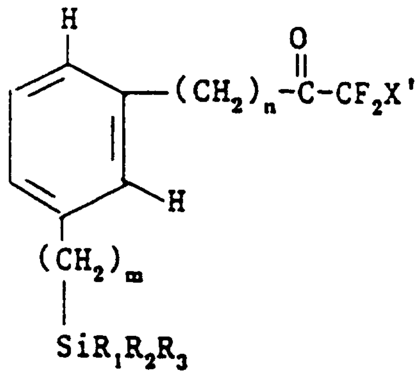

(2)

die Bereitstellung eines Lithiumderivates einer Verbindung der Formeln

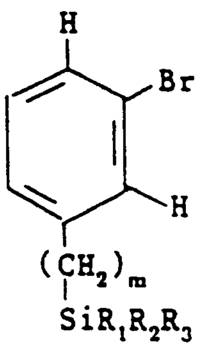

(3)

und

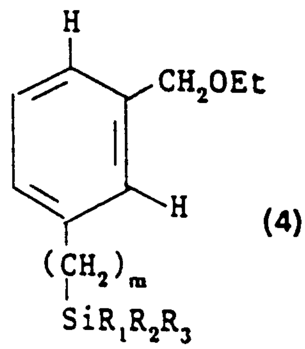

(4)

und Umsetzen des Lithiumderivates mit einer Säure (als ihr Lithiumsalz), einem Ester oder einem Säurechlorid der Formeln $X'CF_2CO_2R$ oder $X_4CF_2COCl$, wobei R ein Wasserstoffatom oder einen $C_{1-6}$-Alkylrest bedeutet;

(b) zur Herstellung einer Verbindung der Untergruppen-Formel

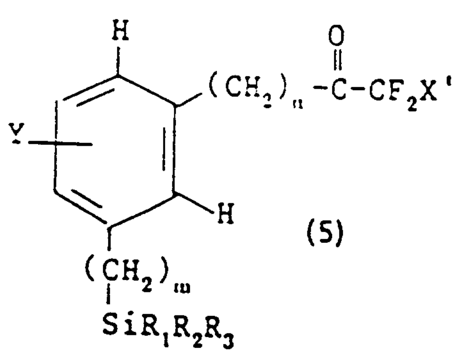

(5)

wobei Y eine andere Bedeutung als ein Wasserstoffatom besitzt, die Oxidation einer Verbindung der Formel

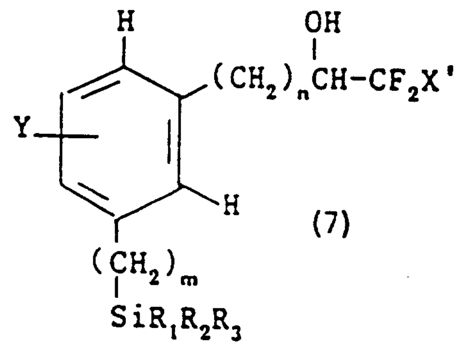

(7)

wobei Y eine andere Bedeutung als ein Wasserstoffatom besitzt;
c) zur Herstellung einer Verbindung der Untergruppen-Formel

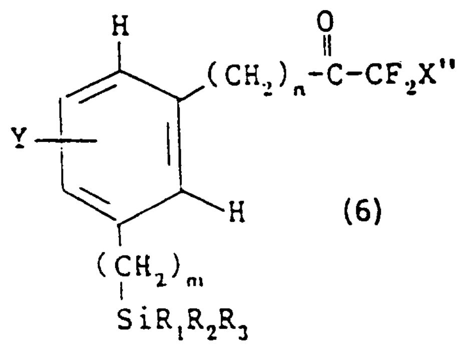

(6)

die Oxidation einer Verbindung der Formel

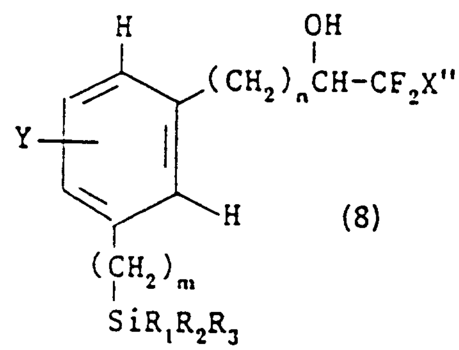

(8)

d) zur Herstellung einer Verbindung der Untergruppen-Formel

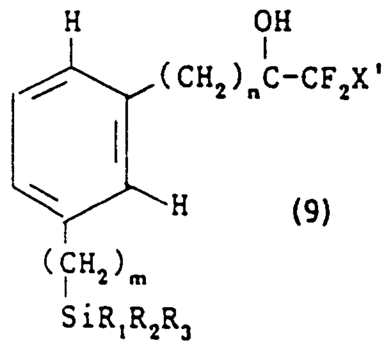

(9)

die chemische Reduktion einer Verbindung der vorstehenden Formel (2);

e) zur Herstellung einer Verbindung der Untergruppen-Formel

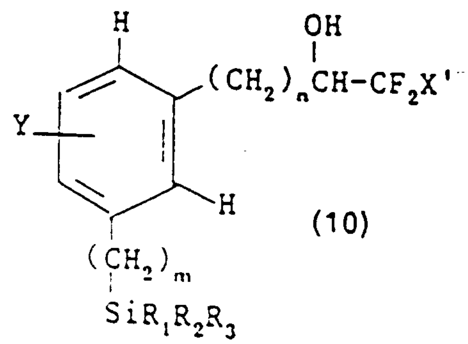

(10)

wobei Y eine andere Bedeutung als ein Wasserstoffatom besitzt, die Hydrolyse einer Verbindung der Formel

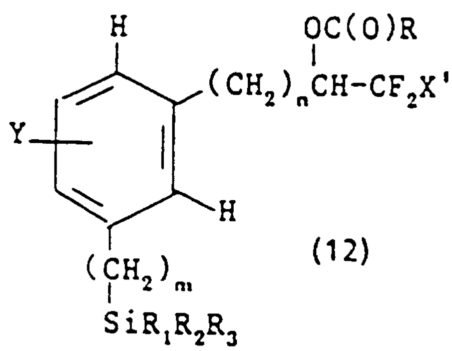

(12)

wobei Y eine andere Bedeutung als ein Wasserstoffatom besitzt;

f) zur Herstellung einer Verbindung der Untergruppen-Formel

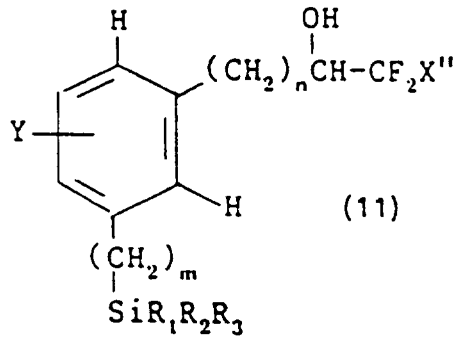

(11)

die Hydrolyse einer Verbindung der Formel

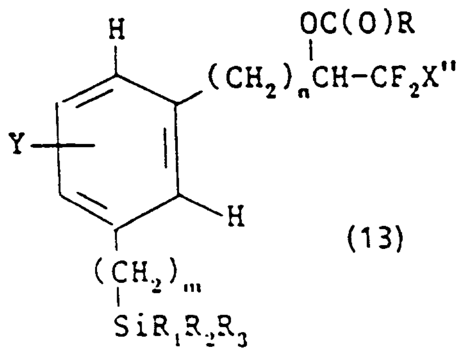

(13)

g) zur Herstellung einer Verbindung der Untergruppen-Formel

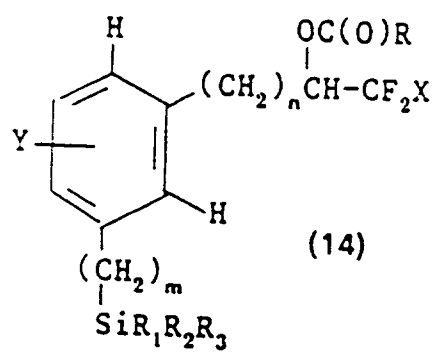

(14)

das Verestern einer Verbindung der Formel

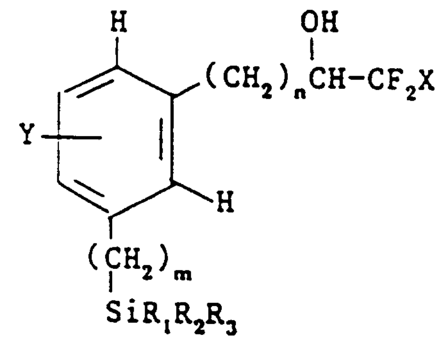

(15)

mit einem Acylhalogenid;

h) zur Herstellung einer Verbindung der Untergruppen-Formel

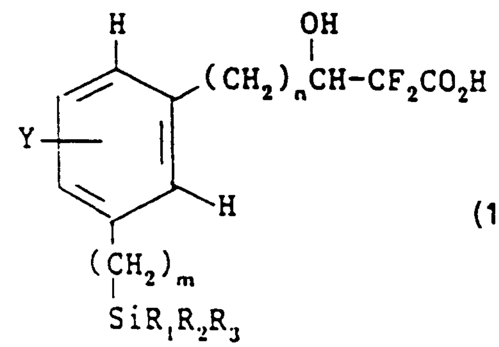

(16)

die Hydrolyse einer Verbindung der Formel

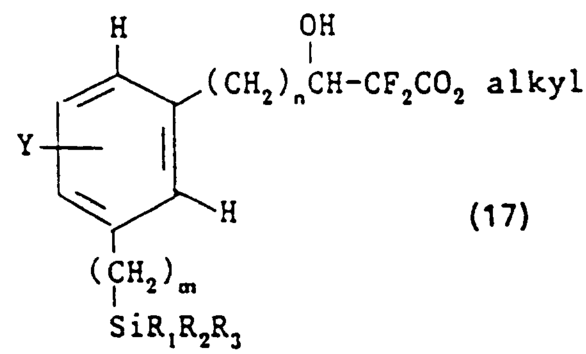

(17)

durch Umsetzen mit Lithiumhydroxid in 80%igem Ethylenglykoldimethylether;

i) zur Herstellung einer Verbindung der Untergruppen-Formel

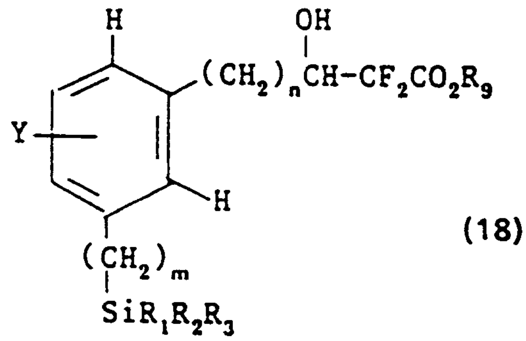

(18)

das Umsetzen einer Verbindung der Formel

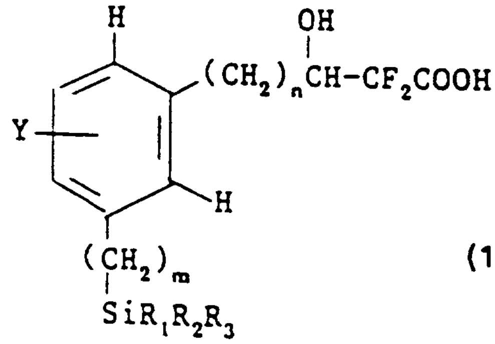

(19)

mit einer Verbindung der Formel $R_9OH$ in Gegenwart von Dicyclohexylcarbodiimid und Dimethylaminopyridin;

j) zur Herstellung einer Verbindung der Untergruppen-Formel

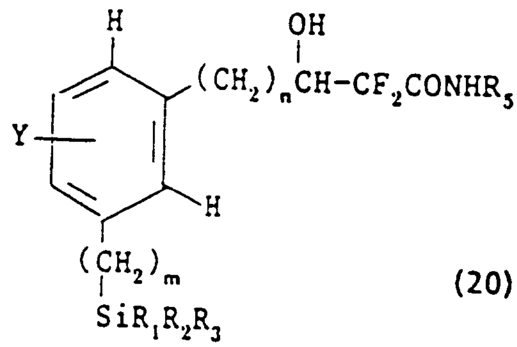

(20)

das Umsetzen einer Verbindung der Formel

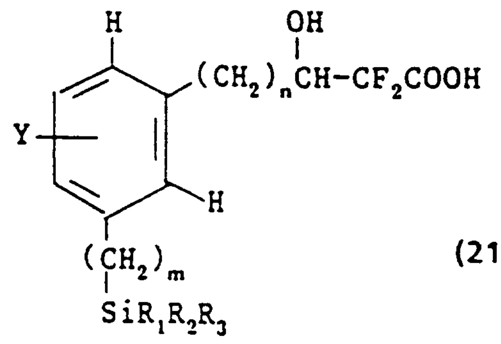

(21)

mit einem Amin der Formel $R_5NH_2$ in Gegenwart von Dicyclohexylcarbodiimid und 1-Hydroxybenzotriazol;

k) zur Herstellung einer Verbindung der Untergruppen-Formel

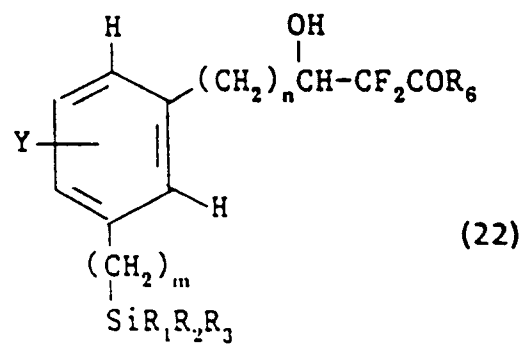

(22)

das Umsetzen einer Verbindung der Formel

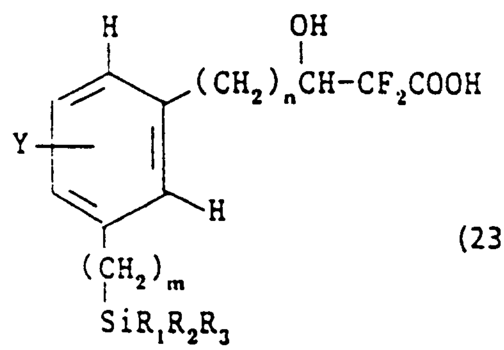

(23)

mit einer Verbindung der Formel

$$(NHCHR_7C(O)-)_qR_8,$$

wobei $R_7$ einen Rest einer $\alpha$-Aminosäure bedeutet, q eine ganze Zahl von 1 bis 4 bedeutet, und $R_8$ $OR_5$ oder $NHR_5$ bedeutet, und gegebenenfalls anschließende Umwandlung der Produkte der Schritte (a) bis (i) zu ihren pharmazeutisch verträglichen Salzen.

**14.** Verbindung gemäß einem der Ansprüche 1 bis 12 zur Verwendung als pharmazeutisch wirksame Verbindung.

**15.** Verwendung der Verbindungen gemäß einem der Ansprüche 1 bis 12 zur Herstellung von Arzneimitteln, die zur Hemmung der Acetylcholinesterase nützlich sind.

**16.** Verwendung der Verbindungen gemäß einem der Ansprüche 1 bis 12 zur Herstellung von Arzneimitteln, die zur Behandlung der Alzheimerschen Krankheit und der Altersdemenz nützlich sind.

**Patentansprüche für folgenden Vertragsstaat : ES**

**1.** Verfahren zur Herstellung einer Verbindung der Formel

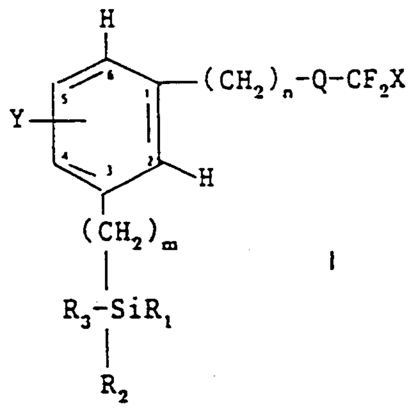

I

und der pharmazeutisch verträglichen Salze davon, wobei m und n jeweils 0 oder 1 bedeuten, mit der Maßgabe, daß die Summe von m und n weniger als zwei ist,
Q

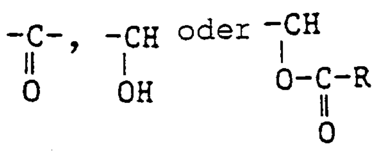

bedeutet, wobei R ein Wasserstoffatom
oder einen $C_{1-10}$-Alkylrest darstellt,
X X' oder X'' bedeutet, wobei
X' ein Wasserstoff-, Brom-, Chlor-, Fluoratom oder $R_4$ bedeutet, und
X'' $COR_9$, $CO_2R_5$, $CONHR_5$ oder $COR_6$ darstellt,
$R_1$, $R_2$, $R_3$ und $R_4$ jeweils einen $C_{1-10}$-Alkylrest, oder $(CH_2)_p$-Arylrest bedeuten, wobei p 0, 1 oder 2 bedeutet,
$R_5$ ein Wasserstoffatom, eine $C_{1-10}$-Alkyl-, Phenyl-, Benzyl- oder Phenethylgruppe bedeutet,
$R_9$ eine $C_{1-10}$-Alkyl-, Phenyl-, Benzyl- oder Phenethylgruppe darstellt,
$R_6$ den $(NHCHR_7C(O))_qR_8$-Rest bedeutet, wobei $R_7$ einen Rest einer natürlich vorkommenden $\alpha$-Aminosäure bedeutet, q 1 bis 4 darstellt und $R_8$ $OR_5$ oder $NHR_5$ bedeutet,
Y ein Wasserstoffatom, eine Hydroxy-, $C_{1-6}$-Alkyl-, $C_{1-6}$-Alkoxy-, Hydroxy-$C_{1-6}$-alkyl-, Amino-$C_{1-6}$-alkyl-, Amino-, Azido-, Cyano-, $CO_2R_5$, $COR_9$, $-SO_3H$-Gruppe, ein Brom-, Chlor-, Fluoratom oder den Rest $-(CH_2)_xSiR_1R_2R_3$ bedeutet, wobei X 0, 1 oder 2 darstellt, umfassend

(a) zur Herstellung einer Verbindung der Untergruppen-Formel

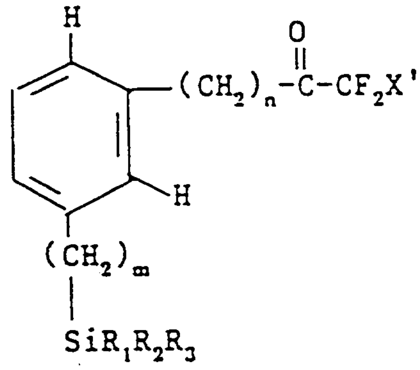

(2)

die Bereitstellung eines Lithiumderivates einer Verbindung der Formeln

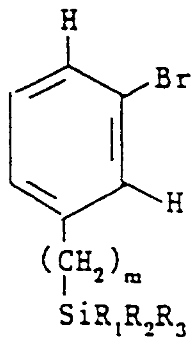

(3)

und

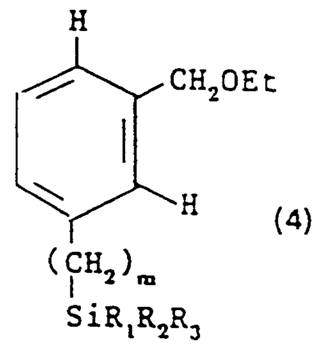

(4)

und Umsetzen des Lithiumderivates mit einer Säure (als ihr Lithiumsalz), einem Ester oder einem Säurechlorid der Formeln $X'CF_2CO_2R$ oder $X_4CF_2COCl$, wobei R ein Wasserstoffatom oder einen $C_{1-6}$-Alkylrest bedeutet;

(b) zur Herstellung einer Verbindung der Untergruppen-Formel

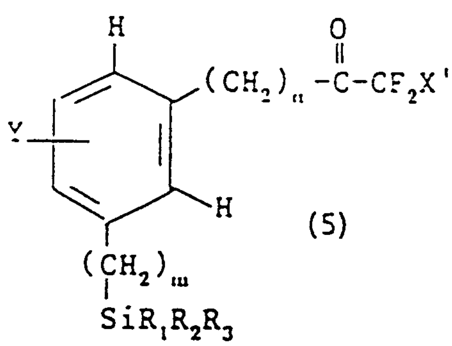

(5)

wobei Y eine andere Bedeutung als ein Wasserstoffatom besitzt, die Oxidation einer Verbindung der Formel

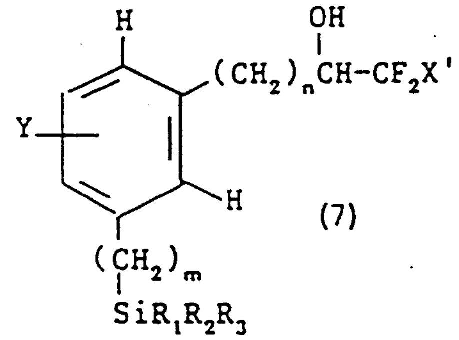

(7)

wobei Y eine andere Bedeutung als ein Wasserstoffatom besitzt;
c) zur Herstellung einer Verbindung der Untergruppen-Formel

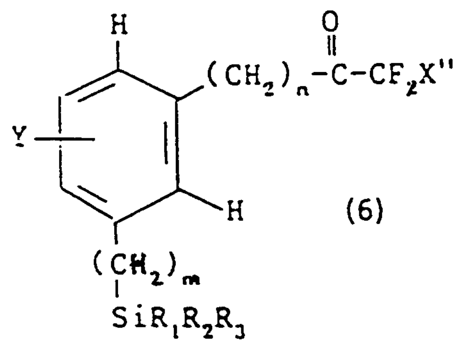

(6)

die Oxidation einer Verbindung der Formel

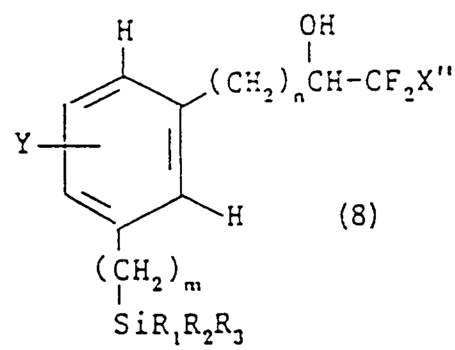

(8)

d) zur Herstellung einer Verbindung der Untergruppen-Formel

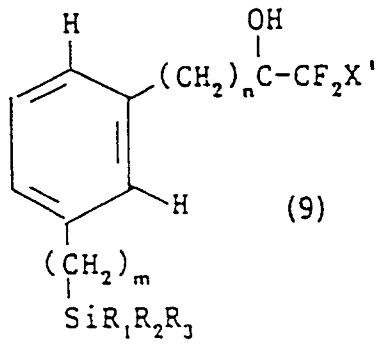

(9)

die chemische Reduktion einer Verbindung der vorstehenden Formel (2);
e) zur Herstellung einer Verbindung der Untergruppen-Formel

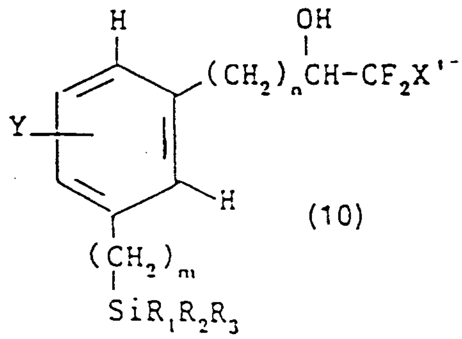

(10)

wobei Y eine andere Bedeutung als ein Wasserstoffatom besitzt, die Hydrolyse einer Verbindung der Formel

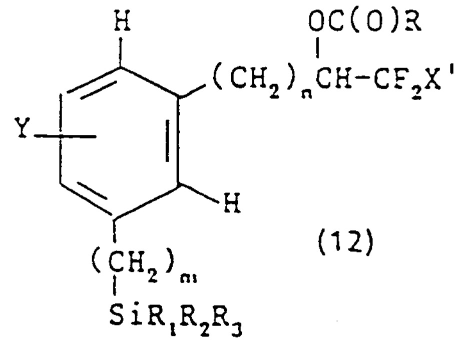

(12)

wobei Y eine andere Bedeutung als ein Wasserstoffatom besitzt;

f) zur Herstellung einer Verbindung der Untergruppen-Formel

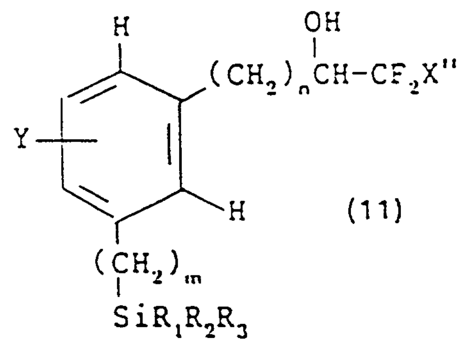

(11)

die Hydrolyse einer Verbindung der Formel

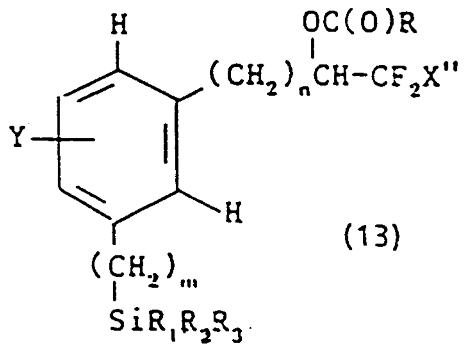

(13)

g) zur Herstellung einer Verbindung der Untergruppen-Formel

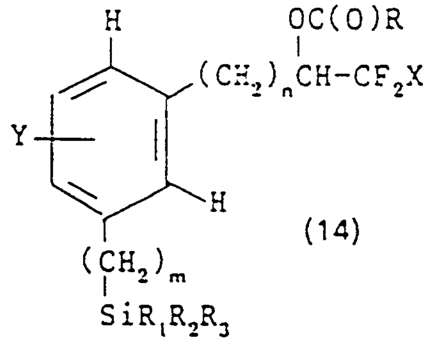

(14)

das Verestern einer Verbindung der Formel

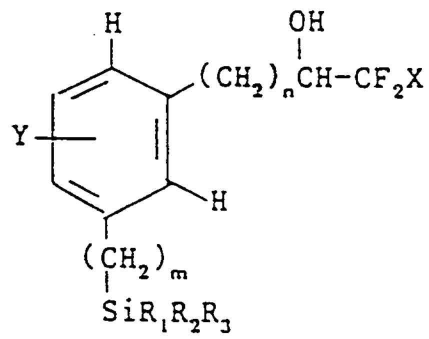

(15)

mit einem Acylhalogenid;

h) zur Herstellung einer Verbindung der Untergruppen-Formel

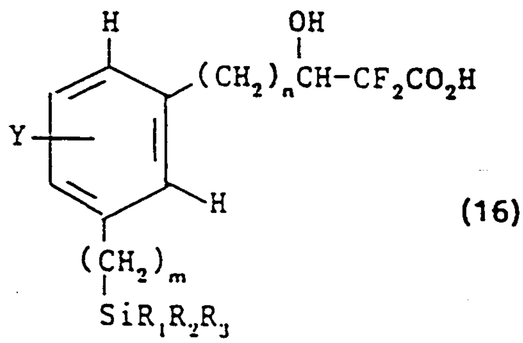

(16)

die Hydrolyse einer Verbindung der Formel

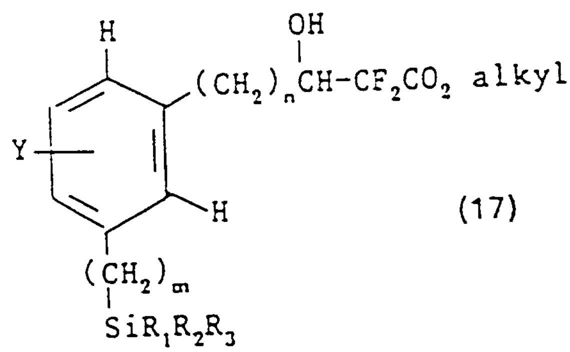

(17)

durch Umsetzen mit Lithiumhydroxid in 80%igem Ethylenglykoldimethylether;

i) zur Herstellung einer Verbindung der Untergruppen-Formel

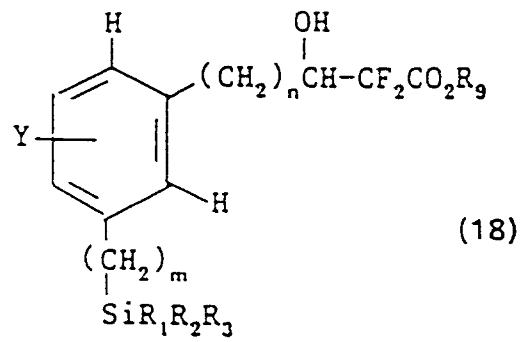

(18)

das Umsetzen einer Verbindung der Formel

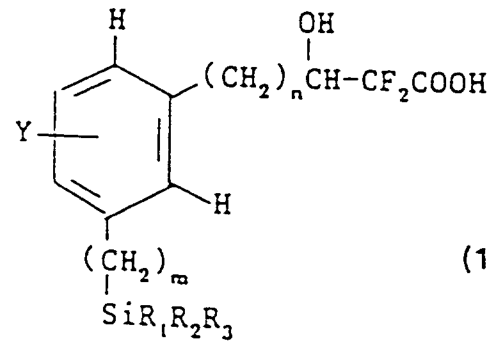

(19)

mit einer Verbindung der Formel $R_9OH$ in Gegenwart von Dicyclohexylcarbodiimid und Dimethylaminopyridin;

j) zur Herstellung einer Verbindung der Untergruppen-Formel

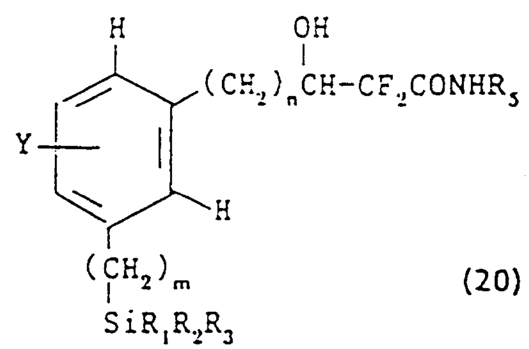

(20)

das Umsetzen einer Verbindung der Formel

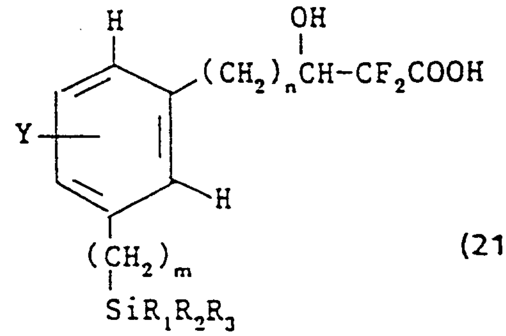

(21)

mit einem Amin der Formel $R_5NH_2$ in Gegenwart von Dicyclohexylcarbodiimid und 1-Hydroxybenzotriazol;

k) zur Herstellung einer Verbindung der Untergruppen-Formel

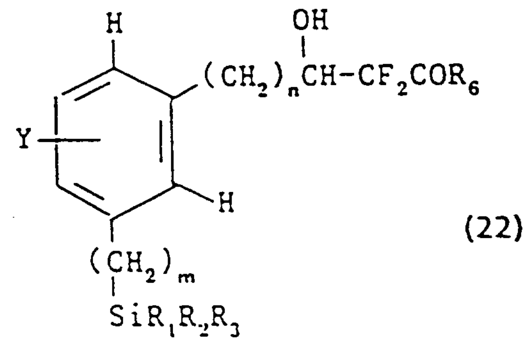

(22)

das Umsetzen einer Verbindung der Formel

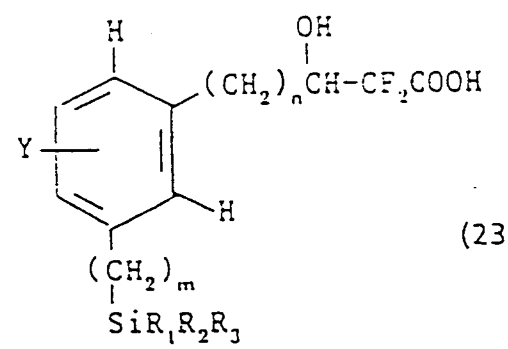

(23)

mit einer Verbindung der Formel

$$(NHCHR_7C(O))_qR_8,$$

wobei $R_7$ einen Rest einer $\alpha$-Aminosäure bedeutet, q eine ganze Zahl von 1 bis 4 bedeutet, und $R_8$ $OR_5$ oder $NHR_5$ bedeutet, und gegebenenfalls anschließende Umwandlung der Produkte der Schritte (a) bis (i) zu ihren pharmazeutisch verträglichen Salzen.

**2.** Verfahren nach Anspruch 1 zur Herstellung einer Verbindung der Formel I, wobei mindestens ein Rest von $R_1$ und $R_2$ eine Methyl- oder Ethylgruppe und $R_3$ eine Methyl-, Ethyl- oder Octylgruppe bedeutet, dadurch gekennzeichnet, daß in den Stufen (a), (b), (c), (d), (e), (f), (g), (h), (i), (j) oder (k) die

Verbindungen der Formeln (3) und (4), (7), (8), (2), (12), (13), (15), (17), (19), (21) oder (23) gemäß Anspruch 1, in denen $R_1$, $R_2$ und $R_3$ die vorstehende Bedeutung haben, verwendet werden.

**3.** Verfahren gemäß Anspruch 1 zur Herstellung von Verbindungen der Formel I, wobei Q >C = O und $R_1$, $R_2$, $R_3$, m, n, X und Y die nachstehende Bedeutung haben:

| $SiR_1R_2R_3$ in 3-Stellung | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, Et | 0 | 0 | F | H |
| $CH_3$, $CH_3$, $CH_3$ | 0 | 0 | F | 5- $SiCH_3CH_3CH_3$ |
| octyl, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| wobei Et die Ethylgruppe bedeutet und Q >C = 0 | | | | |

und den pharmazeutisch verträglichen Salzen davon, dadurch gekennzeichnet, daß

- in Schritt a) Verbindungen der Formeln (3) und (4), in denen m, $R_1$, $R_2$ und $R_3$ die vorstehende Bedeutung haben und Verbindungen der Formeln $X'CF_2CO_2R$ oder $X_4CF_2COCl$, in denen X die vorstehende Bedeutung hat, X' H oder F darstellt und R die in Anspruch 1 angegebene Bedeutung hat, verwendet werden ;
- in Schritt b) eine Verbindung der Formel (7) verwendet wird, in der Y 5-$SiCH_3CH_3CH_3$ bedeutet, und n, m, $R_1$, $R_2$ und $R_3$ die vorstehende Bedeutung haben und X' H oder F bedeutet;
- in Schritt (c) eine Verbindung der Formel (8) verwendet wird, in der Y 5-$SiCH_3CH_3CH_3$ bedeutet, und n, m, $R_1$, $R_2$ und $R_3$ die vorstehende Bedeutung haben und X'' $-CH_3$, $-COCH_3$, $-COOCH_3$ oder $-CONHCH_3$ bedeutet.

**4.** Verfahren zur Herstellung von Arzneimitteln, die zur Hemmung der Acetylcholinesterase nützlich sind, dadurch gekennzeichnet, daß man eine gemäß dem Verfahren nach einem der Ansprüche 1 bis 3 hergestellte Verbindung oder pharmazeutisch verträgliche Salze davon, als Wirkstoff, mit einem pharmazeutisch verträglichen Träger mischt.

**5.** Verfahren zur Herstellung von Arzneimitteln, die zur Behandlung der Alzheimerschen Krankheit und der Altersdemenz nützlich sind, dadurch gekennzeichnet, daß man eine nach dem Verfahren gemäß einem der Ansprüche 1 bis 3 hergestellte Verbindung oder pharmazeutisch verträgliche Salze davon, als Wirkstoff, mit einem pharmazeutisch verträglichen Träger mischt.

**Patentansprüche für folgenden Vertragsstaat : GR**

1. Verbindung der Formel

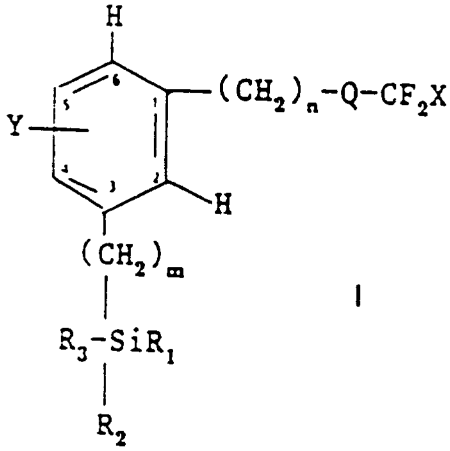

I

und die pharmazeutisch verträglichen Salze davon, wobei m und n jeweils 0 oder 1 bedeuten, mit der Maßgabe, daß die Summe von m und n weniger als zwei ist,
Q

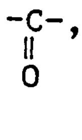, 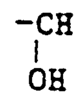 oder 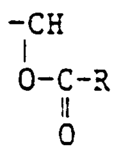

bedeutet, wobei R ein Wasserstoffatom oder einen $C_{1-10}$-Alkylrest darstellt,
X X' oder X'' bedeutet, wobei
X' ein Wasserstoff-, Brom-, Chlor-, Fluoratom oder $R_4$ bedeutet, und
X'' $COR_9$, $CO_2R_5$, $CONHR_5$ oder $COR_6$ darstellt,
$R_1$, $R_2$, $R_3$ und $R_4$ jeweils einen $C_{1-10}$-Alkylrest, oder $(CH_2)_p$-Arylrest bedeuten, wobei p 0, 1 oder 2 bedeutet,
$R_5$ ein Wasserstoffatom, eine $C_{1-10}$-Alkyl-, Phenyl-, Benzyl- oder Phenethylgruppe darstellt,
$R_9$ eine $C_{1-10}$-Alkyl-, Phenyl-, Benzyl- oder Phenethylgruppe darstellt,
$R_6$ einen Rest $(NHCHR_7C(O))_qR_8$ bedeutet, wobei $R_7$ einen Rest einer natürlich vorkommenden α-Aminosäure bedeutet, q 1 bis 4 darstellt und $R_8$ $OR_5$ oder $NHR_5$ bedeutet,
Y ein Wasserstoffatom, eine Hydroxy-, $C_{1-6}$-Alkyl-, $C_{1-6}$-Alkoxy-, Hydroxy-$C_{1-6}$-alkyl-, Amino-$C_{1-6}$-alkyl-, Amino-, Azido-, Cyano-, $CO_2R_5$, $COR_9$, $-SO_3H$-Gruppe, ein Brom-, Chlor-, Fluoratom oder den Rest $-(CH_2)_xSiR_1R_2R_3$ bedeutet, wobei X 0, 1 oder 2 darstellt.

2. Verbindung nach Anspruch 1, wobei Q

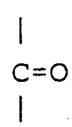

bedeutet.

**3.** Verbindung nach Anspruch 1, wobei Q

|
CHOH
|

bedeutet

**4.** Verbindung nach Anspruch 1, wobei Q

|
HCOC(O)R
|

bedeutet.

**5.** Verbindung nach Anspruch 2, wobei X X' bedeutet.

**6.** Verbindung nach Anspruch 2, wobei X X'' bedeutet.

**7.** Verbindung nach Anspruch 2, wobei m und n jeweils 0 bedeuten.

**8.** Verbindung nach Anspruch 2, wobei m 1 bedeutet.

**9.** Verbindung nach Anspruch 2, wobei n 1 bedeutet.

**10.** Verbindung nach Anspruch 5, wobei X' ein Fluoratom bedeutet.

**11.** Verbindung nach Anspruch 1, wobei mindestens ein Rest von $R_1$ und $R_2$ eine Methyl- oder Ethylgruppe und $R_3$ eine Methyl-, Ethyl- oder Octylgruppe bedeutet.

**12.** Verbindungen nach Anspruch 2, die in der nachstehenden Tabelle gekennzeichnet werden:

| $SiR_1R_2R_3$ in 3-Stellung | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, Et | 0 | 0 | F | H |
| $CH_3$, $CH_3$, $CH_3$ | 0 | 0 | F | 5- $SiCH_3CH_3CH_3$ |
| octyl, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| wobei Et die Ethylgruppe bedeutet und Q $>C = 0$ | | | | |

darstellt.

**13.** Verfahren zur Herstellung einer Verbindung der Formel

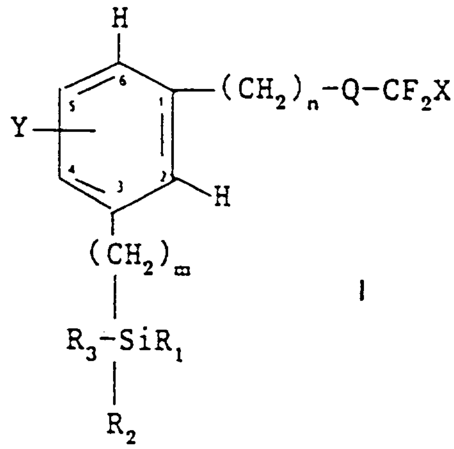

I

und der pharmazeutisch verträglichen Salze davon, wobei m und n jeweils 0 oder 1 bedeuten, mit der Maßgabe, daß die Summe von m und n weniger als zwei ist,
Q

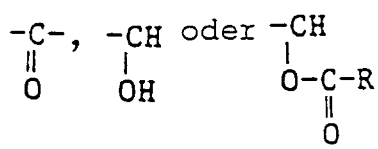

bedeutet, wobei R ein Wasserstoffatom
oder einen $C_{1-10}$-Alkylest darstellt,
X X' oder X'' bedeutet, wobei
X' ein Wasserstoff-, Brom-, Chlor-, Fluoratom oder $R_4$ bedeutet, und
X'' $COR_9$, $CO_2R_5$, $CONHR_5$ oder $COR_6$ darstellt,
$R_1$, $R_2$, $R_3$ und $R_4$ jeweils einen $C_{1-10}$-Alkylrest, oder $(CH_2)_p$-Arylrest bedeuten, wobei p 0, 1 oder 2 bedeutet,
$R_5$ ein Wasserstoffatom, eine $C_{1-10}$-Alkyl-, Phenyl-, Benzyl- oder Phenethylgruppe bedeutet,
$R_9$ eine $C_{1-10}$-Alkyl-, Phenyl-, Benzyl- oder Phenethylgruppe darstellt,
$R_6$ den $(NHCHR_7C(O))_qR_8$-Rest bedeutet, wobei $R_7$ einen Rest einer natürlich vorkommenden $\alpha$-Aminosäure bedeutet, q 1 bis 4 darstellt und $R_8$ $OR_5$ oder $NHR_5$ bedeutet,
Y ein Wasserstoffatom, eine Hydroxy-, $C_{1-6}$-Alkyl-, $C_{1-6}$-Alkoxy-, Hydroxy-$C_{1-6}$-alkyl-, Amino-$C_{1-6}$-alkyl-, Amino-, Azido-, Cyano-, $CO_2R_5$, $COR_9$, $-SO_3H$-Gruppe, ein Brom-, Chlor-, Fluoratom oder den Rest $-(CH_2)_xSiR_1R_2R_3$ bedeutet, wobei X 0, 1 oder 2 darstellt, umfassend
(a) zur Herstellung einer Verbindung der Untergruppen-Formel

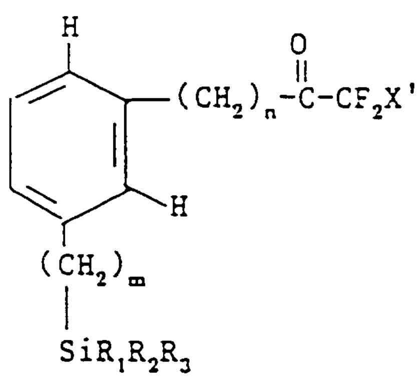

(2)

die Bereitstellung eines Lithiumderivates einer Verbindung der Formeln

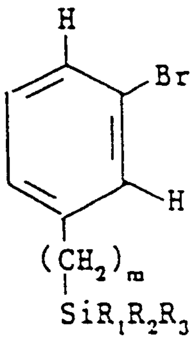

(3)

und

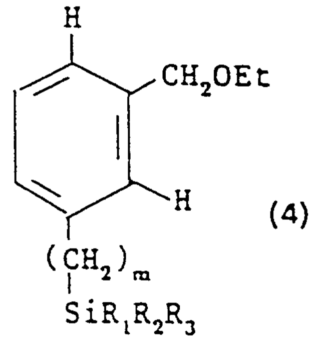

(4)

und Umsetzen des Lithiumderivates mit einer Säure (als ihr Lithiumsalz), einem Ester oder einem Säurechlorid der Formeln $X'CF_2CO_2R$ oder $X_4CF_2COCl$, wobei R ein Wasserstoffatom oder einen $C_{1-6}$-Alkylrest bedeutet;

(b) zur Herstellung einer Verbindung der Untergruppen-Formel

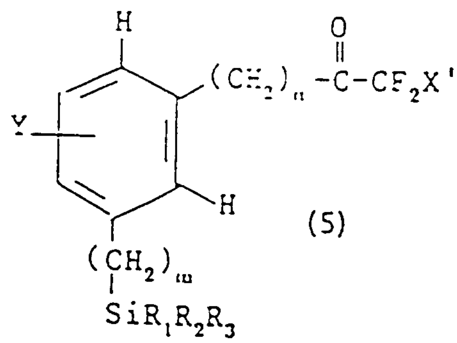

(5)

wobei Y eine andere Bedeutung als ein Wasserstoffatom besitzt, die Oxidation einer Verbindung der Formel

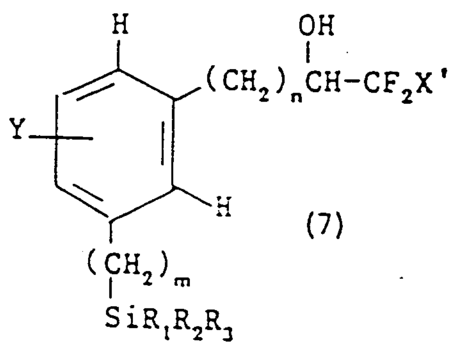

(7)

wobei Y eine andere Bedeutung als ein Wasserstoffatom besitzt;

c) zur Herstellung einer Verbindung der Untergruppen-Formel

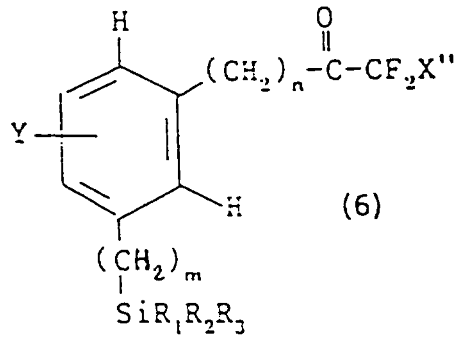

(6)

die Oxidation einer Verbindung der Formel

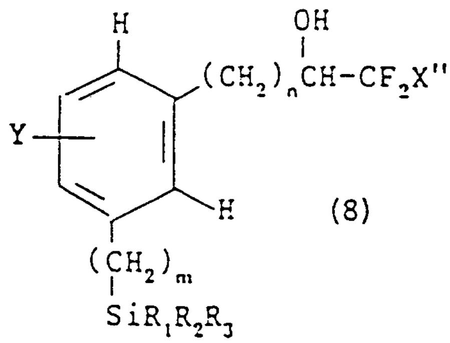

(8)

d) zur Herstellung einer Verbindung der Untergruppen-Formel

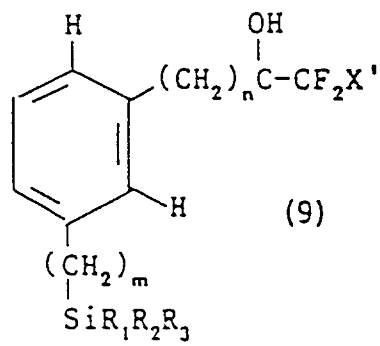

(9)

die chemische Reduktion einer Verbindung der vorstehenden Formel (2);

e) zur Herstellung einer Verbindung der Untergruppen-Formel

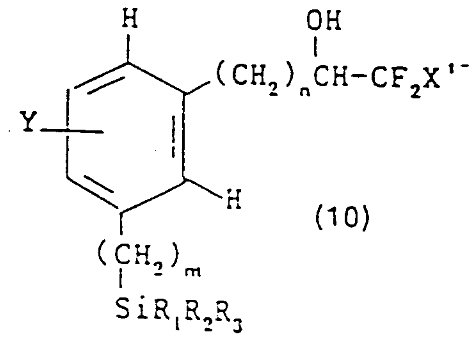

(10)

wobei Y eine andere Bedeutung als ein Wasserstoffatom besitzt, die Hydrolyse einer Verbindung der Formel

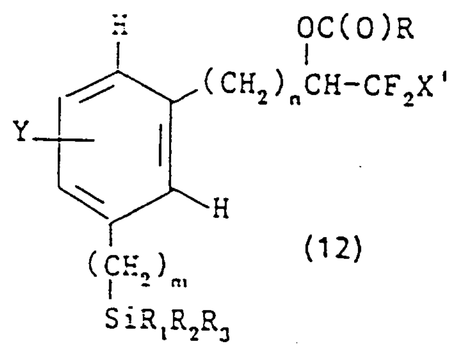

(12)

wobei Y eine andere Bedeutung als ein Wasserstoffatom besitzt;
f) zur Herstellung einer Verbindung der Untergruppen-Formel

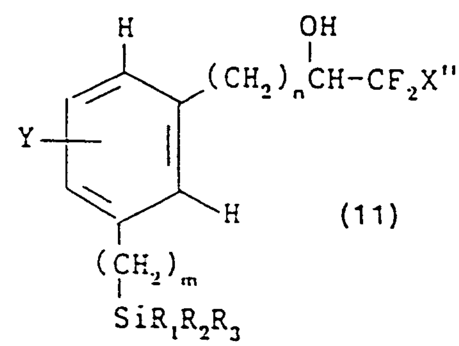

(11)

die Hydrolyse einer Verbindung der Formel

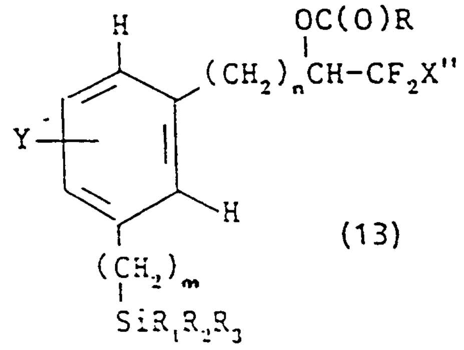

(13)

g) zur Herstellung einer Verbindung der Untergruppen-Formel

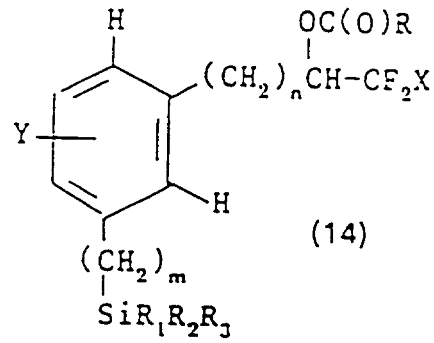

(14)

das Verestern einer Verbindung der Formel

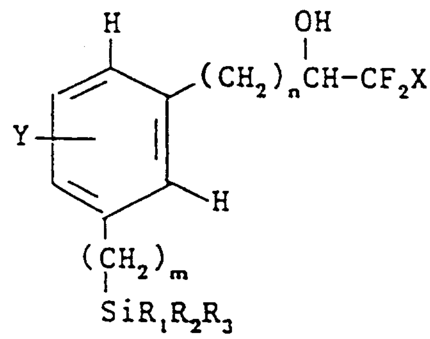

(15)

mit einem Acylhalogenid;

h) zur Herstellung einer Verbindung der Untergruppen-Formel

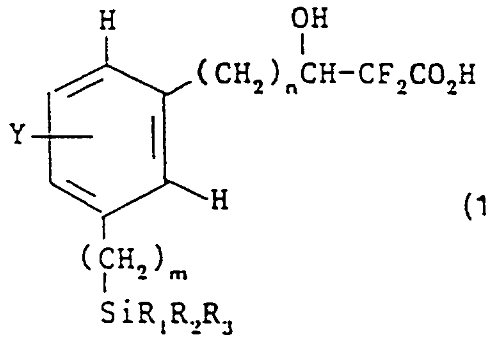

(16)

die Hydrolyse einer Verbindung der Formel

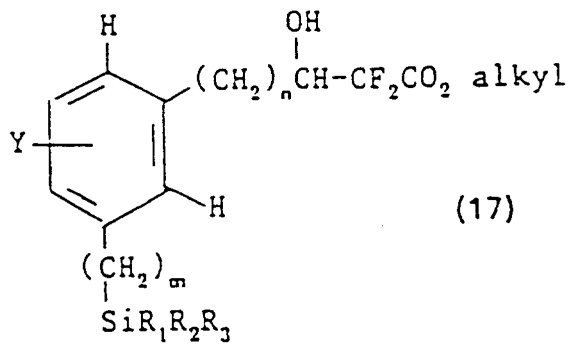

(17)

durch Umsetzen mit Lithiumhydroxid in 80%igem Ethylenglykoldimethylether;
i) zur Herstellung einer Verbindung der Untergruppen-Formel

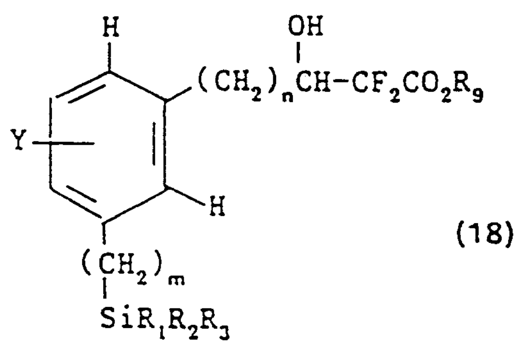

(18)

das Umsetzen einer Verbindung der Formel

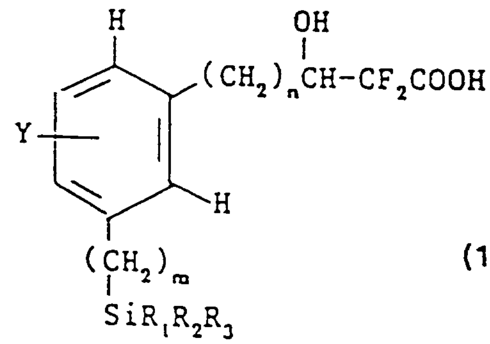

(19)

mit einer Verbindung der Formel $R_9OH$ in Gegenwart von Dicyclohexylcarbodiimid und Dimethylaminopyridin;

j) zur Herstellung einer Verbindung der Untergruppen-Formel

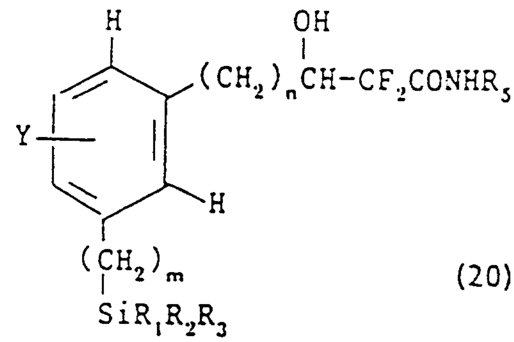

(20)

das Umsetzen einer Verbindung der Formel

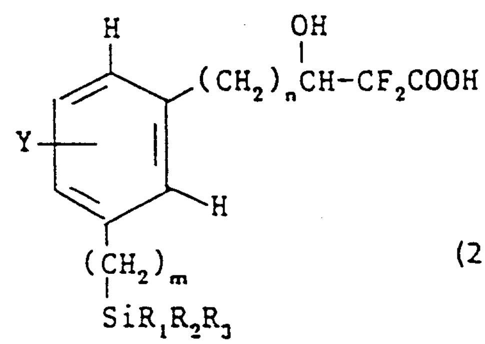

(21)

mit einem Amin der Formel $R_5NH_2$ in Gegenwart von Dicyclohexylcarbodiimid und 1-Hydroxybenzotriazol;

k) zur Herstellung einer Verbindung der Untergruppen-Formel

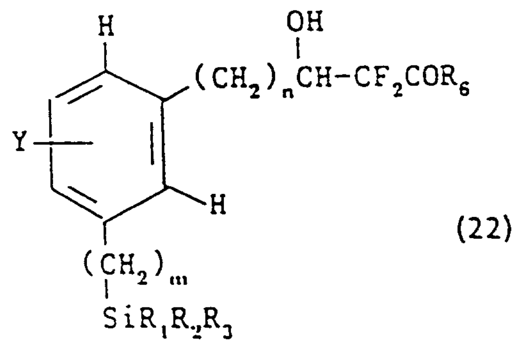

(22)

das Umsetzen einer Verbindung der Formel

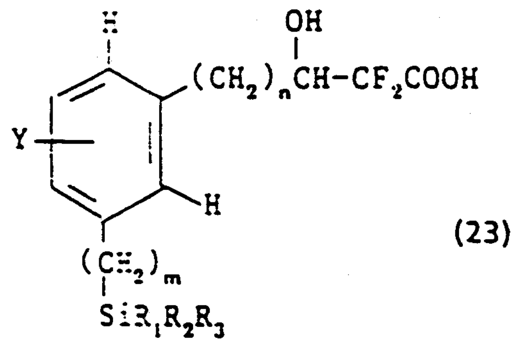

(23)

mit einer Verbindung des Formel

$$(NHCHR_7C(O))_qR_8,$$

wobei $R_7$ einen Rest eines α-Aminosäure bedeutet, q eine ganze Zahl von 1 bis 4 bedeutet, und $R_8$ $OR_5$ oder $NHR_5$ bedeutet, und gegebenenfalls anschließende Umwandlung der Produkte der Schritte (a) bis (i) zu ihren pharmazeutisch verträglichen Salzen.

**14.** Verfahren zur Herstellung von Arzneimitteln, die zur Hemmung der Acetylcholinesterase nützlich sind, dadurch gekennzeichnet, daß man eine Verbindung gemäß einem der Ansprüche 1 bis 12 oder pharmazeutisch verträgliche Salze davon, als Wirkstoff, mit einem pharmazeutisch verträglichen Träger mischt.

**15.** Verfahren zur Herstellung von Arzneimitteln, die zur Behandlung der Alzheimerschen Krankheit und der Altersdemenz nützlich sind, dadurch gekennzeichnet, daß man eine Verbindung gemäß einem der Ansprüche 1 bis 12 oder pharmazeutisch verträgliche Salze davon, als Wirkstoff, mit einem pharmazeutisch verträglichen Träger mischt.

**Revendications**

**Revendications pour les Etats contractants : AT, BE, CH, DE, DK, FR, GB, IT, LI, LU, NL, SE**

1. Composé de formule :

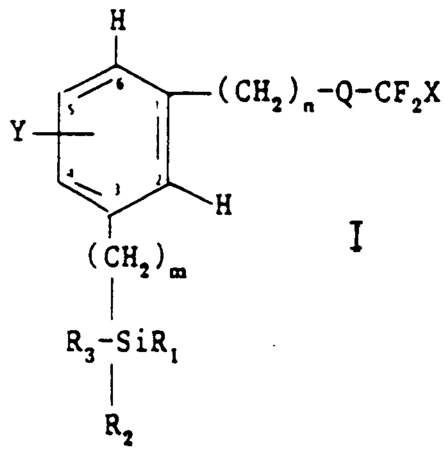

I

et ses sels pharmaceutiquement acceptables, dans laquelle m et n sont chacun 0 ou 1 avec la condition que la somme m + n soit inférieure à 2,
Q est

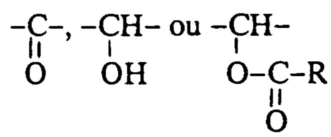

où R est H ou alkyle en $C_1$-$C_{10}$,
X est X' ou X'' et
X' est H, Br, Cl, F ou $R_4$ et
X'' est $COR_9$, $CO_2R_5$, $CONHR_5$ ou $COR_6$,
$R_1$, $R_2$, $R_3$ et $R_4$ étant chacun alkyle en $C_1$-$C_{10}$ ou $-(CH_2)_p$-aryle, où p est égal à 0, 1 ou 2
$R_5$ est H, alkyle en $C_1$-$C_{10}$, phényle, benzyle ou phénéthyle,
$R_9$ est alkyle en $C_1$-$C_{10}$, phényle, benzyle ou phénéthyle,
$R_6$ est $(NHCHR_7C(O))_qR_8$ où $R_7$ est le reste d'un α-aminoacide naturel, q est un nombre de 1 à 4 et $R_8$ est $OR_5$ ou $NHR_5$,
Y est H, OH, alkyle en $C_1$-$C_6$, alcoxy en $C_1$-$C_6$, hydroxyalkyle en $C_1$-$C_6$,aminoalkyle en $C_1$-$C_6$, $NH_2$, azido, CN, $CO_2R_5$, $COR_9$, $-SO_3H$, Br, Cl, F ou $-(CH_2)_xSiR_1R_2R_3$ où x est égal à 0, 1 ou 2.

2. Composé selon la revendication 1 dans lequel Q est

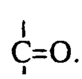

3. Composé selon la revendication 1 dans lequel Q est

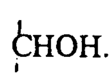

**4.** Composé selon la revendication 1 dans lequel Q est

$$HCOC(O)R$$

**5.** Composé selon la revendication 2 dans lequel X est X'.

**6.** Composé selon la revendication 2 dans lequel X est X''.

**7.** Composé selon la revendication 2 dans lequel m = n = 0.

**8.** Composé selon la revendication 2 dans lequel m = 1.

**9.** Composé selon la revendication 2 dans lequel n = 1.

**10.** Composé selon la revendication 5 dans lequel X' est F.

**11.** Composé selon la revendication 1 dans lequel au moins l'un des restes $R_1$ et $R_2$ est méthyle ou éthyle et $R_3$ est un groupe méthyle, éthyle ou octyle.

**12.** Composés spécifiques selon la revendication 2, tels que définis dans le tableau suivant :

| $SiR_1R_2R_3$ en position 3 | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, $CH_3$ | 0 | 0 | F | 5-$SiCH_3CH_3CH_3$ |
| Octyle, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| où Et est un groupe éthyle et Q est un groupe $>C=O$. | | | | |

**13.** Procédé pour préparer un composé de formule :

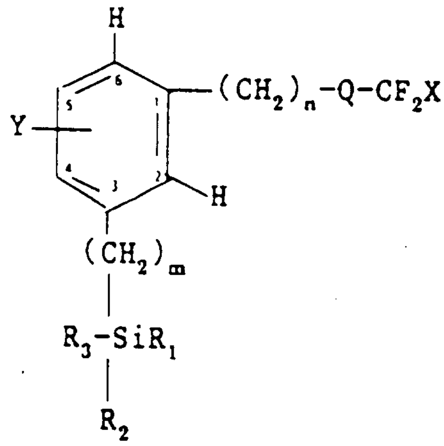

et ses sels pharmaceutiquement acceptables, dans laquelle m et n sont chacun 0 ou 1 avec la condition que la somme m + n soit inférieure à 2.
Q est

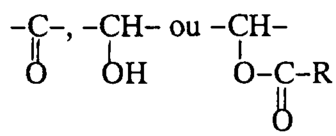

où R est H ou alkyle en $C_1$-$C_{10}$,
X est X' ou X'' et
X' est H, Br, Cl, F ou $R_4$ et
X'' est $COR_9$, $CO_2R_5$, $CONHR_5$ ou $COR_6$,
$R_1$, $R_2$, $R_3$ et $R_4$ étant chacun alkyle en $C_1$-$C_{10}$ ou -$(CH_2)$p-aryle, ou p est égal à 0, 1 ou 2
$R_5$ est H, alkyle en $C_1$-$C_{10}$, phényle, benzyle ou phénéthyle,
$R_9$ est alkyle en $C_1$-$C_{10}$, phényle, benzyle ou phénéthyle,
$R_6$ est $(NHCHR_7C(O))_qR_8$ où $R_7$ est le reste d'un α-aminoacide naturel, q est un nombre de 1 à 4 et $R_8$ est $OR_5$ ou $NHR_5$,
Y est H, OH, alkyle en $C_1$-$C_6$, alcoxy en $C_1$-$C_6$, hydroxyalkyle en $C_1$-$C_6$,aminoalkyle en $C_1$-$C_6$, $NH_2$, azido, CN, $CO_2R_5$, $COR_9$, -$SO_3H$, Br, Cl, F ou -$(CH_2)_xSiR_1R_2R_3$ où x est égal à 0, 1 ou 2,
qui comprend

(a) dans le cas où l'on prépare un composé de la sous-classe de formule :

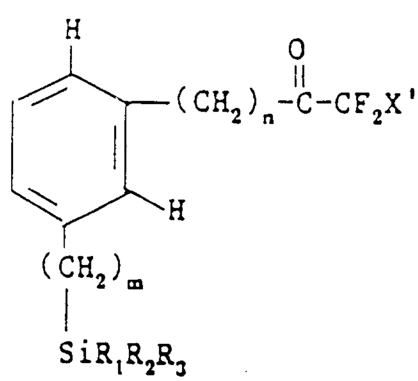

(2)

la formation d'un dérivé lithié d'un composé de formule :

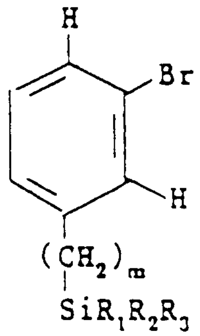

(3)

ou

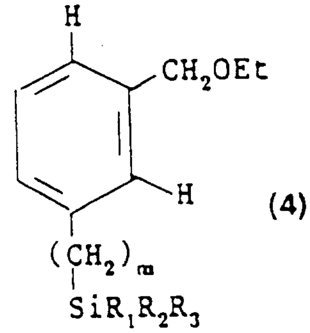

(4)

et la réaction dudit dérivé lithié avec un acide (sous forme de son sel de lithium) un ester ou un chlorure d'acide de formule $X'CF_2CO_2R$ ou $X_4CF_2COCl$ où R est H ou alkyle en $C_1$-$C_6$;

(b) dans le cas ou l'on prépare un composé de la sous-classe de formule :

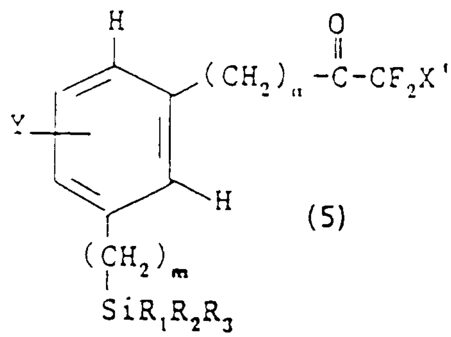

(5)

dans laquelle Y est autre que H

l'oxydation d'un composé de formule :

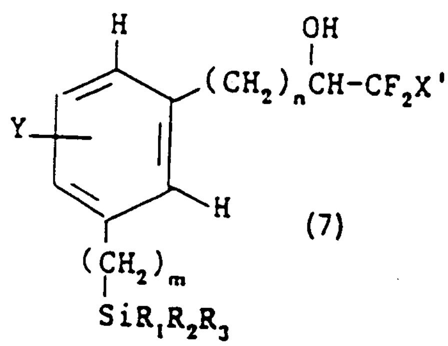

(7)

dans laquelle Y est autre que H

(c) dans le cas où l'on prépare un composé de la sous-classe de formule :

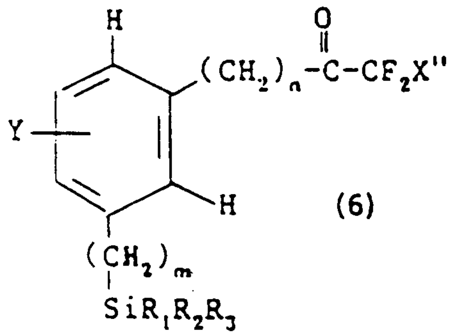

(6)

l'oxydation d'un composé de formule :

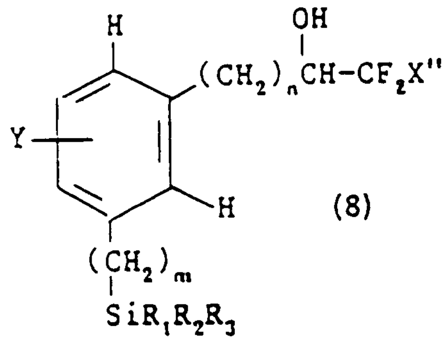

(8)

(d) dans le cas où l'on prépare un composé de la sous-classe de formule :

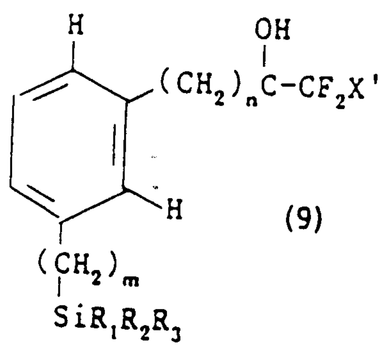

(9)

la réduction chimique d'un composé de formule (2) ci-dessus,

(e) dans le cas où l'on prépare un composé de la sous-classe de formule :

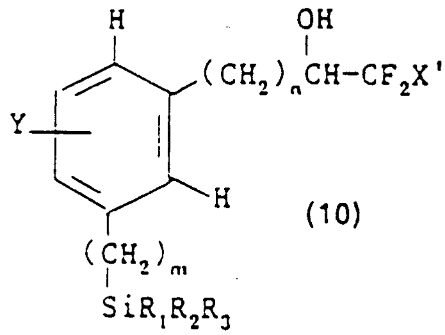

(10)

dans laquelle Y est autre que H, l'hydrolyse d'un composé de formule :

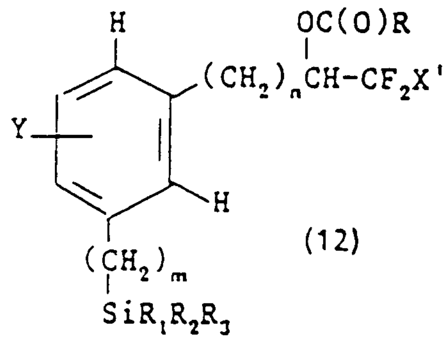

(12)

dans laquelle Y est autre que H, (f) dans le cas où l'on prépare un composé de la sous-classe de formule :

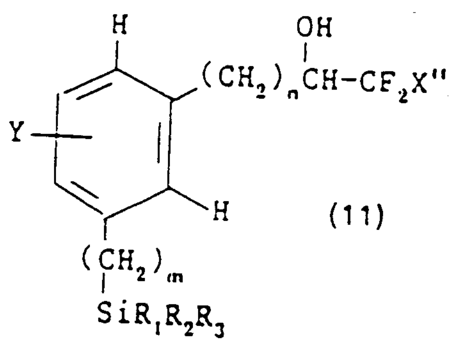

(11)

l'hydrolyse d'un composé de formule :

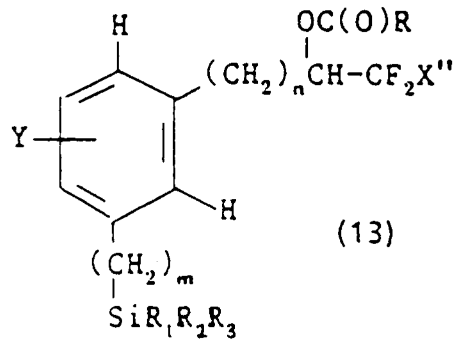

(13)

(g) dans le cas où l'on prépare un composé de la sous-classe de formule :

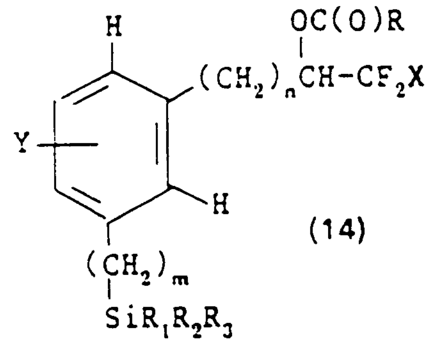

(14)

l'estérification d'un composé de formule :

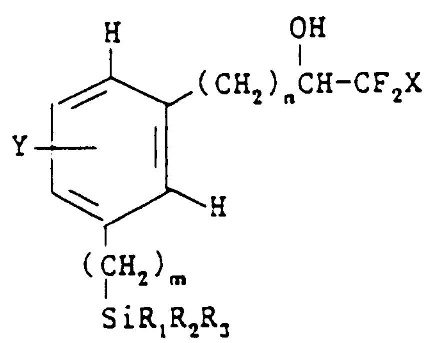

(15)

par un halogénure d'acyle,
(h) dans le cas où l'on prépare un composé de la sous-classe de formule :

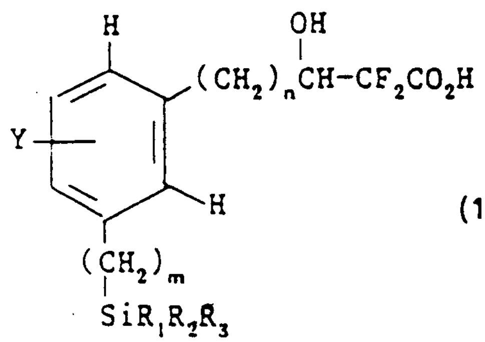

(16)

l'hydrolyse d'un composé de formule :

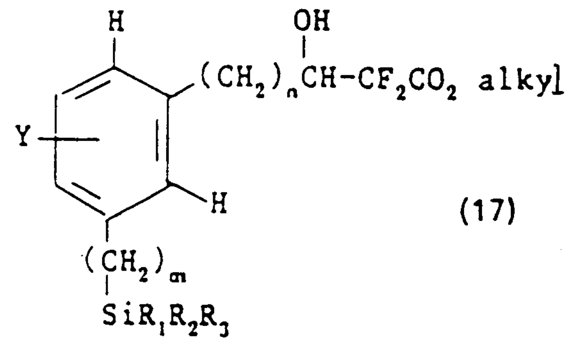

(17)

par réaction avec l'hydroxyde de lithium dans l'éther diméthylique d'éthylèneglycol à 80 %.
(i) dans le cas où l'on prépare un composé de la sous-classe de formule :

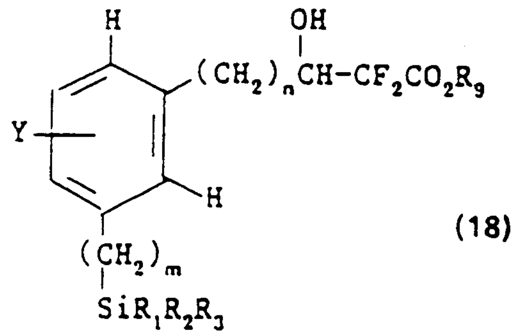

(18)

la réaction d'un composé de formule :

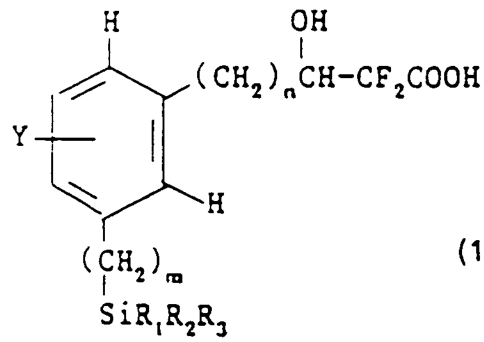

(19)

avec un composé de formule $R_9OH$ en présence de dicyclohexylcarbodiimide et de diméthylamino-pyridine.

(j) dans le cas où l'on prépare un composé de la sous-classe de formule :

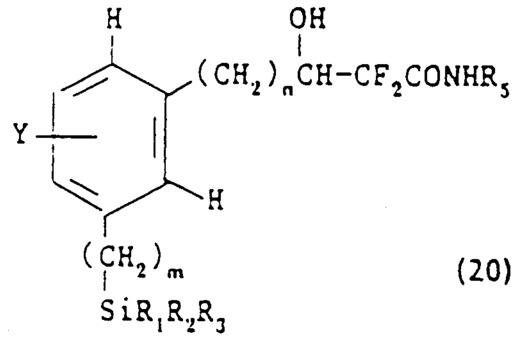

(20)

la réaction d'un composé de formule :

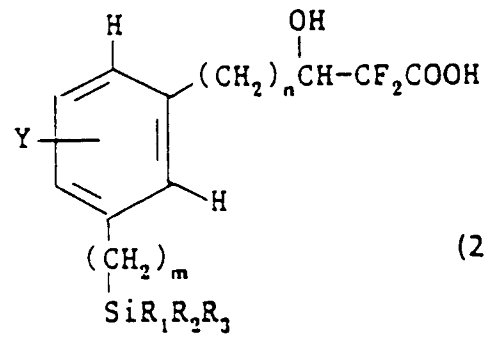

(21)

avec une amine de formule $R_5NH_2$ en présence de dicyclohexylcarbodiimide et de 1-hydroxybenzotriazole.

(k) dans le cas où l'on prépare un composé de la sous-classe de formule :

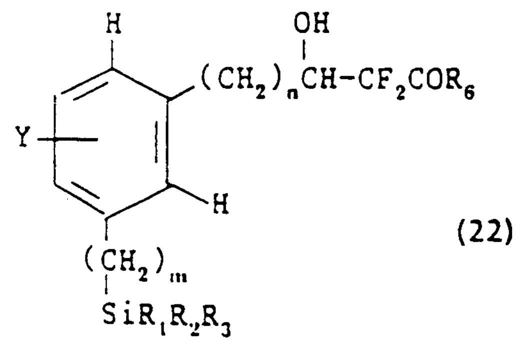

(22)

la réaction d'un composé de formule :

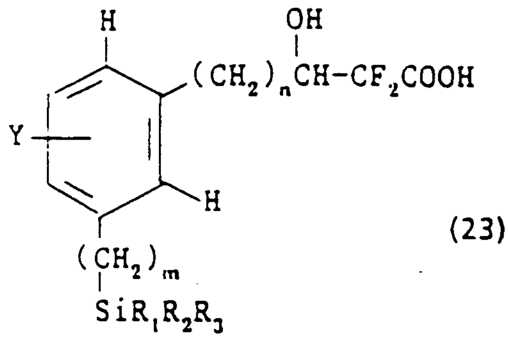

(23)

avec un composé de formule $(NHCHR_7C(O))_qR_8$
dans laquelle $R_7$ est le résidu d'un α-aminoacide, q est un entier de 1 à 4 et $R_8$ est $OR_5$ ou $NHR_5$, suivies facultativement de la conversion des produits des étapes (a) à (i) en leurs sels pharmaceutiquement acceptables.

**14.** Composé selon l'une quelconque des revendications 1 à 12 pour l'utilisation comme composé pharmaceutiquement actif.

**15.** Utilisation des composés selon l'une quelconque des revendications 1 à 12 pour la préparation de médicaments utiles pour inhiber l'acétylcholinestérase.

**16.** Utilisation des composés selon l'une quelconque des revendications 1 à 12 pour la préparation de médicaments utiles pour le traitement de la maladie d'Alzheimer et de la démence sénile.

**Revendications pour l'Etat contractant suivant : ES**

**1.** Procédé pour préparer un composé de formule :

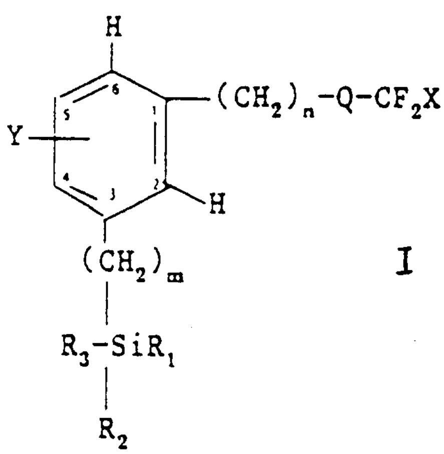

I

et ses sels pharmaceutiquement acceptables, dans laquelle m et n sont chacun 0 ou 1 avec la condition que la somme m + n soit inférieure à 2.
Q est

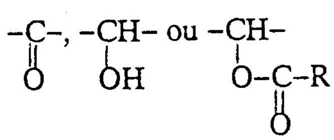

ou

où R est H ou alkyle en $C_1$-$C_{10}$,

X est X' ou X'' et
X' est H, Br, Cl, F ou $R_4$ et
X'' est $COR_9$, $CO_2R_5$, $CONHR_5$ ou $COR_6$,
$R_1$, $R_2$, $R_3$ et $R_4$ étant chacun alkyle en $C_1$-$C_{10}$ ou -$(CH_2)$p-aryle, ou p est égal à 0, 1 ou 2
$R_5$ est H, alkyle en $C_1$-$C_{10}$, phényle, benzyle ou phénéthyle,
$R_9$ est alkyle en $C_1$-$C_{10}$, phényle, benzyle ou phénéthyle,
$R_6$ est $(NHCHR_7C(O))_qR_8$ où $R_7$ est le reste d'un $\alpha$-aminoacide naturel, q est un nombre de 1 à 4 et $R_8$ est $OR_5$ ou $NHR_5$,
Y est H, OH, alkyle en $C_1$-$C_6$, alcoxy en $C_1$-$C_6$, hydroxyalkyle en $C_1$-$C_6$,aminoalkyle en $C_1$-$C_6$, $NH_2$, azido, CN, $CO_2R_5$, $COR_9$, $-SO_3H$, Br, Cl, F ou $-(CH_2)_xSiR_1R_2R_3$ où x est égal à 0, 1 ou 2,
qui comprend

(a) dans le cas où l'on prépare un composé de la sous-classe de formule :

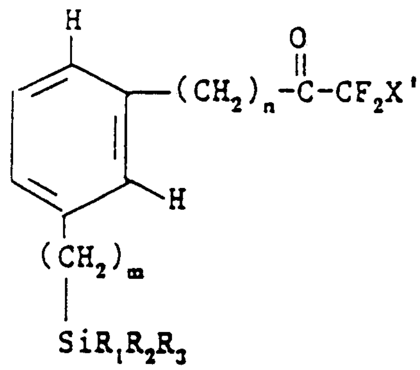

(2)

la formation d'un dérivé lithié d'un composé de formule :

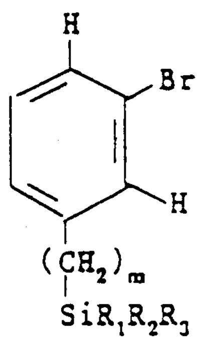

(3)

ou

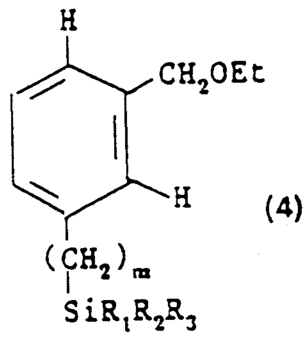

(4)

et la réaction dudit dérivé lithié avec un acide (sous forme de son sel de lithium) un ester ou un chlorure d'acide de formule $X'CF_2CO_2R$ ou $X_4CF_2COCl$ où R est H ou alkyle en $C_1$-$C_6$;

(b) dans le cas ou l'on prépare un composé de la sous-classe de formule :

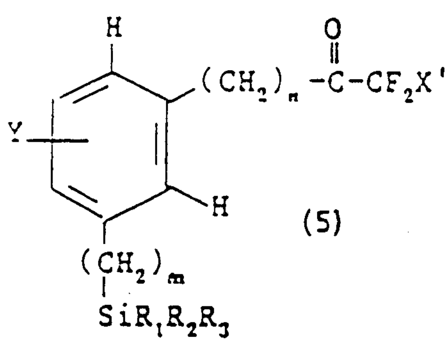

(5)

dans laquelle Y est autre que H
l'oxydation d'un composé de formule :

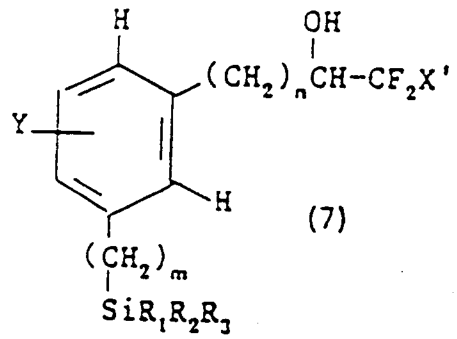

(7)

dans laquelle Y est autre que H
(c) dans le cas où l'on prépare un composé de la sous-classe de formule :

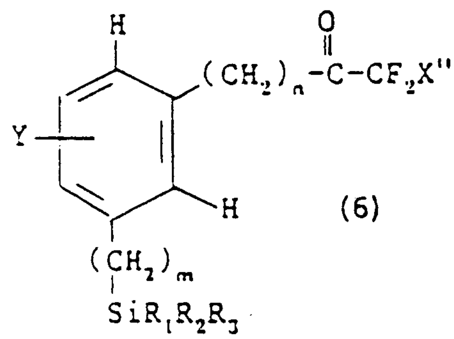

(6)

l'oxydation d'un composé de formule :

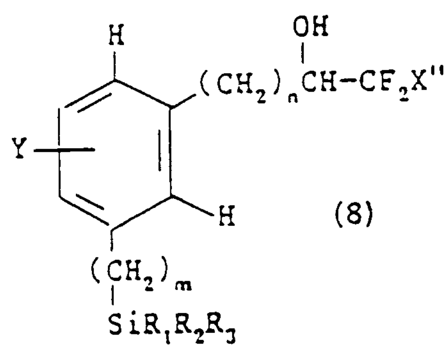

(8)

(d) dans le cas où l'on prépare un composé de la sous-classe de formule :

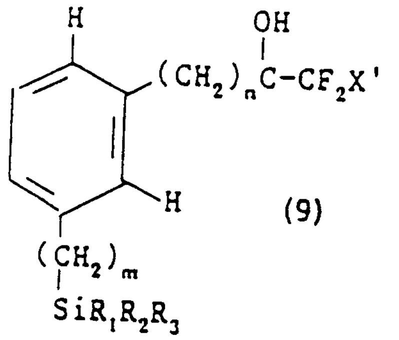

(9)

la réduction chimique d'un composé de formule (2) ci-dessus,
(e) dans le cas où l'on prépare un composé de la sous-classe de formule :

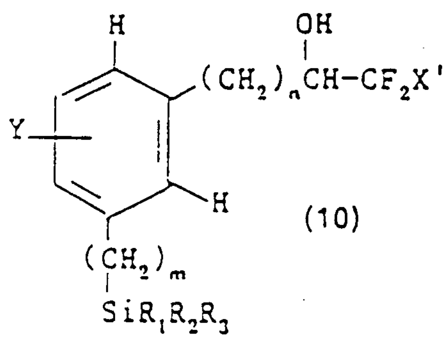

(10)

dans laquelle Y est autre que H,
l'hydrolyse d'un composé de formule :

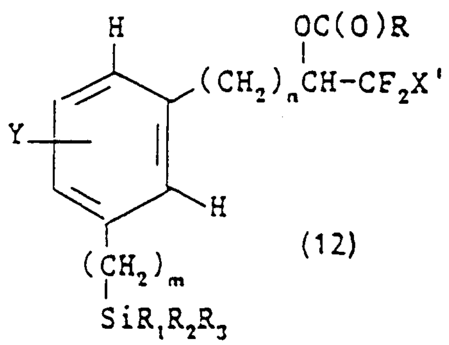

(12)

dans laquelle Y est autre que H,

(f) dans le cas où l'on prépare un composé de la sous-classe de formule :

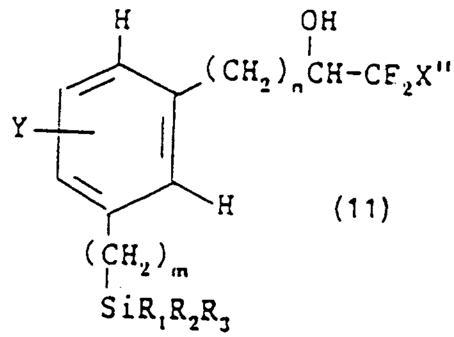

(11)

l'hydrolyse d'un composé de formule :

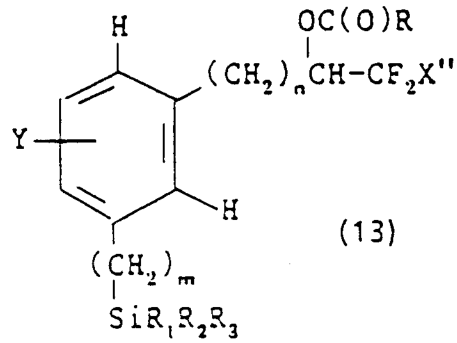

(13)

(g) dans le cas où l'on prépare un composé de la sous-classe de formule :

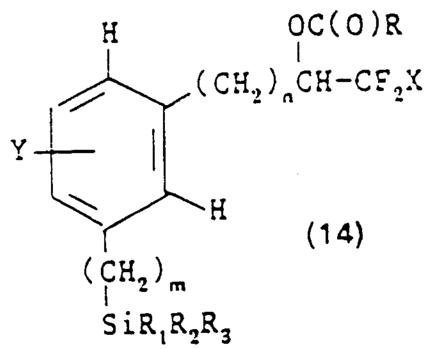

(14)

l'estérification d'un composé de formule :

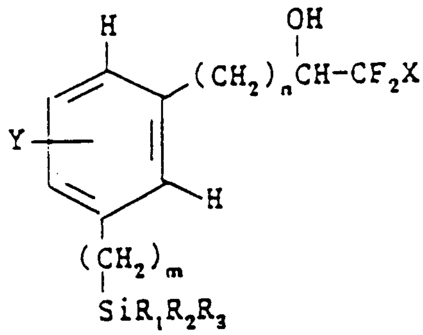

(15)

par un halogénure d'acyle,

(h) dans le cas où l'on prépare un composé de la sous-classe de formule :

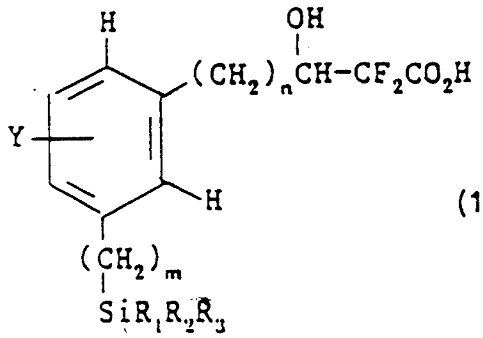

(16)

l'hydrolyse d'un composé de formule :

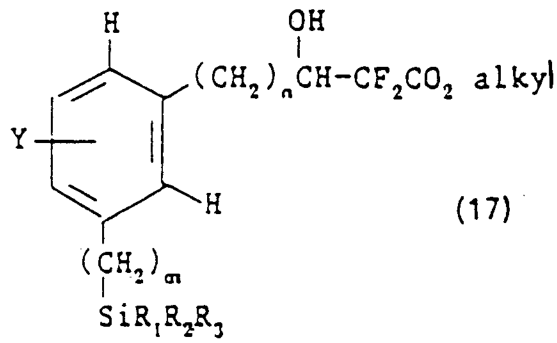

(17)

par réaction avec l'hydroxyde de lithium dans l'éther diméthylique d'éthylèneglycol à 80 %.

(i) dans le cas où l'on prépare un composé de la sous-classe de formule :

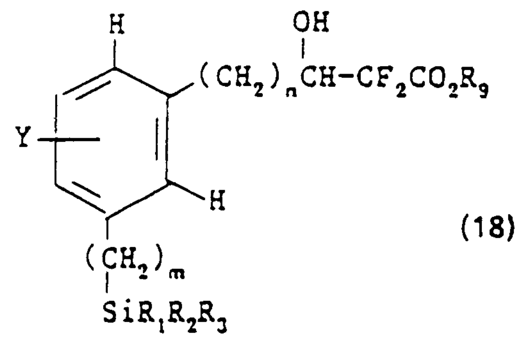

(18)

la réaction d'un composé de formule :

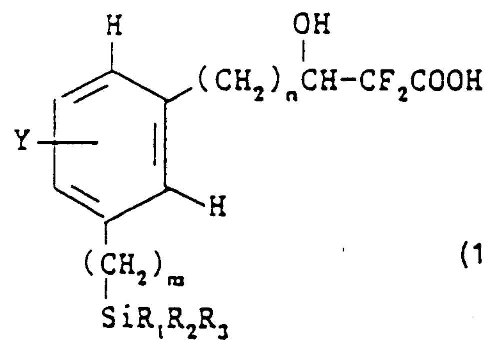

(19)

avec un composé de formule $R_9OH$ en présence de dicyclohexylcarbodiimide et de diméthylamino-pyridine.

(j) dans le cas où l'on prépare un composé de la sous-classe de formule :

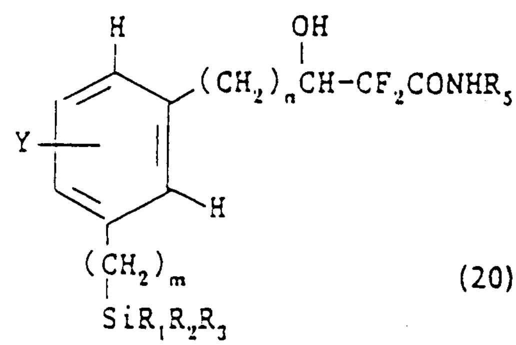

(20)

la réaction d'un composé de formule :

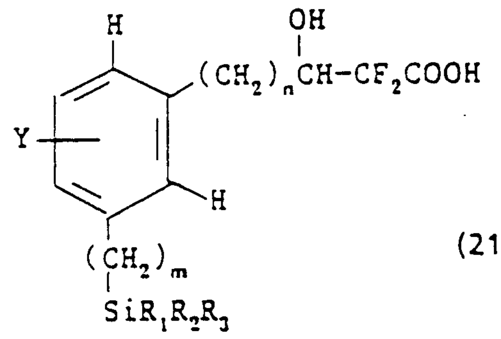

(21)

avec une amine de formule $R_5NH_2$ en présence de dicyclohexylcarbodiimide et de 1-hydroxybenzotriazole.

(k) dans le cas où l'on prépare un composé de la sous-classe de formule :

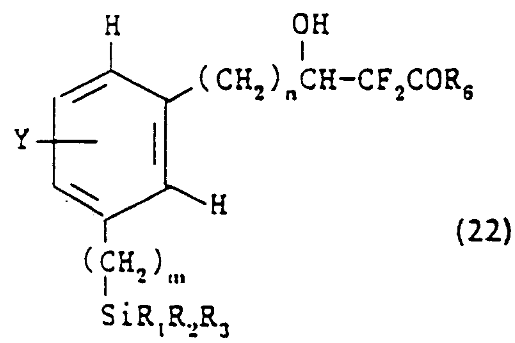

(22)

la réaction d'un composé de formule :

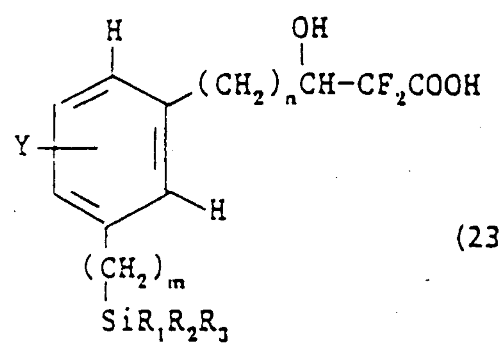

(23)

avec un composé de formule $(NHCHR_7C(O))_qR_8$

dans laquelle $R_7$ est le résidu d'un $\alpha$-aminoacide, q est un entier de 1 à 4 et $R_8$ est $OR_5$ ou $NHR_5$, suivies facultativement de la conversion des produits des étapes (a) à (i) en leurs sels pharmaceutiquement acceptables.

2. Procédé selon la revendication 1 pour préparer un composé de formule (I) dans lequel au moins l'un de $R_1$ et $R_2$ est méthyle ou éthyle et $R_3$ est méthyle, éthyle ou octyle, caractérisé en ce que dans les points (a), (b), (c), (d), (e), (f), (g), (h), (i), (j) ou (k) on utilise des composés de formules (3) et (4), (7), (8), (9), (12), (13), (15), (17), (19), (21) ou (23) tels que définies dans la revendication 1, dans lesquelles $R_1$, $R_2$ et $R_3$ sont tels que définis ci-dessus.

3. Procédé selon la revendication 1 pour préparer des composés de formule (I) dans laquelle Q est $>C=O$ et $R_1$, $R_2$, $R_3$, m, n, X et Y sont tels que définis ci-dessous

| $SiR_1R_2R_3$ en position 3 | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, $CH_3$ | 0 | 0 | F | 5-$SiCH_3CH_3CH_3$ |
| Octyle, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| où Et est un groupe éthyle | | | | |

et leurs sels pharmaceutiquement acceptables, caractérisé en ce que :

- dans le point (a), on utilise des composés de formules (3) et (4) dans lesquelles m, $R_1$, $R_2$ et $R_3$ sont tels que définis ci-dessus, et des composés de formules $X'CF_2CO_2R$ ou $X_4CF_2COCl$ dans lesquelles X' est H ou F, X est tel que défini ci-dessus et R est tel que défini ci-dessus ;
- dans le point (b), on utilise un composé de formule (7) dans laquelle Y est 5-$SiCH_3CH_3CH_3$, n, m, $R_1$, $R_2$, $R_3$ sont tels que définis ci-dessus et X' = H ou F;
- dans le point (c), on utilise un composé de formule (8) dans laquelle Y est 5-$SiCH_3CH_3CH_3$, n, m, $R_1$, $R_2$, $R_3$ sont tels que définis ci-dessus et X" est $CH_3$, $-COCH_3$, $-COOCH_3$ ou $-CONHCH_3$.

**4.** Procédé de préparation de médicaments utiles pour inhiber l'acétylcholinestérase, caractérisé par le mélange d'un composé préparé selon le procédé selon l'une quelconque des revendications 1 à 3 ou de ses sels pharmaceutiquement acceptables, en tant qu'ingrédient actif, avec un véhicule pharmaceutiquement acceptable.

**5.** procédé de préparation de médicaments utiles pour le traitement de la maladie d'Alzheimer et de la démence sénile, caractérisé par le mélange d'un composé préparé par le procédé selon l'une quelconque des revendications 1 à 3 ou de ses sels pharmaceutiquement acceptables, en tant qu'ingrédient actif, avec un véhicule pharmaceutiquement acceptable.

**Revendications pour l'Etat contractant suivant : GR**

**1.** Composé de formule :

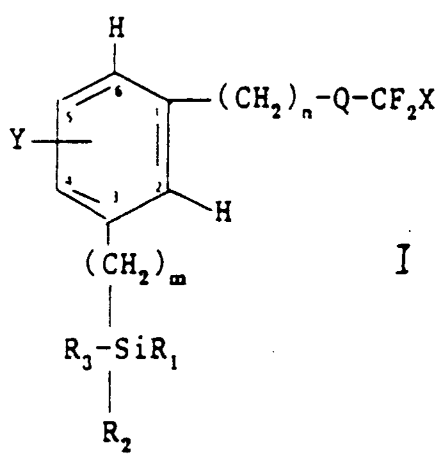

I

et ses sels pharmaceutiquement acceptables, dans laquelle m et n sont chacun 0 ou 1 avec la

condition que la somme m + n soit inférieure à 2,
Q est

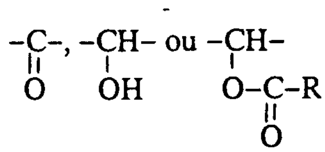

où R est H ou alkyle en $C_1$-$C_{10}$,
X est X' ou X'' et
X' est H, Br, Cl, F ou $R_4$ et
X'' est $COR_9$, $CO_2R_5$, $CONHR_5$ ou $COR_6$,
$R_1$, $R_2$, $R_3$ et $R_4$ étant chacun alkyle en $C_1$-$C_{10}$ ou -$(CH_2)$p-aryle, où p est égal à 0, 1 ou 2
$R_5$ est H, alkyle en $C_1$-$C_{10}$, phényle, benzyle ou phénéthyle,
$R_9$ est alkyle en $C_1$-$C_{10}$, phényle, benzyle ou phénéthyle,
$R_6$ est $(NHCHR_7C(O))_qR_8$ où $R_7$ est le reste d'un α-aminoacide naturel, q est un nombre de 1 à 4 et $R_8$ est $OR_5$ ou $NHR_5$,
Y est H, OH, alkyle en $C_1$-$C_6$, alcoxy en $C_1$-$C_6$, hydroxyalkyle en $C_1$-$C_6$,aminoalkyle en $C_1$-$C_6$, $NH_2$, azido, CN, $CO_2R_5$, $COR_9$, $-SO_3H$, Br, Cl, F ou $-(CH_2)_xSiR_1R_2R_3$ où x est égal à 0, 1 ou 2.

**2.** Composé selon la revendication 1 dans lequel Q est

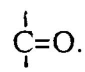

**3.** Composé selon la revendication 1 dans lequel Q est

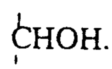

**4.** Composé selon la revendication 1 dans lequel Q est

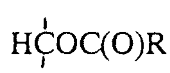

**5.** Composé selon la revendication 2 dans lequel X est X'.

**6.** Composé selon la revendication 2 dans lequel X est X''.

**7.** Composé selon la revendication 2 dans lequel m = n = 0.

**8.** Composé selon la revendication 2 dans lequel m = 1.

**9.** Composé selon la revendication 2 dans lequel n = 1.

**10.** Composé selon la revendication 5 dans lequel X' est F.

**11.** Composé selon la revendication 1 dans lequel au moins l'un des restes $R_1$ et $R_2$ est méthyle ou éthyle et $R_3$ est un groupe méthyle, éthyle ou octyle.

**12.** Composés spécifiques selon la revendication 2, tels que définis dans le tableau suivant :

| $SiR_1R_2R_3$ en position 3 | m | n | X | (Y) |
|---|---|---|---|---|
| $CH_3$, $CH_3$, $CH_3$ | 0 | 0 | F | H |
| $CH_3$, Et, Et | 0 | 0 | F | H |
| Et, Et, Et | 0 | 0 | F | H |
| Et, Et, $CH_3$ | 0 | 0 | F | H |
| $CH_3$, $CH_3$, $CH_3$ | 0 | 0 | F | 5-$SiCH_3CH_3CH_3$ |
| Octyle, $CH_3$, $CH_3$ | 0 | 0 | F | H |
| $CH_3$, $CH_3$, $CH_3$ | 1 | 0 | F | H |
| $CH_3$, $CH_3$, $CH_3$ | 0 | 1 | F | H |
| $CH_3$, $CH_3$, $CH_3$ | 0 | 0 | H | H |
| $CH_3$, $CH_3$, $CH_3$ | 0 | 0 | $CH_3$ | H |
| $CH_3$, $CH_3$, $CH_3$ | 0 | 0 | $-COCH_3$ | H |
| $CH_3$, $CH_3$, $CH_3$ | 0 | 0 | $-COOCH_3$ | H |
| $CH_3$, $CH_3$, $CH_3$ | 0 | 0 | $-CONHCH_3$ | H |
| où Et est un groupe éthyle et Q est un groupe $>C=O$. | | | | |

**13.** Procédé pour préparer un composé de formule :

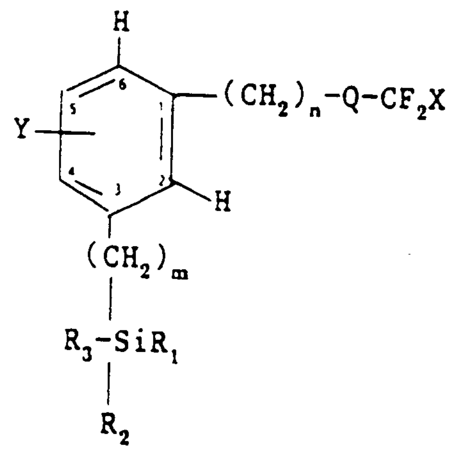

et ses sels pharmaceutiquement acceptables, dans laquelle m et n sont chacun 0 ou 1 avec la condition que la somme $m+n$ soit inférieure à 2.
Q est

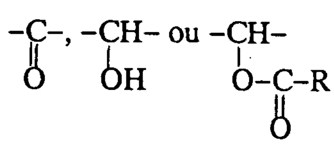

où R est H ou alkyle en $C_1$-$C_{10}$,
X est X' ou X'' et
X' est H, Br, Cl, F ou $R_4$ et
X'' est $COR_9$, $CO_2R_5$, $CONHR_5$ ou $COR_6$,
$R_1$, $R_2$, $R_3$ et $R_4$ étant chacun alkyle en $C_1$-$C_{10}$ ou $-(CH_2)p$-aryle, ou p est égal à 0, 1 ou 2
$R_5$ est H, alkyle en $C_1$-$C_{10}$, phényle, benzyle ou phénéthyle,
$R_9$ est alkyle en $C_1$-$C_{10}$, phényle, benzyle ou phénéthyle,
$R_6$ est $(NHCHR_7C(O))_qR_8$ où $R_7$ est le reste d'un $\alpha$-aminoacide naturel, q est un nombre de 1 à 4 et $R_8$ est $OR_5$ ou $NHR_5$,
Y est H, OH, alkyle en $C_1$-$C_6$, alcoxy en $C_1$-$C_6$, hydroxyalkyle en $C_1$-$C_6$,aminoalkyle en $C_1$-$C_6$, $NH_2$ azido, CN, $CO_2R_5$, $COR_9$, $-SO_3H$, Br, Cl, F ou $-(CH_2)_xSiR_1R_2R_3$ où x est égal à 0, 1 ou 2,

qui comprend

(a) dans le cas où l'on prépare un composé de la sous-classe de formule :

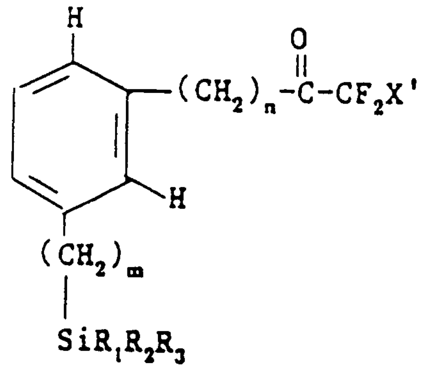

(2)

la formation d'un dérivé lithié d'un composé de formule :

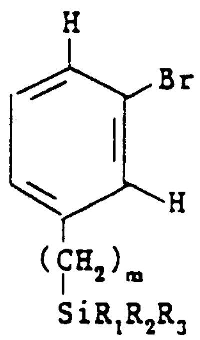

(3)

ou

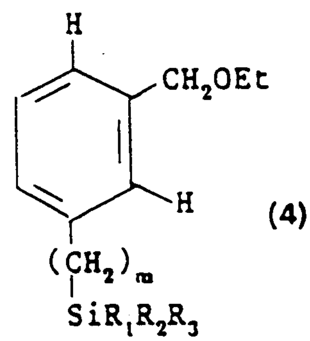

(4)

et la réaction dudit dérivé lithié avec un acide (sous forme de son sel de lithium) un ester ou un chlorure d'acide de formule $X'CF_2CO_2R$ ou $X_4CF_2COCl$ où R est H ou alkyle en $C_1$-$C_6$;

(b) dans le cas ou l'on prépare un composé de la sous-classe de formule :

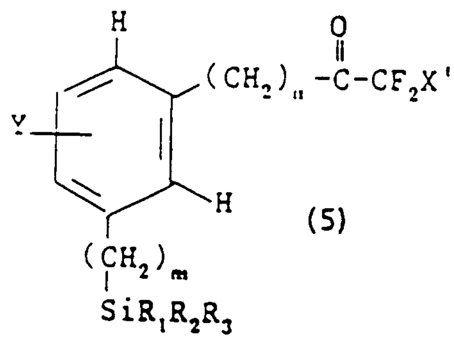

(5)

dans laquelle Y est autre que H

l'oxydation d'un composé de formule :

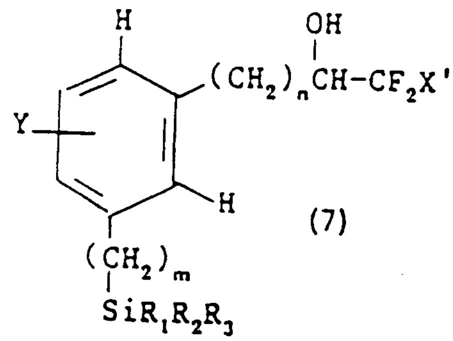

(7)

dans laquelle Y est autre que H

(c) dans le cas où l'on prépare un composé de la sous-classe de formule :

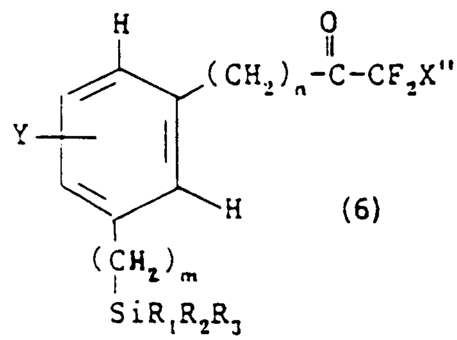

(6)

l'oxydation d'un composé de formule :

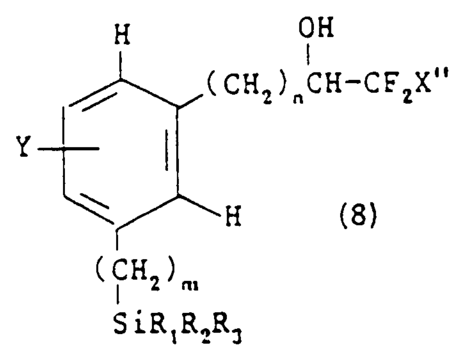

(8)

(d) dans le cas où l'on prépare un composé de la sous-classe de formule :

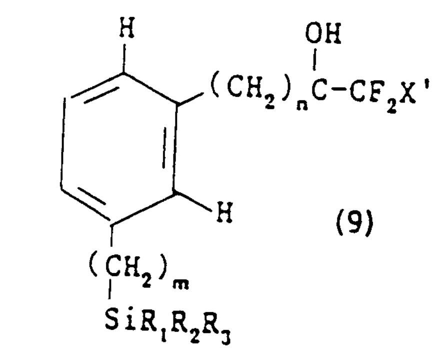

(9)

la réduction chimique d'un composé de formule (2) ci-dessus,
(e) dans le cas où l'on prépare un composé de la sous-classe de formule :

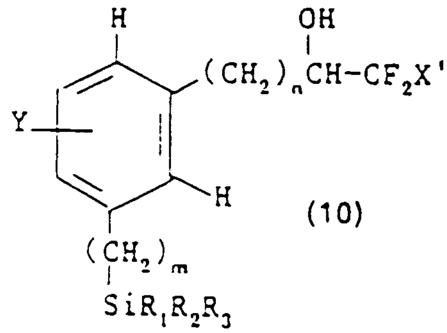

(10)

dans laquelle Y est autre que H,
l'hydrolyse d'un composé de formule :

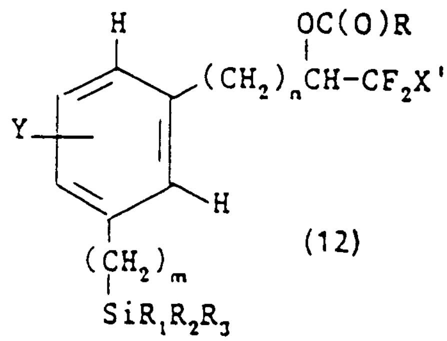

(12)

dans laquelle Y est autre que H,
(f) dans le cas où l'on prépare un composé de la sous-classe de formule :

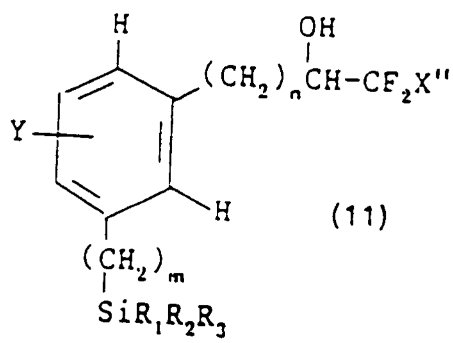

(11)

l'hydrolyse d'un composé de formule :

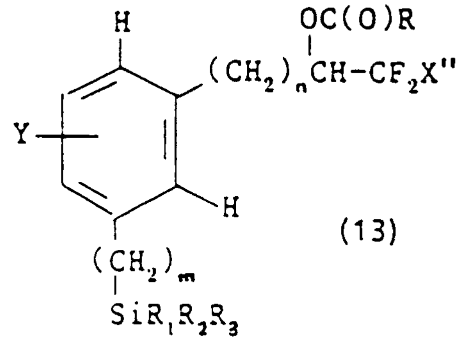

(13)

(g) dans le cas où l'on prépare un composé de la sous-classe de formule :

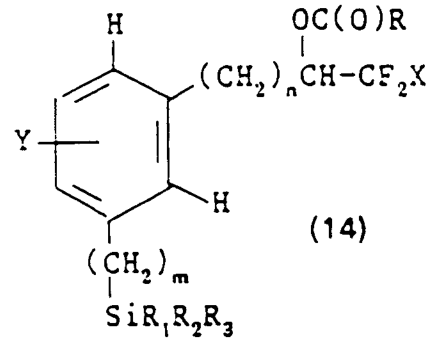

(14)

l'estérification d'un composé de formule :

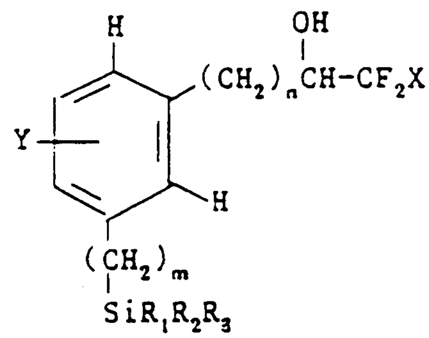

(15)

par un halogénure d'acyle,
(h) dans le cas où l'on prépare un composé de la sous-classe de formule :

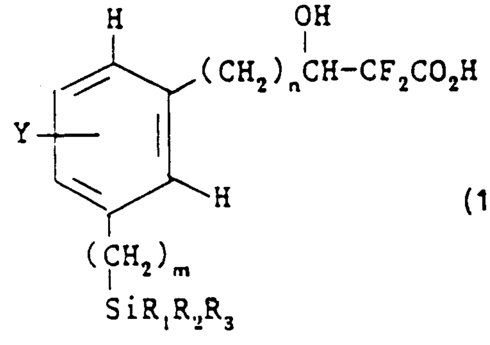

(16)

l'hydrolyse d'un composé de formule :

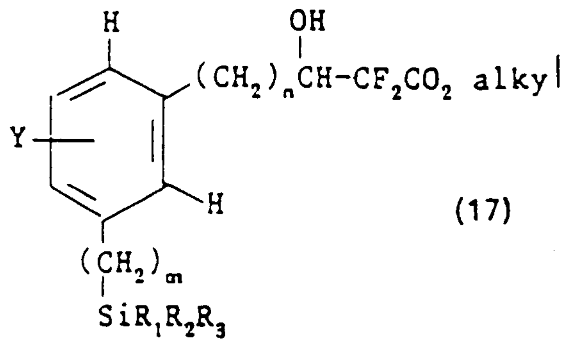

(17)

par réaction avec l'hydroxyde de lithium dans l'éther diméthylique d'éthylèneglycol à 80 %.
(i) dans le cas où l'on prépare un composé de la sous-classe de formule :

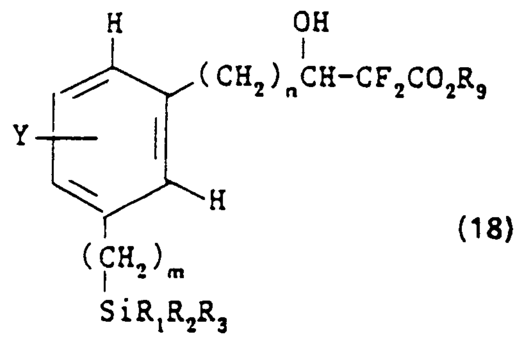

(18)

la réaction d'un composé de formule :

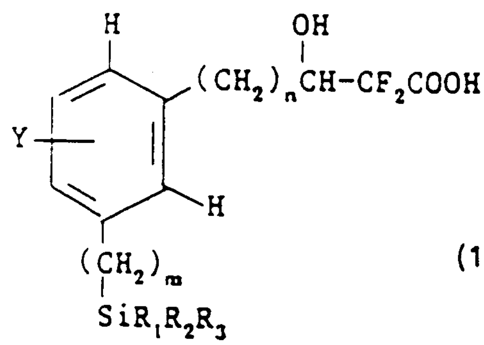

(19)

avec un composé de formule $R_9OH$ en présence de dicyclohexylcarbodiimide et de diméthylamino-pyridine.

(j) dans le cas où l'on prépare un composé de la sous-classe de formule :

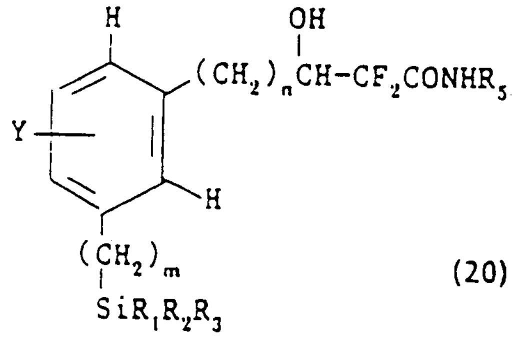

(20)

la réaction d'un composé de formule :

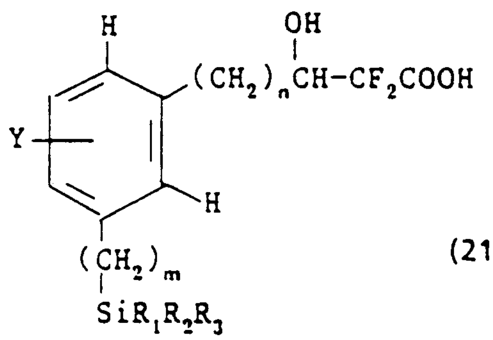

(21)

avec une amine de formule $R_5NH_2$ en présence de dicyclohexylcarbodiimide et de 1-hydroxybenzotriazole.

(k) dans le cas où l'on prépare un composé de la sous-classe de formule :

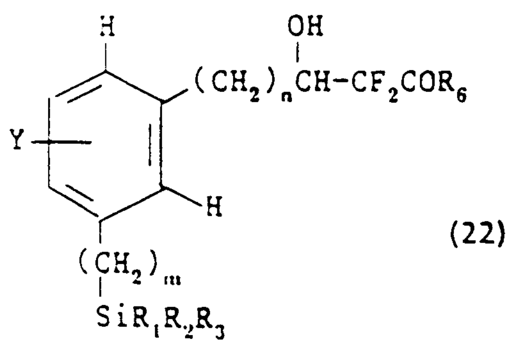

(22)

la réaction d'un composé de formule :

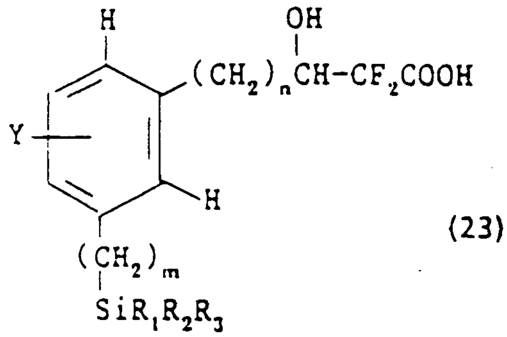

(23)

avec un composé de formule $(NHCHR_7C(O))_qR_8$
dans laquelle $R_7$ est le résidu d'un α-aminoacide, q est un entier de 1 à 4 et $R_8$ est $OR_5$ ou $NHR_5$, suivies facultativement de la conversion des produits des étapes (a) à (i) en leurs sels pharmaceutiquement acceptables.

**14.** Procédé de préparation de médicaments utiles pour inhiber l'acétylcholinestérase, caractérisé par le mélange d'un composé selon l'une quelconque des revendications 1 à 12 ou de ses sels pharmaceutiquement acceptables, en tant qu'ingrédient actif, avec un véhicule pharmaceutiquement acceptable.

**15.** Procédé de préparation de médicaments utiles pour le traitement de la maladie d'Alzheimer et de la démence sénile, caractérisé par le mélange d'un composé selon l'une quelconque des revendications 1 à 12 ou de ses sels pharmaceutiquement acceptables, en tant qu'ingrédient actif, avec un véhicule pharmaceutiquement acceptable.